(19) Europäisches Patentamt
European Patent Office
Office européen des brevets

(11) EP 1 355 149 A2

(12) **EUROPEAN PATENT APPLICATION**

(43) Date of publication:
**22.10.2003 Bulletin 2003/43**

(51) Int Cl.[7]: **G01N 33/48**, C07H 21/04, C12Q 1/68

(21) Application number: **03252022.3**

(22) Date of filing: **31.03.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **29.03.2002 US 368798 P**

(71) Applicant: **Ortho-Clinical Diagnostics, Inc.**
**Rochester, NY 14626-5101 (US)**

(72) Inventor: **Wang, Yixin**
**San Diego, CA 92130 (US)**

(74) Representative: **Mercer, Christopher Paul et al**
**Carpmaels & Ransford**
**43, Bloomsbury Square**
**London WC1A 2RA (GB)**

(54) **Assessing colorectal cancer**

(57) A method of assessing the presence or absence of colorectal cancer or the likely condition of a person believed to have colorectal cancer is conducted by analyzing the expression of a group of genes. Gene expression profiles in a variety of medium such as microarrays are included as are kits that contain them.

EP 1 355 149 A2

## Description

## BACKGROUND

This application claims the benefit of U.S Provisional Application No.60/368,798 filed on March 29, 2002.

This invention relates to diagnostics and prognostics for colorectal cancer based on the gene expression profiles of biological samples.

Colorectal cancer is a heterogenous disease, consisting of tumors thought to emerge through three major molecular mechanisms: 1) mutations in the adenomatous polyposis coli (APC) gene, or the β-catenin gene, combined with chromosomal instability, 2) mutations in DNA mismatch repair genes, such as MLH1, MSH2, PMS1, PMS2 and MSH6, associated with microsatellite instability and mutations in genes containing short repeats, and 3) gene silencing induced by hypermethylation of the promoter regions of tumor suppressor genes. The genetic complement of individual colorectal cancers is likely to include different combinations of genetic instability, specific mutations, and gene silencing. Chromosomal instability (CIN) is a common feature of cancers in general. It implies an aneuploid phenotype, in which whole chromosomes or large parts of them are being lost or gained. Microsomal instability (MIN) is found in diploid tumors with an increased mutation rate in short repeats. Both forms of genetic instability are common in colorectal cancer.

Colorectal cancers thus have complex origins and involve a number of interactions in different biological pathways. Serum markers, histological, and cytological examinations historically used to assist in providing diagnostic, prognostic, or therapy monitoring decisions often do not have desired reliability. Likewise, while use of a single genetic marker (e.g., increased expression of a particular gene) may be beneficial, the diversity of the cancers make it more likely that a portfolio of genetic markers is the best approach.

## SUMMARY OF THE INVENTION

The invention is a method of assessing the presence or absence of colorectal cancer or the likely condition of a person believed to have colorectal cancer. In the method, a gene expression profile of a patient sample is analyzed to determine whether a patient has a colorectal cancer, whether a patient does not have colorectal cancer, whether a patient is likely to get colorectal cancer, or the response to treatment of a patient being treated for colorectal cancer.

Articles used in practising the methods are also an aspect of the invention. Such articles include gene expression profiles or representations of them that are fixed in machine-readable media such as computer readable media.

Articles used to identify gene expression profiles can also include substrates or surfaces, such as microarrays, to capture and/or indicate the presence, absence, or degree of gene expression.

## DETAILED DESCRIPTION

The mere presence or absence of particular nucleic acid sequences in a tissue sample has only rarely been found to have diagnostic or prognostic value. Information about the expression of various proteins, peptides or mRNA, on the other hand, is increasingly viewed as important. The mere presence of nucleic acid sequences having the potential to express proteins, peptides, or mRNA ( such sequences referred to as "genes") within the genome by itself is not determinative of whether a protein, peptide, or mRNA is expressed in a given cell. Whether or not a given gene capable of expressing proteins, peptides, or mRNA does so and to what extent such expression occurs, if at all, is determined by a variety of complex factors. Irrespective of difficulties in understanding and assessing these factors, assaying gene expression can provide useful information about the occurrence of important events such as tumerogenesis, metastasis, apoptosis, and other clinically relevant phenomena. Relative indications of the degree to which genes are active or inactive can be found in gene expression profiles. The gene expression profiles of this invention are used to diagnose and treat patients for colorectal cancer.

Sample preparation requires the collection of patient samples. Patient samples used in the inventive method are those that are suspected of containing diseased cells such as epithelial cells taken from a colon sample or from surgical margins. One useful technique for obtaining suspect samples is Laser Capture Microdisection (LCM). LCM technology provides a way to select the cells to be studied, minimizing variability caused by cell type heterogeneity. Consequently, moderate or small changes in gene expression between normal and cancerous cells can be readily detected. In a preferred method, the samples comprise circulating epithelial cells extracted from peripheral blood. These can be obtained according to a number of methods but the most preferred method is the magnetic separation technique described in U.S. Patent 6,136,182 assigned to Immunivest Corp which is incorporated herein by reference. Once the sample containing the cells of interest has been obtained, RNA is extracted and amplified and a gene expression profile is obtained, preferably via micro-array, for genes in the appropriate portfolios.

Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify complimentary DNA (cDNA) or complimentary RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637; the disclosures of which are incorporated herein by reference.

Microarray technology allows for the measurement of the steady-state mRNA level of thousands of genes simultaneously thereby presenting a powerful tool for identifying effects such as the onset, arrest, or modulation of uncontrolled cell proliferation. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002 to Linsley, et al.; 6,218,122 to Friend, et al.; 6,218,114 to Peck, et al.; and 6,004,755 to Wang, et al., the disclosure of each of which is incorporated herein by reference.

Analysis of the expression levels is conducted by comparing such intensities. This is best done by generating a ratio matrix of the expression intensities of genes in a test sample versus those in a control sample. For instance, the gene expression intensities from a diseased tissue can be compared with the expression intensities generated from normal tissue of the same type (e.g., diseased colon tissue sample vs. normal colon tissue sample). A ratio of these expression intensities indicates the fold-change in gene expression between the test and control samples.

Gene expression profiles can also be displayed in a number of ways. The most common method is to arrange a raw fluorescence intensities or ratio matrix into a graphical dendogram where columns indicate test samples and rows indicate genes. The data is arranged so genes that have similar expression profiles are proximal to each other. The expression ratio for each gene is visualized as a color. For example, a ratio less than one (indicating down-regulation) may appear in the blue portion of the spectrum while a ratio greater than one (indicating up-regulation) may appear as a color in the red portion of the spectrum. Commercially available computer software programs are available to display such data including "GENESPRING" from Silicon Genetics, Inc. and "DISCOVERY" and "INFER" software from Partek, Inc.

Modulated genes used in the methods of the invention are shown in Table 1. The genes that are differentially expressed are shown as being either up regulated or down regulated in diseased cells. Up regulation and down regulation are relative terms meaning that a detectable difference (beyond the contribution of noise in the system used to measure it) is found in the amount of expression of the genes relative to some baseline. In this case, the baseline is the measured gene expression of a normal cell. The genes of interest in the diseased cells are then either up regulated or down regulated relative to the baseline level using the same measurement method. Diseased, in this context, refers to an alteration of the state of a body that interrupts or disturbs, or has the potential to disturb, proper performance of bodily functions as occurs with the uncontrolled proliferation of cells. Someone is diagnosed with a disease when some aspect of that person's genotype or phenotype is consistent with the presence of the disease. However, the act of conducting a diagnosis or prognosis includes the determination disease/status issues such as therapy monitoring. In therapy monitoring, clinical judgments are made regarding the effect of a given course of therapy by comparing the expression of genes over time to determine whether the gene expression profiles have changed or are changing to patterns more consistent with normal tissue.

Preferably, levels of up and down regulation are distinguished based on fold changes of the intensity measurements of hybridized microarray probes. A 2.0 fold difference is preferred for making such distinctions or a p-value less than .05. That is, before a gene is said to be differentially expressed in diseased versus normal cells, the diseased cell is found to yield at least 2 more, or 2 times less intensity than the normal cells. The greater the fold difference, the more preferred is use of the gene as a diagnostic. Genes selected for the gene expression profiles of the instant invention have expression levels that result in the generation of a signal that is distinguishable from those of the normal or non-modulated genes by an amount that exceeds background using clinical laboratory instrumentation.

Statistical values can be used to confidently distinguish modulated from non-modulated genes and noise. Statistical tests find the genes most significantly different between diverse groups of samples. The Student's t-test is an example of a robust statistical test that can be used to find significant differences between two groups. The lower the p-value, the more compelling the evidence that the gene is showing a difference between the different groups. Nevertheless, since microarrays measure more than one gene at a time, tens of thousands of statistical tests may be asked at one time. Because of this, there is likelihood to see small p-values just by chance and adjustments for this using a Sidak correction as well as a randomization/permutation experiment can be made. A p-value less than .05 by the t-test is evidence that the gene is significantly different. More compelling evidence is a p-value less then .05 after the Sidak correct is factored in. For a large number of samples in each group, a p-value less than 0.05 after the randomization/permutation test is the most compelling evidence of a significant difference.

Another parameter that can be used to select genes that generate a signal that is greater than that of the non-modulated gene or noise is the use of a measurement of absolute signal difference. Preferably, the signal generated by the modulated gene expression is at least 20% different than those of the normal or non-modulated gene (on an absolute basis). It is even more preferred that such genes produce expression patterns that are at least 30% different than those of normal or non-modulated genes.

Genes can be grouped so that information obtained about the set of genes in the group provides a sound basis for making a clinically relevant judgment such as a diagnosis, prognosis, or treatment choice. These sets of genes make up the portfolios of the invention. In this case, the judgments supported by the portfolios involve colorectal cancer. Portfolios of gene expression profiles can be comprised of combinations of genes described in Example 3. As with most diagnostic markers, it is often desirable to use the fewest number of markers sufficient to make a correct medical judgment. This prevents a delay in treatment pending further analysis as well inappropriate use of time and resources. In this case, such a minimal portfolio can be comprised of a combination of genes from Example 4.

Preferably, portfolios are established such that the combination of genes in the portfolio exhibit improved sensitivity and specificity relative to individual genes or randomly selected combinations of genes. In the context of the instant invention, the sensitivity of the portfolio can be reflected in the fold differences exhibited by a gene's expression in the diseased state relative to the normal state. Specificity can be reflected in statistical measurements of the correlation of the signaling of gene expression with the condition of interest. For example, standard deviation can be a used as such a measurement. In considering a group of genes for inclusion in a portfolio, a small standard deviation in expression measurements correlates with greater specificity. Other measurements of variation such as correlation coefficients can also be used in this capacity. The most preferred method of establishing gene expression portfolios is through the use of optimization algorithms such as the mean variance algorithm widely used in establishing stock portfolios. This method is described in detail in the co-pending patent application entitled "Portfolio Selection" by Tim Jatkoe, et. al., of equal date hereto. Essentially, the method calls for the establishment of a set of inputs (stocks in financial applications, expression as measured by intensity here) that will optimize the return (e.g., signal that is generated) one receives for using it while minimizing the variability of the return. Many commercial software programs are available to conduct such operations. "Wagner Associates Mean-Variance Optimization Application", referred to as "Wagner Software" throughout this specification, is preferred. This software uses functions from the "Wagner Associates Mean-Variance Optimization Library" to determine an efficient frontier and optimal portfolios in the Markowitz sense is preferred.

Use of this type of software requires that microarray data be transformed so that it can be treated as an input in the way stock return and risk measurements are used when the software is used for its intended financial analysis purposes. For example, when Wagner Software is employed in conjunction with microarray intensity measurements the following data transformation method is employed.

Genes are first pre-selected by identifying those genes whose expression shows at least some minimal level of differentiation. The preferred pre-selection process is conducted as follows. A baseline class is selected. Typically, this will comprise genes from a population that does not have the condition of interest. For example, if one were interested in selecting a portfolio of genes that are diagnostic for breast cancer, samples from patients without breast cancer can be used to make the baseline class. Once the baseline class is selected, the arithmetic mean and standard deviation is calculated for the indicator of gene expression of each gene for baseline class samples. This indicator is typically the fluorescent intensity of a microarray reading. The statistical data computed is then used to calculate a baseline value of (X*Standard Deviation + Mean) for each gene. This is the baseline reading for the gene from which all other samples will be compared. X is a stringency variable selected by the person formulating the portfolio. Higher values of X are more stringent than lower. Preferably, X is in the range of .5 to 3 with 2 to 3 being more preferred and 3 being most preferred.

Ratios between each experimental sample (those displaying the condition of interest) versus baseline readings are then calculated. The ratios are then transformed to base 10 logarithmic values for ease of data handling by the software. This enables down regulated genes to display negative values necessary for optimization according to the Markman mean-variance algorithm using the Wagner Software.

The preprocessed data comprising these transformed ratios are used as inputs in place of the asset return values that are normally used in the Wagner Software when it is used for financial analysis purposes.

Once an efficient frontier is formulated, an optimized portfolio is selected for a given input level (return) or variance that corresponds to a point on the frontier. These inputs or variances are the predetermined standards set by the person formulating the portfolio. Stated differently, one seeking the optimum portfolio determines an acceptable input level (indicative of sensitivity) or a given level of variance (indicative of specificity) and selects the genes that lie along the efficient frontier that correspond to that input level or variance. The Wagner Software can select such genes when an input level or variance is selected. It can also assign a weight to each gene in the portfolio as it would for a stock in a stock portfolio.

Determining whether a sample has the condition for which the portfolio is diagnostic can be conducted by comparing the expression of the genes in the portfolio for the patient sample with calculated values of differentially expressed genes used to establish the portfolio. Preferably, a portfolio value is first generated by summing the multiples of the intensity value of each gene in the portfolio by the weight assigned to that gene in the portfolio selection process. A boundary value is then calculated by (Y*standard deviation + mean of the portfolio value for baseline groups) where Y is a stringency value having the same meaning as X described above. A sample having a portfolio value greater than the portfolio value of the baseline class is then classified as having the condition. If desired, this process can be conducted iteratively in accordance with well known statistical methods for improving confidence levels.

Optionally one can reiterate this process until best prediction accuracy is obtained.

The process of portfolio selection and characterization of an unknown is summarized as follows:

1. Choose baseline class.
2. Calculate mean, and standard deviation of each gene for baseline class samples.
3. Calculate (X*Standard Deviation + Mean) for each gene. This is the baseline reading from which all other samples will be compared. X is a stringency variable with higher values of X being more stringent than lower.
4. Calculate ratio between each Experimental sample versus baseline reading calculated in step 3.
5. Transform ratios such that ratios less than 1 are negative (eg.using Log base 10). (Down regulated genes now correctly have negative values necessary for MV optimization).
6. These transformed ratios are used as inputs in place of the asset returns that are normally used in the software application.
7. The software will plot the efficient frontier and return an optimized portfolio at any point along the efficient frontier.
8. Choose a desired return or variance on the efficient frontier.
9. Calculate the Portfolio's Value for each sample by summing the multiples of each gene's intensity value by the weight generated by the portfolio selection algorithm.
10. Calculate a boundary value by adding the mean Portfolio Value for Baseline groups to the multiple of Y and the Standard Deviation of the Baseline's Portfolio Values. Values greater than this boundary value shall be classified as the Experimental Class.
11. Optionally one can reiterate this process until best prediction accuracy is obtained.

Alternatively, genes can first be pre-selected by identifying those genes whose expression shows some minimal level of differentiation. The pre-selection in this alternative method is preferably based on a threshold given by

$$1 \leq \left|\frac{(\mu_t - \mu_n)}{(\sigma_t + \sigma_n)}\right|,$$

where $\mu_t$ is the mean of the subset known to possess the disease or condition, $\mu_n$ is the mean of the subset of normal samples, and $\sigma_t + \sigma_n$ represent the combined standard deviations. A signal to noise cutoff can also be used by pre-selecting the data according to a relationship such as

$$0.5 \leq \left|\frac{(\mu_t - MAX_n)}{(\sigma_t + \sigma_n)}\right|.$$

This ensures that genes that are pre-selected based on their differential modulation are differentiated in a clinically significant way. That is, above the noise level of instrumentation appropriate to the task of measuring the diagnostic parameters. For each marker pre-selected according to these criteria, a matrix is established in which columns represents samples, rows represent markers and each element is a normalized intensity measurement for the expression of that marker according to the relationship:

$$\left|\frac{(\mu_t - I)}{\mu_t}\right|$$

where I is the intensity measurement.

It is also possible to set additional boundary conditions to define the optimal portfolios. For example, portfolio size can be limited to a fixed range or number of markers. This can be done either by making data pre-selection criteria more stringent (e.g,

$$.8 \leq \left|\frac{(\mu_t - MAX_n)}{(\sigma_t + \sigma_n)}\right|$$

instead of

$$0.5 \leq \left|\frac{(\mu_t - MAX_n)}{(\sigma_t + \sigma_n)}\right|$$

or by using programming features such as restricting portfolio size. One could, for example, set the boundary condition that the efficient frontier is to be selected from among only the most optimal 10 genes. One could also use all of the genes pre-selected for determining the efficient frontier and then limit the number of genes selected (e.g., no more than 10).

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with breast cancer. Output from the method would be an optimized set of genes that could include some genes that are expressed in peripheral blood as well as in diseased breast tissue. If sample used in the testing method are obtained from peripheral blood and certain genes differentially expressed in instances of breast cancer could also be differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data pre-selection.

Other heuristic rules can be applied that are not necessarily related to the biology in question. For example, one can apply the rule that only a given percentage of the portfolio can be represented by a particular gene or genes. Commercially available software such as the Wagner Software readily accommodates these types of heuristics. This can be useful, for example, when factors other than accuracy and precision (e.g., anticipated licensing fees) have an impact on the desirability of including one or more genes.

One method of the invention involves comparing gene expression profiles for various genes (or portfolios) to conduct diagnoses as described above. The gene expression profiles of each of the genes comprising the portfolio are fixed in a medium such as a computer readable medium. This can take a number of forms. For example, a table can be established into which the range of signals (e.g., intensity measurements) indicative of disease is input. Actual patient data can then be compared to the values in the table to determine whether the patient samples are normal or diseased. In a more sophisticated embodiment, patterns of the expression signals (e.g., flourescent intensity) are recorded digitally or graphically. The gene expression patterns from the gene portfolios used in conjunction with patient samples are then compared to the expression patterns. Pattern comparison software can then be used to determine whether the patient samples have a pattern indicative of the disease in question. Of course, these comparisons can also be used to determine whether the patient results are normal. The expression profiles of the samples are then compared to the portfolio of a normal or control cell. If the sample expression patterns are consistent with the expression pattern for a colorectal cancer then (in the absence of countervailing medical considerations) the patient is diagnosed as positive for colorectal cancer. If the sample expression patterns are consistent with the expression pattern from the normal/control cell then the patient is diagnosed negative for colorectal cancer.

Numerous well known methods of pattern recognition are available. The following references provide some examples:

<u>Weighted Voting:</u> Golub, TR., Slonim, DK., Tamaya, P., Huard, C., Gaasenbeek, M., Mesirov, JP., Coller, H., Loh, L., Downing, JR., Caligiuri, MA., Bloomfield, CD., Lander, ES. *Molecular classification of cancer: class discovery and class prediction by gene expression monitoring.* Science 286:531-537, 1999

<u>Support Vector Machines:</u> Su, AI., Welsh, JB., Sapinoso, LM., Kern, SG., Dimitrov, P., Lapp, H., Schultz, PG., Powell, SM., Moskaluk, CA., Frierson, HF. Jr., Hampton, GM. *Molecular classification of human carcinomas by use of gene expression signatures.* Cancer Research 61:7388-93, 2001 and

Ramaswamy, S., Tamayo, P., Rifkin, R., Mukherjee, S., Yeang, CH., Angelo, M., Ladd, C., Reich, M., Latulippe, E., Mesirov, JP., Poggio, T., Gerald, W., Loda, M., Lander, ES., Gould, TR. *Multiclass cancer diagnosis using tumor gene expression signatures* Proceedings of the National Academy of Sciences of the USA 98:15149-15154, 2001

K-nearest Neighbors: Ramaswamy, S., Tamayo, P., Rifkin, R., Mukherjee, S., Yeang, CH., Angelo, M., Ladd, C., Reich, M., Latulippe, E., Mesirov, JP., Poggio, T., Gerald, W., Loda, M., Lander, ES., Gould, TR. *Multiclass cancer diagnosis using tumor gene expression signatures* Proceedings of the National Academy of Sciences of the USA 98:15149-15154, 2001

Correlation Coefficients: van 't Veer LJ, Dai H, van de Vijver MJ, He YD, Hart AA, Mao M, Peterse HL, van der Kooy K, Marton MJ, Witteveen AT, Schreiber GJ, Kerkhoven RM, Roberts C, Linsley PS, Bernards R, Friend SH. Gene expression profiling predicts clinical outcome of breast cancer. Nature. 2002 Jan 31;415(6871):530-6.

The gene expression profiles of this invention can also be used in conjunction with other non-genetic diagnostic methods useful in cancer diagnosis, prognosis, or treatment monitoring. For example, in some circumstances it is beneficial to combine the diagnostic power of the gene expression based methods described above with data from conventional markers such as serum protein markers (e.g., carcinoembryonic antigen). A range of such markers exists including such analytes as CA19-9, CA 125, CK-BB, and Guanylyl Cyclase C. In one such method, blood is periodically taken from a treated patient and then subjected to an enzyme immunoassay for one of the serum markers described above. When the concentration of the marker suggests the return of tumors or failure of therapy, a sample source amenable to gene expression analysis is taken. Where a suspicious mass exists, a fine needle aspirate is taken and gene expression profiles of cells taken from the mass are then analyzed as described above. Alternatively, tissue samples may be taken from areas adjacent to the tissue from which a tumor was previously removed. This approach can be particularly useful when other testing produces ambiguous.

Combining the use of genetic markers with other diagnostics is most preferred when the reliability of the other diagnostic is suspect. For example, it is known that serum levels of CEA can be substantially affected by factors having nothing to do with a patient's cancer status. It can be beneficial to conduct a combination gene expression/CEA assay when a patient being monitored following treatment for colon cancer shows heightened levels of routine CEA assays.

Articles of this invention include representations of the gene expression profiles useful for treating, diagnosing, prognosticating, and otherwise assessing diseases. These profile representations are reduced to a medium that can be automatically read by a machine such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Clustering algorithms such as those incorporated in "GENESPRING" and "DISCOVER" computer programs mentioned above can best assist in the visualization of such data.

Different types of articles of manufacture according to the invention are media or formatted assays used to reveal gene expression profiles. These can comprise, for example, microarrays in which sequence complements or probes are affixed to a matrix to which the sequences indicative of the genes of interest combine creating a readable determinant of their presence. Alternatively, articles according to the invention can be fashioned into reagent kits for conducting hybridization, amplification, and signal generation indicative of the level of expression of the genes of interest for detecting colorectal cancer.

Kits made according to the invention include formatted assays for determining the gene expression profiles. These can include all or some of the materials needed to conduct the assays such as reagents and instructions.

The invention is further illustrated by the following non-limiting examples.

**Examples:** Genes analyzed according to this invention are identified by reference to Gene ID Numbers in the GenBank database. These are typically related to full-length nucleic acid sequences that code for the production of a protein or peptide. One skilled in the art will recognize that identification of full-length sequences is not necessary from an analytical point of view. That is, portions of the sequences or ESTs can be selected according to well-known principles for which probes can be designed to assess gene expression for the corresponding gene.

**Example 1- Sample Handling and LCM.**

Twenty-seven fresh frozen tissue samples were collected from patients who had surgery for a colorectal tumor. Nineteen of the samples were colorectal malignancy specimens, and eight of the samples were of normal colon mucosa. The tissues were snap frozen in liquid nitrogen within 20-30 minutes of harvesting, and stored at -80C° thereafter. For laser capture, the samples were cut (6μm), and one section was mounted on a glass slide, and the second on film (P.A.L.M.), which had been fixed onto a glass slide (Micro Slides Colorfrost, VWR Scientific, Media, PA). The section mounted on a glass slide was after fixed in cold acetone, and stained with Mayer's Haematoxylin (Sigma, St. Louis, MO). A pathologist analyzed the samples for diagnosis and grade. The clinical stage was estimated from the accompanying surgical pathology and clinical reports, using the Dukes classification. The section mounted on film was after fixed for five minutes in 100% ethanol, counter stained for 1 minute in eosin/100% ethanol (100μg of Eosin in 100ml of dehydrated ethanol), quickly soaked once in 100% ethanol to remove the free stain, and air dried for 10 minutes.

Two of the colorectal adenocarcinomas were of grade 1, 10 of grade 2, and 5 of grade 3. One of the malignant samples was a carcinoid tumor of the caecum, and one a metastatic melanoma lesion. Two of the adenocarcinoma samples represented the mucinous subtype, and one the signet cell subtype. The Dukes staging of the adenocarcinomas divided them as follows: Dukes A: 2, Dukes B: 5, Dukes C: 7, Dukes D: 3. Six of the adenocarcinomas had been irradiated preoperatively.

Before use in LCM, the membrane (LPC-MEMBRANE PEN FOIL 1.35 μm No 8100, P.A.L.M. GmbH Mikrolaser Technologie, Bernried, Germany) and slides were pretreated to abolish RNases, and to enhance the attachment of the tissue sample onto the film. Briefly, the slides were washed in DEP $H_2O$, and the film was washed in RNase AWAY (Molecular Bioproducts, Inc., San Diego, CA) and rinsed in DEP $H_2O$. After attaching the film onto the glass slides, the slides were baked at +120°C for 8 hours, treated with TI-SAD (Diagnostic Products Corporation, Los Angeles, CA, 1:50 in DEP $H_2O$, filtered through cotton wool), and incubated at +37°C for 30 minutes. Immediately before use, a 10μl aliquot of RNase inhibitor solution (Rnasin Inhibitor 2500U=33U/μl N211A, Promega GmbH, Mannheim, Germany, 0.5μl in 400μl of freezing solution, containing 0.15 mol NaCl, 10 mmol Tris pH 8.0, 0.25 mmol dithiothreitol) was spread onto the film, where the tissue sample was to be mounted.

The tissue sections mounted on film were used for LCM. Approximately 2000 epithelial cells/sample were captured using the PALM Robot-Microbeam technology (P.A.L.M. Mikrolaser Technologie, Carl Zeiss, Inc., Thornwood, NY), coupled into Zeiss Axiovert 135 microscope (Carl Zeiss Jena GmbH, Jena, Germany). The surrounding stroma in the normal mucosa, and the occasional intervening stromal components in cancer samples, were included. The captured cells were put in tubes in 100% ethanol and preserved at -80°C.

**Example 2- RNA Extraction and Amplification.**

Zymo-Spin Column (Zymo Research, Orange, CA 92867) was used to extract total RNA from the LCM captured samples. About 2 ng of total RNA was resuspended in 10 ul of water and 2 rounds of the T7 RNA polymerase based amplification were performed to yield about 50 ug of amplified RNA.

**Example 3- cDNA Microarray Hybridization and Quantitation.**

A set of cDNA microarrays consisting of approximately 20,000 human cDNA clones was used to test the samples. About 30 plant genes were also printed on the microarrays as a control for non-specific hybridization. Cy3-labeled cDNA probes were synthesized from 5 ug of aRNA of the LCM captured cells. The probes were purified with Qiagen's Nucleotide Removal Columns and then hybridized to the microarrays for 14-16 hours. The slides were washed and air-dried before scanning. cDNA microarrays were scanned for cy3 fluorescence and ImaGene software (Biodiscovery, Los Angeles, CA) was used for quantitation. For each cDNA clone, four measurements were obtained using duplicate spots and duplicate arrays and the intensities were averaged.

cDNAs were printed on amino silane-coated slides (Coming) with a Generation III Micro-array Spotter (Molecular Dynamics). The cDNAs were PCR amplified, purified (Qiagen PCR purification kit), and mixed 1:1 with 10 M NaSCN printing buffer. Prior to hybridization micro-arrays were incubated in isopropanol at room temperature for 10 min. The probes were incubated at 95°C for 2 min, at room temperature for 5 min, and then applied to three replicate slides. Cover slips were sealed onto the slides with DPX (Fluka) and incubated at 42°C overnight. Slides were then washed at 55°C for 5min in 1X SSC/0.2% SDS and 0.1X SSC/0.2% SDS, dipped in 0.1X SSC and dried before being scanned by a GenIII Array Scanner (Molecular Dynamics). The fluorescence intensity for each spot was analyzed with AUTOGENE software (Biodiscovery, Los Angeles).

Chip intensities were linearly normalized forcing the intensity reading at the $75^{th}$ percentile equivalent to a value of 100 on each chip. Every gene on the chip was normalized to itself by dividing the intensity reading for that gene by the median of the gene's expression value readings over all the samples. Prior to clustering, genes that did not have an intensity reading of 100 or greater in at least one sample were filtered out in order to limit the background affect on the similarity metrics. A set of 6,225 genes was selected for clustering analysis. Hierarchical clustering was performed using correlation as ) a measure of similarity, which groups together samples with genes that are showing positive changes at the same time without any consideration for negative changes (Silicon Genetics, Sunnyville, CA). Each of the major nodes in the dendrogram was then considered a subgroup of samples. Differentially expressed genes were identified by comparing each tumor subgroup to the normal group. The selection was based on a signal to noise measurement threshold given by

$$1 \leq \left|\frac{(\mu_t - \mu_n)}{(\sigma_t + \sigma_n)}\right|,$$

where $\mu_t$ is the mean of the tumor subset, $\mu_n$ is the mean of the subset of normal samples, and $\sigma_t + \sigma_n$ represent the combined standard deviations. The within-group coefficient of variation of the intensity readings of a gene had to be less than 0.33, for the gene to be included in the pair-wise comparisons. The median of the tumor group over the median of the normal group had to ) be greater than, or equal to 2 for up-regulation, and less than, or equal to 0.5 for down-regulation. If a gene met all the criteria, it was selected. The genes selected in all the comparisons were considered consistently dysregulated in colorectal cancer. The p-values for the statistical significance were calculated using a T-test assuming unequal variance. The gene set for clustering was also subjected to principal component analysis (PCA) using a software package (Partek, St Louis, MO). The data was then projected onto the reduced 3-dimensional space. The normal and tumor colorectal samples were represented by the projected expression levels.

A list of genes with large up-regulated differentials was created to distinguish between the tumor and normal samples. One-hundred and twenty-three genes were preselected by using

$$0.5 \leq \left|\frac{(\mu_t - MAX_n)}{(\sigma_t + \sigma_n)}\right|$$

as a signal to noise cutoff. A ratio equal to, or greater than 1.5 was the minimal criterion for up-regulation. Genes were also included if

$$0.9 \leq \left|\frac{(\mu_t - \mu_n)}{(\sigma_t + \sigma_n)}\right|.$$

A portfolio of four genes was established, each having at least a three fold expression differential between tumor and normal cells.

Differentially Expressed Genes in Colorectal Cancer. Thirty-nine genes were differentially expressed in all tumor samples as compared to normal colon mucosa. Thirty-seven of them were significantly down-regulated in all the tumors, except for an outlier. Two of them were up-regulated. The identities of the genes were verified by sequencing the cDNA clones placed on the microarray. Results are shown in Table 1.

Table 1

| Modulated Genes | | | | | |
|---|---|---|---|---|---|
| **ACCESSION** | **GENE DESCRIPTION** | **MEAN SIGNAL INTENSITY (NORMAL)** | **MEAN SIGNAL INTENSITY (TUMOR)** | **P-VALUE** | |
| AF071569 | CaM kinase II gene subtype delta 2. | 93 | 39 | 4.64E-09 | Seq. ID No. 1 |
| AB014530 | Homo sapiens mRNA for KIAA0630 protein | 108 | 50 | 4.83E-07 | Seq. ID No.2 |
| AK000319 | Human cDNA KIAA0630 | 236 | 69 | 7.84E-06 | Seq. ID No.3 |
| U81504 | beta-3A-adaptin subunit of the AP-3 complex mRNA, | 241 | 75 | 3.52E-05 | Seq. ID No.4 |
| AB011166 | Human cDNA KIAA0594 | 116 | 55 | 3.53E-05 | Seq. ID No.5 |
| AB040914 | Human cDNA KIAA1481 | 187 | 59 | 8.85E-05 | Seq. ID No.6 |

Table 1 (continued)

| Modulated Genes | | | | | |
|---|---|---|---|---|---|
| **ACCESSION** | **GENE DESCRIPTION** | **MEAN SIGNAL INTENSITY (NORMAL)** | **MEAN SIGNAL INTENSITY (TUMOR)** | **P-VALUE** | |
| AK025205 | Human cDNA FLJ21552 | 322 | 97 | 0.00013 | Seq. ID No.7 |
| AJ278219 | Fatty acid hydroxylase | 143 | 53 | 0.00011 | Seq. ID No.8 |
| AB046854 | Human cDNA KIAA1634 | 142 | 59 | 0.00020 | Seq. ID No.9 |
| R00585 | Unknown | 149 | 57 | 1.28E-09 | Seq. ID No.10 |
| S45844 | Spi-B transcription factor | 140 | 43 | 0.00043 | Seq. ID No.11 |
| X98311 | Carcinoembryonic antigen family member 2 (CGM2) | 6137 | 223 | 0.00044 | Seq. ID No.12 |
| BAA78050 | NADPH oxidoreductase homolog | 153 | 84 | 0.00048 | Seq. ID No.40 |
| N72128 | Unknown | 164 | 77 | 0.00068 | Seq. ID No.13 |
| AB040955 | Human cDNA KIAA1552 | 334 | 120 | 0.00067 | Seq. ID No.14 |
| AF125101 | HSPC040 protein | 363 | 115 | 0.0011 | Seq. ID No.15 |
| AB023229 | Human cDNA KIAA1012 | 263 | 88 | 0.00099 | Seq. ID No.16 |
| N95761 | a-L-fucosidase gene | 429 | 104 | 0.00047 | Seq. ID No.17 |
| AK025033 | Human cDNA FLJ21380 | 180 | 85 | 0.0010 | Seq. ID No.18 |
| L10844 | Human cellular growth regulating protein | 206 | 101 | 0.0013 | Seq. ID No.19 |
| H96534 | H.sapiens mRNA for gp25L2 protein. | 147 | 58 | 0.0015 | Seq. ID No.20 |
| AK001521 | Human cDNA FLJ10659 | 157 | 60 | 0.0019 | Seq. ID No.21 |
| AF151039 | HSPC205 protein | 117 | 60 | 0.0017 | Seq. ID No.22 |
| AF052059 | SEL 1L protein | 168 | 53 | 0.0016 | Seq. ID No.23 |
| N24597 | Unknown | 166 | 62 | 0.0016 | Seq. ID No.24 |
| AK001950 | Inner centromere protein | 148 | 64 | 0.0029 | Seq. ID No.25 |
| BAA02649 | Macrophage scavenger receptor type I | 118 | 44 | 0.0031 | Seq. ID No.41 |
| N75004 | Unknown | 98 | 48 | 0.0031 | Seq. ID No.26 |

Table 1 (continued)

| Modulated Genes | | | | | |
|---|---|---|---|---|---|
| **ACCESSION** | **GENE DESCRIPTION** | **MEAN SIGNAL INTENSITY (NORMAL)** | **MEAN SIGNAL INTENSITY (TUMOR)** | **P-VALUE** | |
| W16916 | Human cDNA KIAA0260 | 162 | 61 | 0.0037 | Seq. ID No.27 |
| X52001 | H.sapiens endothelin 3 mRNA. | 89 | 33 | 0.0042 | Seq. ID No.28 |
| T50788 | Unknown | 364 | 102 | 0.0059 | Seq. ID No.38 |
| AJ005866 | Putative Sqv-7 like protein | 381 | 163 | 0.0049 | Seq. ID No.29 |
| AF113535 | MAID protein | 218 | 100 | 0.0053 | Seq. ID No.39 |
| AB037789 | Human cDNA KIAA1368 | 164 | 62 | 0.0068 | Seq. ID No.30 |
| M33987 | Carbonic anhydrase | 652 | 46 | 0.0074 | Seq. ID No.31 |
| M77830 | Desmoplakin 1 (DPI) | 184 | 81 | 0.0092 | Seq. ID No.32 |
| H81220 | EST domain tanscription factor ELF1 | 113 | 55 | 0.017 | Seq. ID No.33 |
| AF000592 | Human chromosome 21q11-q21 genomic clone | 33 | 69 | 1.16E-05 | Seq. ID No.35 |
| AK021701 | Human cDNA FLJ11639 | 31 | 63 | 0.00070 | Seq. ID No.36 |

**Example 4: Optimized Portfolio for Colorectal Tumors.**

The mean-variance optimization algorithm was used to generate a multiple genebased signature, where the genes that are included can be used in combination to distinguish between the normal and tumor samples. Intensity measurements were processed using the samples and microarrays described in Examples 1-3. The data to be analyzed was first preselected based on a pre-specified 5-fold differential between tumor and normal cells. The expression data from genes preselected according to this criteria were then used as follows. The mean and standard deviation of the intensity measurements for each gene were calculated using the non-metastatic samples as the baseline. A discriminating value of X*(Standard Deviation + Mean) was then calculated for each baseline gene ( X was assigned a value of 3). This value was used to ensure the resulting portfolio would be stringent. A ratio of the discriminating value to the baseline value was then calculated for each metastatic sample. This ratio was then converted to a common logarithm. This data was then imported into Wagner Software which produced an efficient frontier from which a portfolio of 4 genes was selected. The set included an unknown sequence, procollagen type I, large subunit of ribosomal protein L21 and fibronectin. These genes are identified as Seq. ID No 42, Seq. ID No. 43, Seq. ID No. 44 and Seq. ID No. 45. Alternatively, a combination of genes used to make up the portfolio can be used to produce diagnostic information that is useful for making clinical decisions regarding colorectal cancer. This is particularly beneficial in the case when a combination of genes selected from the portfolio are combined with additional markers (genetic or not).

Optimized Gene Portfolio:

>gi|1264443|gb|N92134.1|N92134 za23f09.r1 Soares fetal liver spleen 1NFLS Homo sapiens cDNA clone IMAGE: 293417 5' similar to gb|M87908|HUMALNE32 Human carcinoma cell-derived Alu RNA transcript, (rRNA); gb: X57025_mal INSULIN-LIKE GROWTH FACTOR IA PRECURSOR (HUMAN)
>gi|2221047|gb|AA490172.1|AA490172 ab06b08.s1 Stratagene fetal retina 937202 Homo sapiens cDNA clone IMAGE:839991 3' similar to gb:J03464 PROCOLLAGEN ALPHA 2(I) CHAIN PRECURSOR (HUMAN)
>gi|2188918|gb|AA464034.1|AA464034 zx86b09.r1 Soares ovary tumor NbHOT Homo sapiens cDNA clone IMAGE:810617 5' similar to SW:RL21_HUMAN P46778 60S RIBOSOMAL PROTEIN L21.
>gi|834491|gb|R62612.1|R62612 yi12d01.s1 Soares placenta Nb2HP Homo sapiens cDNA clone IMAGE:139009 3' similar to gb:X02761_cds1 FIBRONECTIN PRECURSOR (HUMAN);

Using a different set of criteria but the same method, a further four gene portfolio was selected by the software. These are Seq. ID no. 46, Seq. ID No. 47, Seq. ID No. 48 and Seq. ID No. 49. Two genes overlap with the first four-gene portfolio. The two optimized portfolios can also be combined to form a six-gene portfolio.

Optimized Gene Portfolio:

>gi|2114953|gb|AA431245.1|AA431245 zw78d06.r1 Soares_testis_NHT Homo sapiens cDNA clone IMAGE: 782315 5' similar to WP:F36H1.2 CE05814 ANKYRIN LIKE
>gi|2156172|gb|AA443497.1|AA443497 zw34d03.r1 Soares ovary tumor NbHOT Homo sapiens cDNA clone IMAGE:771173
>gi|2221047|gb|2|AA490172 ab06b08.s1 Stratagene fetal retina 937202 Homo sapiens cDNA clone IMAGE: 839991 3' similar to gb:J03464 PROCOLLAGEN ALPHA 2(I) CHAIN PRECURSOR (HUMAN)
>gi|1264443|gb|N92134.1|N92134 za23f09.r1 Soares fetal liver spleen 1NFLS Homo sapiens cDNA clone IMAGE: 293417 5' similar to gb|M87908|HUMALNE32 Human carcinoma cell-derived Alu RNA transcript, (rRNA); gb: X57025_rnal INSULIN-LIKE GROWTH FACTOR IA PRECURSOR (HUMAN);

## Claims

1. A method of assessing colorectal cancer status comprising identifying differential modulation of each gene (relative to the expression of the same genes in a normal population) in a combination of genes selected from the group consisting of Seq. ID. No. 42-49.

**2.** The method of claim 1 wherein there is at least a 2 fold difference in the expression of the modulated genes.

**3.** The method of claim 1 wherein the p-value indicating differential modulation is less than .05.

**4.** The method of claim 1 further comprising employing a colorectal diagnostic that is not genetically based.

**5.** The method of claim wherein the cancer marker that is not genetically based is selected from the group consisting of carcinomebryonic antigen, CA19-9, CA 125, CK-BB, and Guanylyl Cyclase C.

**6.** A diagnostic portfolio comprising isolated nucleic acid sequences, their complements, or portions thereof of a combination of genes selected from the group consisting of Seq. ID. No. 42-49.

**7.** The diagnostic portfolio of claim 6 in a matrix suitable for identifying the differential expression of the genes contained therein.

**8.** The diagnostic portfolio of claim 7 wherein said matrix is employed in a microarray.

**9.** The diagnostic portfolio of claim 8 wherein said microarray is a cDNA microarray.

**10.** The diagnostic portfolio of claim 8 wherein said microarray is an oligonucleotide microarray.

**11.** A diagnostic portfolio comprising isolated nucleic acid sequences, their complements, or portions thereof of a combination of genes selected from the group consisting of Seq. ID. No. 42-49.

**12.** A kit for diagnosing colorectal cancer comprising isolated nucleic acid sequences, their compliments, or portions thereof of a combination of genes selected from the group consisting of Seq. ID. No. 42-49.

**13.** The kit of claim 12 further comprising reagents for conducting a microarray analysis.

**14.** The kit of claim 12 further comprising a medium through which said nucleic acid sequences, their complements, or portions thereof are assayed.

**15.** A method of assessing response to treatment for colorectal cancer comprising identifying differential modulation of each gene (relative to the expression of the same genes in a normal population) in a combination of genes selected from the group consisting of Seq. ID. No. 42-49.

**16.** The method of claim 15 wherein the assessment of the response to therapy includes a determination of whether the patient is improving, not improving, relapsing, likely to improve, or likely to relapse.

**17.** Articles for assessing colorectal cancer statuts comprising isolated nucleic acid sequences, their complements, or portions thereof of a combination of genes selected from the group consisting of Seq. ID. No. 42-49.

**18.** Articles for assessing colorectal cancer status comprising representations of isolated nucleic acid sequences, their complements, or portions thereof of a combination of genes selected from the group consisting of Seq. ID. No. 42-49.

SEQUENCE LISTING

<110> WANG, YIXIN

<120> COLORECTAL CANCER DIAGNOSTICS

<130> CDS 267 US NP

<140> TBD
<141> 2003-03-21

<150> 60/368,798
<151> 2002-03-29

<160> 49

<170> PatentIn version 3.1

<210> 1
<211> 1500
<212> DNA
<213> human

<400> 1
atggcttcga ccaccacctg caccaggttc acggacgagt atcagctttt cgaggagctt
60

ggaaaggggg cattctcagt ggtgagaaga tgtatgaaaa ttcctactgg acaaggatat
120

gctgccaaaa ttatcaacac caaaaagctt tctgctaggg atcatcagaa actagaaaga
180

gaagctagaa tctgccgtct tttgaagcac cctaatattg tgcgacttca tgatagcata
240

tcagaagagg gctttcacta cttggtgttt gatttagtta ctggaggtga actgtttgaa
300

gacatagtgg caagagaata ctacagtgaa gctgatgcca gtcattgtat acagcagatt
360

ctagaaagtg ttaatcattg tcacctaaat ggcatagttc acagggacct gaagcctgag
420

aatttgcttt tagctagcaa atccaaggga gcagctgtga aattggcaga ctttggctta
480

gccatagaag ttcaagggga ccagcaggcg tggtttggtt ttgctggcac acctggatat
540

ctttctccag aagttttacg taaagatcct tatggaaagc cagtggatat gtgggcatgt
600

ggtgtcattc tctatattct acttgtgggg tatccaccct tctgggatga agaccaacac
660

agactctatc agcagatcaa ggctggagct tatgattttc catcaccaga atgggacacg
720

gtgactcctg aagccaaaga cctcatcaat aaaatgctta ctatcaaccc tgccaaacgc
780

atcacagcct cagaggcact gaagcaccca tggatctgtc aacgttctac tgttgcttcc
840

atgatgcaca gacaggagac tgtagactgc ttgaagaaat ttaatgctag aagaaaacta
900

aagggtgcca tcttgacaac tatgctggct acaaggaatt tctcagcagc caagagtttg
960

ttgaagaaac cagatggagt aaaggagtca actgagagtt caaatacaac aattgaggat
1020

gaagatgtga aagcacgaaa gcaagagatt atcaaagtca ctgaacaact gatcgaagct
1080

atcaacaatg ggactttga agcctacaca aaaatctgtg acccaggcct tactgctttt
1140

gaacctgaag ctttgggtaa tttagtggaa gggatggatt ttcaccgatt ctactttgaa
1200

aatgctttgt ccaaaagcaa taaaccaatc cacactatta ttctaaaccc tcatgtacat
1260

ctggtagggg atgatgccgc ctgcatagca tatattaggc tcacacagta catggatggc
1320

agtggaatgc caaagacaat gcagtcagaa gagactcgtg tgtggcaccg ccgggatgga
1380

aagtggcaga atgttcattt tcatcgctcg ggtcaccaa cagtacccat caagccaccc
1440

tgtattccaa atgggaaaga aaacttctca ggaggcacct ctttgtggca aaacatctga
1500

<210> 2
<211> 5761
<212> DNA
<213> human

<400> 2
cacaccgcag tatgcggtgc cctttactct gagctgcgca gccggccggc cggcgctggt
60

tgaacagact gccgctgtac tggcgtggcc tggagggact cagcaaattc tcctgccttc
120

aacttggcaa cagttgcctg ggtagctct acacaactct gtccagccca cagcaatgat
180

tccagaggcc atggggagtg gacagcagct agctgactgg aggaatgccc actctcatgg
240

caaccagtac agcactatca tgcagcagcc atccttgctg actaaccatg tgacattggc
300

cactgctcag cctctgaatg ttggtgttgc ccatgttgtc agacaacaac aatccagttc
360

cctcccttcg aagaagaata agcagtcagc tccagtctct tccaagtcct ctctagatgt
420

tctgccttcc caagtctatt ctctggttgg gagcagtccc ctccgcacca catcttctta
480

taattccttg gtccctgtcc aagatcagca tcagcccatc atcattccag atactcccag
540

ccctcctgtg agtgtcatca ctatccgaag tgacactgat gaggaagagg acaacaaata 600

caagcccagt agctctggac tgaagccaag gtctaatgtc atcagttatg tcactgtcaa 660

tgattctcca gactctgact cttctttgag cagcccttat tccactgata ccctgagtgc 720

tctccgaggc aatagtggat ccgttttgga ggggcctggc agagttgtgg cagatggcac 780

tggcacccgc actatcattg tgcctccact gaaaactcag cttggtgact gcactgtagc 840

aacccaggcc tcaggtctcc tgagcaataa gactaagcca gtcgcttcag tgagtgggca 900

gtcatctgga tgctgtatca cccccacagg gtatcgagct caacgcgggg ggaccagtgc 960

agcacaacca ctcaatctta gccagaacca gcagtcatcg gcggctccaa cctcacagga 1020

gagaagcagc aacccagccc cccgcaggca gcaggcgttt gtggcccctc tctcccaagc 1080

cccctacacc ttccagcatg gcagcccgct acactcgaca gggcacccac accttgcccc 1140

ggcccctgct cacctgccaa gccaggctca tctgtatacg tatgctgccc cgacttctgc 1200

tgctgcactg ggctcaacca gctccattgc tcatcttttc tccccacagg gttcctcaag 1260

gcatgctgca gcctatacca ctcaccctag cactttggtg caccaggtcc ctgtcagtgt 1320

tgggcccagc ctcctcactt ctgccagcgt ggcccctgct cagtaccaac accagtttgc 1380

cacccaatcc tacattgggt cttcccgagg ctcaacaatt tacactggat acccgctgag 1440

tcctaccaag atcagccagt attcctactt atagttggtg agcatgaggg aggaggaatc 1500

atggctacct tctcctggcc ctgcgttctt aatattgggc tatggagaga tcctccttta 1560

ccctcttgaa atttcttagc cagcaacttg ttctgcaggg gcccactgaa gcagaaggtt 1620

tttctctggg ggaacctgtc tcagtgttga ctgcattgtt gtagtcttcc caaagtttgc 1680

cctattttta aattcattat ttttgtgaca gtaattttgg tacttggaag agttcagatg 1740

cccatcttct gcagttacca aggaagagag attgttctga agttaccctc tgaaaaatat 1800

tttgtctctc tgacttgatt tctataaatg cttttaaaaa caagtgaagc ccctctttat 1860

ttcattttgt gttattgtga ttgctggtca ggaaaaatgc tgatagaagg agttgaaatc
1920

tgatgacaaa aaaagaaaaa ttactttttg tttgtttata aactcagact tgcctatttt
1980

attttaaaag cggcttacac aatctccctt ttgtttattg gacatttaaa cttacagagt
2040

ttcagttttg ttttaatgtc atattatact taatgggcaa ttgttatttt tgcaaaactg
2100

gttacgtatt actctgtgtt actattgaga ttctctcaat tgctcctgtg tttgttataa
2160

agtagtgttt aaaaggcagc tcaccatttg ctggtaactt aatgtgagag aatccatatc
2220

tgcgtgaaaa caccaagtat tctttttaaa tgaagcacca tgaattcttt tttaaattat
2280

tttttaaaag tctttctctc tctgattcag cttaaatttt tttatcgaaa aagccattaa
2340

ggtggttatt attacatggt ggtggtggtt ttattatatg caaaatctct gtctattatg
2400

agatactggc attgatgagc tttgcctaaa gattagtatg aattttcagt aatacacctc
2460

tgttttgctc atctctccct tctgttttat gtgatttgtt tggggagaaa gctaaaaaaa
2520

cctgaaacca gataagaaca tttcttgtgt atagctttta tacttcaaag tagcttcctt
2580

tgtatgccag cagcaaattg aatgctctct tattaagact tatataataa gtgcatgtag
2640

gaattgcaaa aaatatttta aaaatttatt actgaattta aaaatatttt agaagttttg
2700

taatggtggt gttttaatat tttacataat taaatatgta catattgatt agaaaaatat
2760

aacaagcaat ttttcctgct aacccaaaat gttatttgta atcaaatgtg tagtgattac
2820

acttgaattg tgtacttagt gtgtatgtga tcctccagtg ttatcccgga gatggattga
2880

tgtctccatt gtatttaaac caaaatgaac tgatacttgt tggaatgtat gtgaactaat
2940

tgcaattata ttagagcata ttactgtagt gctgaatgag caggggcatt gcctgcaagg
3000

agaggagacc cttggaattg ttttgcacag gtgtgtctgg tgaggagttt ttcagtgtgt
3060

gtctcttcct tccctttctt cctccttccc ttattgtagt gccttatatg ataatgtagt
3120

ggttaataga gtttacagtg agcttgcctt aggatggacc agcaagcccc cgtggaccct
3180

aagttgttca ccgggattta tcagaacagg attagtagct gtattgtgta atgcattgtt
3240

ctcagtttcc ctgccaacat tgaaaaataa aaacagcagc ttttctcctt taccaccacc
3300

tctacccctt tccattttgg attctcggct gagttctcac agaagcattt tccccatgtg
3360

gctctctcac tgtgcgttgc taccttgctt ctgtgagaat tcaggaagca ggtgagagga
3420

gtcaagccaa tattaaatat gcattctttt aaagtatgtg caatcacttt tagaatgaat
3480

tttttttcc ttttcccatg tggcagtcct tcctgcacat agttgacatt cctagtaaaa
3540

tatttgcttg ttgaaaaaaa catgttaaca gatgtgttta taccaaagag cctgttgtat
3600

tgcttaccat gtccccatac tatgaggaga agttttgtgg tgccgctggt gacaaggaac
3660

tcacagaaag gtttcttagc tggtgaagaa tatagagaag gaaccaaagc ctgttgagtc
3720

attgaggctt ttgaggtttc tttttaaca gcttgtatag tcttggggcc cttcaagctg
3780

tgaaattgtc cttgtactct cagctcctgc atggatctgg gtcaagtaga aggtactggg
3840

gatggggaca ttcctgccca taaaggattt ggggaaagaa gattaatcct aaaatacagg
3900

tgtgttccat ccgaattgaa aatgatatat ttgagatata attttaggac tggttctgtg
3960

tagatagaga tggtgtcaag gaggtgcagg atggagatgg gagatttcat ggagcctggt
4020

cagccagctc tgtaccaggt tgaacaccga ggagctgtca aagtatttgg agtttcttca
4080

ttgtaaggag taagggcttc caagatgggg caggtagtcc gtacagccta ccaggaacat
4140

gttgtgtttt ctttattttt taaaatcatt atattgagtt gtgttttcag cactatattg
4200

gtcaagatag ccaagcagtt tgtataattt ctgtcactag tgtcatacag ttttctggtc
4260

aacatgtgtg atctttgtgt ctccttttg ccaagcacat tctgattttc ttgttggaac
4320

acaggtctag tttctaaagg acaaattttt tgttccttgt cttttttctg taagggacaa
4380

gatttgttgt ttttgtaaga aatgagatgc aggaaagaaa accaaatccc attcctgcac
4440

cccagtccaa taagcagata ccacttaaga taggagtcta aactccacag aaaaggataa
4500

taccaagagc ttgtattgtt accttagtca cttgcctagc agtgtgtggc tttaaaaact
4560

agagattttt cagtcttagt ctgcaaactg gcatttccga ttttccagca taaaaatcca
4620

cctgtgtctg ctgaatgtgt atgtatgtgc tcactgtggc tttagattct gtccctgggg
4680

ttagccctgt tggccctgac aggaagggag gaagcctggt gaatttagtg agcagctggc
4740

ctgggtcaca gtgacctgac ctcaaaccag cttaaggctt taagtcctct ctcagaactt
4800

ggcatttcca acttcttcct ttccgggtga gagaagaagc ggagaagggt tcagtgtagc
4860

cactctgggc tcatagggac acttggtcac tccagagttt ttaatagctc ccaggaggtg
4920

atattatttt cagtgctcag ctgaaatacc aaccccagga ataagaactc catttcaaac
4980

agttctggcc attctgagcc tgcttttgtg attgctcatc cattgtcctc cactagaggg
5040

gctaagcttg actgccctta gccaggcaag cacagtaatg tgtgttttgt tcagcattat
5100

tatgcaaaaa ttcactagtt gagatggttt gttttaggat aggaaatgaa attgcctctc
5160

agtgacagga gtggcccgag cctgcttcct attttgattt tttttttttt taactgatag
5220

atggtgcagc atgtctacat ggttgtttgt tgctaaactt tatataatgt gtggtttcaa
5280

ttcagcttga aaaataatct cactacatgt agcagtacat tatatgtaca ttatatgtaa
5340

tgttagtatt tctgctttga atccttgata ttgcaatgga attcctactt tattaaatgt
5400

atttgatatg ctagttattg tgtgcgattt aaactttttt tgctttctcc cttttttttgg
5460

ttgtgcgctt tcttttacaa caagcctcta gaaacagata gtttctgaga attactgagc
5520

tatgtttgta atgcagatgt acttagggag tatgtaaaat aatcatttta acaaaagaaa
5580

tagatattta aaatttaata ctaactatgg gaaaagggtc cattgtgtaa aacatagttt
5640

atctttggat tcaatgtttg tctttggttt tacaaagtag cttgtatttt cagtattttc
5700

tacataatat ggtaaaatgt agagcaattg caatgcatca ataaaatggg taaattttct
5760

g 5
761

<210> 3
<211> 2129
<212> DNA
<213> human

<400> 3
ctgtattgag acaaaggaag ggatctgtca gaaagcaaca cttgttatct tgggcttggc
60

agcaaggaag aggacaggta gtggagatcc tgcaatctga aaagcagact gaaaggtgac
120

aaagaagctg aagatgggtg gtggagagag gtataacatt ccagcccctc aatctagaaa
180

tgttagtaag aaccaacaac agcttaacag acagaagacc aaggaacaga attcccagat
240

gaagattgtt cataagaaaa aagaaagagg acatggttat aactcatcag cagctgcctg
300

gcaggccatg caaaatgggg ggaagaacaa aaattttcca aataatcaaa gttggaattc
360

tagcttatca ggtcccaggt tactttttaa atctcaagct aatcagaact atgctggtgc
420

caaatttagt gagccgccat caccaagtgt tcttcccaaa ccaccaagcc actgggtccc
480

tgtttccttt aatccttcag ataaggaaat aatgacattt caacttaaaa ccttacttaa
540

agtacaggta taaaataaga caaatgttta aatttagtta tgttcacggg tagttgtcaa
600

ttggtctgaa acaaatttgc tagggaatct atttgtgtag aactaattaa tgtaaaaaaa
660

atagaccatc tcgtgttgtg tgcactgtga tataatggta gtatcagtgc aacttaaact
720

aatgattgta cttgatatta agtgttctca actgagtaac ttttaagtgg aaaccaagtt
780

tagatttggg gagtggtaaa ggaatcagct ttttctattg ttaggggaag acagtaattt
840

atcattcatg gaccagtaga ttgttgaaag ttggtgaatc ggattataag cttctagcta
900

acacaaggat tcagaattag gtaaacatct gaaggtttag tatattagaa acacccaaac
960

cagtaatatg ctaacctgat gcactgctga aagaaaatgt gaatttttcg taataattgc
1020

attttagtga attgtacagt gggtggaaag ggcatttgga gctcattaga atgagacata
1080

gtacacccca atggccctgt ttattaaatg tagtggatta agtgtctgtc aacaaataca
1140

ccaaaaccat tttttataga aacagtattt aatggtcact caatagcttt caaaatacat

1200

ttttgtatta cagcactgca caagctattc taatagtgct ctggcctcat cattcctgca
1260

aagcttgctt tggggagttg gataatgtga aaattttaag tacctagggg agaaagagcc
1320

atgtaaatat ctgtaataaa cttgtagcat atgtaaagtt ttcttggcct ttatcttaca
1380

aaaatggagt attttagtat gaatttgctg aatgtaagac cgtggactgt tttttataat
1440

atggcctaat tttaaaggtc caaaataact tgtttttaaa gtttgccctt gtgctaaagt
1500

gccagtgtat gtatgttata cttgatttgg ttgtaaacta tatttcaaag taaaccctag
1560

tgtaataagt tttataacta aaaaggttta agctgctaaa actattttta agagatgtga
1620

aatgcagtat gggactatct ttttttcctc ctctaagccc aaagattaac tagagtccct
1680

ccaaccttat agattgttgg ctttcacaat cttataacct aggatacagg tagtttcgag
1740

tatggtgcca gtgatgtttt gtttttgttt ggtcaagggg taggtgcaac ccaatggacc
1800

acttatgcaa aagatgtaaa ctcttgcata atacattgat aacatgtttt gccaacttta
1860

aatgcttaaa cataagcgaa accagtagca agtatgtggg tcagcttaaa aattttgatt
1920

gttaatgccc tattttctaa tttggcacct cttgatgcct aagcaggtaa gcagatgcct
1980

aagctgtatt tctccaaata aatcaagatg aagtactgcc caagttaaat attgatagcc
2040

taaagacaag tttatgtagt acttaatgta catgatatga agcataaaat taaataaaat
2100

ttttccccat tgaaaaaaaa aaaaaaaaa
2129

<210> 4
<211> 3950
<212> DNA
<213> human

<400> 4
cgagaactag ttttgttccg tgccctctgg actggaacct tttggagaga acccccggca
60

ggaccaaccc cgcacccgcc agcaccgcgg caatgtccag caatagtttt ccttacaatg
120

agcagtccgg aggaggggag gcgacggagc tgggtcagga ggcgacctca accatttccc
180

cctcgggggc cttcggcctc tttagcagcg atttgaagaa gaatgaagat ctaaagcaaa 240

tgttagagag caacaaagat tctgctaaac tggatgctat gaagcggatt gttgggatga 300

ttgcaaaagg gaaaaatgca tctgaactgt ttcctgctgt tgtgaagaat gtggccagta 360

aaaatattga gatcaagaag ttggtatatg tttacctggt tcgatatgct gaagaacagc 420

aggatcttgc actcctgtcc ataagcactt ttcagcgagc tctgaaggac ccaaaccaac 480

taattcgtgc aagcgctttg agagttctgt caagtattag agtgccaatt attgtaccta 540

tcatgatgct tgctattaag gaagcttctg ctgacttatc accatatgtt aggaagaatg 600

cagcccatgc aatacaaaaa ttatacagcc ttgatccaga gcagaaggaa atgttaattg 660

aagtaattga aaaacttctg aaagataaaa gcacattggt agctggcagt gttgtgatgg 720

cttttgaaga agtatgcccg gacagaatag atctgattca taaaaattac cgcaagctat 780

gtaacttact agtggatgtt gaagagtggg ggcaggttgt cataatccac atgctaactc 840

gatatgctcg gacacagttt gtcagcccct ggaaagaggg tgatgaatta gaagacaatg 900

gaaagaattt ctacgaatct gatgatgatc agaaggaaaa gactgacaaa aagaagaagc 960

cgtatactat ggatccagat catagactct taattagaaa tacaaagcct ttgcttcaga 1020

gcaggaatgc tgcggtggtt atggcagttg ctcagctgta ttggcacata tcaccaaaat 1080

ctgaagctgg cataatttct aaatcactag tgcgtttact tcgtagcaat agggaggtgc 1140

agtatattgt cctacaaaat atagcaacta tgtcaattca aagaaagggg atgtttgaac 1200

cttatctgaa gagtttctat gttaggtcaa ctgatccaac tatgatcaag acactgaagc 1260

ttgaaatttt gacaaacttg gcaaatgaag ccaacatatc aactcttctt cgagaatttc 1320

agacctatgt gaaaagccag gataaacaat ttgcagcagc cactattcag actataggca 1380

gatgtgcaac caacatcttg gaagtcactg acacgtgcct caatggcttg gtctgtctgc 1440

tgtccaacag ggatgaaata gttgttgctg aaagtgtggt tgttataaag aaattactgc 1500

aaatgcaacc tgcacaacat ggtgaaatta ttaaacatat ggccaaactc ctggacagta 1560

tcactgttcc tgttgctaga gcaagtattc tttggctaat tggagaaaac tgtgaacgag 1620

ttcctaaaat tgcccctgat gttttgagga agatggctaa aagcttcact agtgaagatg 1680

atctggtaaa actgcagata ttaaatctgg gagcaaaatt gtatttaacc aactccaaac 1740

agacaaaatt gcttacccag tacatattaa atctcggcaa gtatgatcaa aactacgaca 1800

tcagagaccg tacaagattt attaggcagc ttattgttcc gaatgaaaag agtggagctt 1860

taagtaaata tgccaaaaaa atattcctag cacaaaagcc tgcaccactg cttgagtctc 1920

cttttaaaga tagagatcat ttccagcttg gcaccttatc tcatactctc aacattaaag 1980

ctactgggta cctggaatta tctaattggc cagaggtggc gcccgaccca tcagttcgaa 2040

atgtagaagt aatagagttg gcaaaagaat ggaccccagc aggaaaagca aagcaagaga 2100

attctgctaa gaagttttat tctgaatctg aggaagagga ggactcttct gatagtagca 2160

gtgacagtga gagtgaatct ggaagtgaaa gtggagaaca aggcgaaagt ggggaggaag 2220

gagacagcaa tgaggacagc agtgaggact cctccagtga gcaggacagt gagagtggac 2280

gggagtcagg cctagaaaac aaaagaacag ccaagaggaa ctcaaaagcc aaaggaaaaa 2340

gtgattctga agatggggag aaggaaaatg aaaaatctaa aacttcagat tcttcaaatg 2400

acgaatctag ttcaatagaa gacagttctt ccgattctga atcagagtca gaacctgaaa 2460

gtgaatctga atccagaaga gtcactaagg agaaagaaaa gaaaacaaag caagatagaa 2520

ctcctcttac caaagatgtt tcacttctag atctggatga ttttaaccca gtatccactc 2580

cagttgcact tcccacacca gctctttctc caagtttgat ggctgatctt gaaggtttac 2640

acttgtcaac ttcctcttca gtcatcagtg tcagtactcc tgcatttgta ccaacgaaaa 2700

ctcacgtgct gcttcatcga atgagtggaa aaggactagc tgcccattat ttctttccaa 2760

gacagccttg catttttggt gataagatgg tctctataca aataacactg aataacacta 2820

ctgatcgaaa gatagaaaat atccacatag gggaaaaaaa acttcctata ggcatgaaaa
2880

tgcatgtttt taatccaata gactctcttg agcctgaggg atccattaca gtttcaatgg
2940

gtattgactt ttgtgattct actcagactg ccagtttcca gttgtgtacc aaggatgatt
3000

gcttcaatgt taatattcag ccacctgttg gagaactgct tttacctgtg gccatgtcag
3060

agaaagattt taagaaagag caaggagtgc taacaggaat gaatgaaact tctgctgtaa
3120

tcattgctgc accacagaat ttcactccct ctgtgatctt tcagaaggtt gtaaatgtag
3180

ccaatgtagg tgcagtccct tctggccagg ataatataca caggtttgca gctaaaactg
3240

tgcacagtgg gtcattgatg ctagtcacag tggaactgaa ggaaggctct acagcccagc
3300

ttatcataaa cactgagaaa actgtgattg gctctgttct gctgcgggaa ctgaagcctg
3360

tcctgtctca ggggtaacct gcttacatct ggactttaga atctggcaca caacaaaagt
3420

gcctggcatc cactactgct gcctttcatt tataataata gcccttccat ctggcagtgg
3480

gggtagaata cactcttgac attcttgtct cctgctttag aatgctagtg tgtatctatc
3540

atgtatgcaa tactttcccc ctttttgctt tgctaaccga agagcatata ttttactgtc
3600

agttgtctca actcttgaat ccatgtggcg ttttctctgt cctgctgctt cttttggcct
3660

cctcgttttc cttctctttt tcgacaatgg tagacatgaa tgagatattt aaagttcatt
3720

ggaaatcttc ttccctacag cagtaagcaa aaattagcaa agagatagtc taaatggcct
3780

ctcagcttgg tatgtgaaaa tgagatcaca tactttttaa atccaaatac aaaagcatag
3840

tctctgcaag attttgttct ttgaatttct tgatattgta attgattatt gataactgtc
3900

atcatgaaat tatctctcaa taataagata aataaactag catatgaatc
3950

<210> 5
<211> 5191
<212> DNA
<213> human

<400> 5
gagaaagaaa aacagctcga gacctcatgc aaagagaaaa ctgagtatct acagaaaatg
60

gttcagagga atgaaagata taaacaagat gtggagaggt tctatgaacg gaagcgacat 120

ttagatttaa ttgagatgct tgaagcaaaa aggccatggg tggaatatga aaatgttcgt 180

caggaatatg aagaagtaaa actagttcgt gaccgagtga aggaagaggt cagaaaactt 240

aaagaagggc agattcctat aacatgtcga attgaagaaa tggaaaacga gcgtcacaat 300

ttggaggctc gaatcaaaga aaaggcaaca gatattaagg aggcatctca aaaatgcaaa 360

cagaagcaag atgttataga aaggaaagat aaacatattg aggaacttca gcaggcttta 420

atagtaaagc aaaatgaaga gcttgaccga cagaggagaa taggtaatac ccgcaaaatg 480

atagaggatt tgcaaaatga actaaagacc acggaaaact gcgagaatct tcagccccag 540

attgatgcca ttacaaatga tctgagacgg attcaggatg aaaaggcatt atgtgaaggc 600

gaaataattg ataagcgaag agagagggaa actctagaga aggagaaaaa gagtgtggac 660

gatcatattg tacgttttga caatcttatg aatcagaagg aagataagct aagacagaga 720

ttccgtgaca cgtatgatgc tgttttatgg ctaagaaata acagagacaa atttaaacaa 780

agagtctgtg agcccataat gctcacgatc aatatgaaag ataataaaaa tgccaaatat 840

attgaaaatc atattccatc aaatgactta agagcctttg tatttgaaag tcaagaagat 900

atggaggttt tcctcaaaga ggttcgtgac aataaaaaat taagagtaaa tgctgttatt 960

gctcccaaga gttcatatgc agacaaagca ccttcaagat ctttgaatga acttaaacaa 1020

tacggatttt tctcttattt gagagaatta tttgatgcac ctgatcctgt aatgagttac 1080

ctttgctgtc agtatcatat tcatgaagtt cctgtaggaa ctgaaaagac cagagaaaga 1140

attgaacggg taatacaaga aacccgatta aaacagattt atacagcaga agaaaagtat 1200

gtggtgaaaa cttcttttta ttcaaacaaa gttatttcta gtaacacatc tctaaaagta 1260

gcgcagtttc tcactgtcac tgtggaccta gagcagagaa gacacttaga agaacagcta 1320

aaggaaattc atagaaaatt gcaagcagtg gattcagggt tgattgcctt acgtgaaaca 1380

agcaaacatc tggagcacaa agacaatgaa cttagacaaa agaagaagga gcttcttgag 1440

agaaaaacca agaaaagaca actggaacaa aaaatcagtt ccaaactagg aagtttaaag 1500

ctgatggaac aggatacttg caatcttgaa gaggaagagc gaaaagcaag taccaaaatc 1560

aaagaaataa atgttcaaaa agcgaaactt gttaccgaat taacaaacct aataaagatt 1620

tgtacttctt tgcatataca aaaagtagat ttaattctcc aaaatactac agtgatctct 1680

gagaagaaca aattagaatc agattatatg gccgcatctt cacaactccg tcttacagag 1740

caacatttca ttgaattgga tgaaaataga cagagattat tgcagaaatg caaggaactt 1800

atgaaaagag ctaggcaagt atgtaacctg ggtgcagagc agactcttcc tcaagaatac 1860

cagacacaag tacccaccat tccaaatgga cacaactcct cactccccat ggttttccaa 1920

gaccttccaa acacattgga tgaaattgat gctttattaa ctgaagaaag atcaagagct 1980

tcctgcttca cgggactgaa tcctacaatt gttcaggaat atacaaaaag agaagaagaa 2040

atagaacagt taactgagga actaaaggga aagaaagttg aactagatca atacagggaa 2100

aacatttcac aggtaaaaga aaggtggctt aatcctttaa aagagctggt agaaaaaatt 2160

aatgaaaaat tcagcaattt ttttagttcc atgcagtgtg ctggtgaagt tgatctccat 2220

acagaaaatg aggaagatta tgataaatat ggaattcgaa ttagagtcaa atttcgaagt 2280

agtactcaac tgcatgaatt aactcctcat catcaaagtg gaggtgaaag aagtgtttct 2340

accatgttat acttgatggc acttcaggag ctaaatagat gtccattcag agtagttgat 2400

gaaatcaatc agggaatgga cccaatcaat gaacggagag tgtttgaaat ggttgtaaat 2460

actgcctgta aagaaaatac atctcaatac tttttcataa caccaaagct cctgcaaaat 2520

cttccttatt ctgaaaagat gacagttttg tttgtctaca atggccctca tatgctggaa 2580

ccaaacacat ggaatttaaa ggctttccaa aggcggcggc gccgtattac attcactcaa 2640

ccttcttaat aaaagtaaag agagggaact tgggaatttt ttttgttaaa ttctgtttat 2700

aagtatggct caactgaata aaaggagatt cactaaaacg aaaagcagtt atttttggaa 2760

acctgctttt aaatacaaat aggttgataa tggaaactat aatgaccttt ccaaaatagc 2820

agctggtagt aaaagttaag tcttcttcag tcttggttga acttgagttc ttggcactct 2880

gaccatgagt cattcagttc tcatgttaaa atgtacttaa tattacaaat caaaggtaca 2940

gtggaagaag ggttaatcac aagaagttac ttatatggta gccctgagct ttaattgcag 3000

agtaacttta attactttta gagcctaaag atgactctag agcctaagtc ctagtttctc 3060

ccaatgttat atttaatttt aaaaaattga tatgaaaatg tctaatgtat agtaataatt 3120

tatgacagat ctagtcattt cttcctatta aaaaagatta ccttatctcc agtaggaaat 3180

ggaattttat gggcctttaa aagaaagttt tatgaaactt gatgctataa ttttattggt 3240

atttcaaggg gaaaaaagca ctggggttca aaaatggtag cagaactgct ttgaaatgct 3300

gcaaggtggc cactagatga tgcaaaatac aaccaaaaga ttgactgaga ataaaattag 3360

gtgacaaggg tttttaaaga ataacctttt aaagtgtggg ggcaggggtt gctttttttt 3420

attttattta aagtcaatta tattttacat cttacatttc taaaagcatt ttataattat 3480

ttttagtaag atttttctta aaatttcata tactggtttc tacaatttat atttgaaatt 3540

tctcagtgtt atgtaaagag tgatggaaaa gcattgattt ctttaaaacc gtaatgtttt 3600

tagaacttaa gcctataggg cctttcttac aatgttgatg tacccattat cttagaaaat 3660

ctagtttaaa ctgttttctt tcaccgcaaa agaattaaat gggaaaatca tttgtttatc 3720

tctaagttat actaattagt agaaccaaac aaattatctt cttttaaaaa ataaatctta 3780

taggaaaata gacagtccaa agtcatgtct ttgaacagtg gattggatct gtgccagtaa 3840

tgacaaaatt atttttttga cttgcttgcc tgaataaatt gaagaattgc tttcagtttg 3900

ggttttgtat attcttaagt agccattgaa atttatattc ttaactaggt caaaaaataa 3960

tgagccataa gtttatgtcc tctcacttag acattttctc tttaaaaagg tattttcttc 4020

tttataaaca ttttaaaaga gccttccctt cttaaactaa ctccagtgca tgaagtgtga
4080

aaatatttta aaatgacatt tttactaata tgagcaagtc atgtaaacat tgaagaactt
4140

ggtaacatat tagtaaatgg atattaccaa atgttttcat cgttaattac tttgcgttcc
4200

accaaaatat ctttactaaa atgtgcttgg tgtagtttgt ttattgtcta aattagtacc
4260

agtcatctta tttctgcaaa atgagtatca atgtgaaaaa gacacgtgaa gattaagcat
4320

gtttgaaaat aaaatggtca attacatttc aatttacata ggccaacaac tgttccatac
4380

tttgtttgta aacatttaat ttctctactg gacaaaatta atatttggct ttacattgaa
4440

ttttgagctg tgaagaataa attatgtatc attttagcat attaaacagt agtaagtcta
4500

gcacatagtc tcagccactt aaaacaaaag tttttttgtt tgtttgtttg tttgtttttt
4560

tgagatggag tctcactctg ttgcccaggc tggagtgcag tggcgtgatc tcggcttact
4620

gcaacctccg cctcccgggt tcaagcgatt ctcctgcctc agcctcccaa gtaactggga
4680

caacaggcgc gtcccaccac acccagctaa tttttttatac ttttagtaga gatggggttt
4740

cagcatattg gccaggctgg tctcgaactc ctgaccttgt gatccacccg cctcggcctc
4800

ccaaagtgct gggattatag gcgtgagccc ctgcacccgg ccaaaagttg atttttaatt
4860

acataaaaat cgtaaaaact tctagtaaaa acttgatttg gtgaatacag ttatatttta
4920

aaaccttaag gtgacaagca ttttctatgc ctaaatcttc attggtttgc ctggaaagag
4980

tctctgttaa aagattttcc atattcaaag taaaaggaaa gatttcttgc ttcctaattg
5040

tcttttggac acatgcctat tttctttgag gtataaacct ttagatgtga aaaatgtaat
5100

ttcattctgc tattgtgtgt gcttgtgtgt gtgtaattga aaaaactggg aaatcctgct
5160

ttgttggtaa taaatcaata tttttatatt c
5191

<210> 6
<211> 4755
<212> DNA
<213> human

<400> 6
aagagatctt ccaggctctc agagccctgg gagggcgatt tccaggaaga ccacaatgcc 60

aacctctgga ggaggctgga gagagaaggc ctaggccaga gcctgtcagg caactttggc 120

aagaccaagt cagccttctc atctctccag aacattcctg agagtctgag aagacacagc 180

agcctggagc taggccgggg aacccaggag ggttaccccg ggggcaggcc cacctgtgca 240

gtcaacacca aggcagaaga ccctgggagg aaagccgctc ctgacctcgg gagccatctg 300

gaccggcagg tttcctaccc gcggcccgag gggaggaccg gtgcctcggc ttctttcaac 360

agcacagacc caagtcccga agagccgcct gccccctcgc acccgcacac atccagtctg 420

ggccggaggg ggcccggccc aggcagcgcc tcggctcttc agggctttca gtacgggaag 480

ccccactgct cggtgctgga gaaggtctcc aaattcgagc agcgagagca agggagccag 540

agaccgagtg tgggcggctc tggttttggc cataactata ggccccacag gaccgtctca 600

acttccagta cttctgggaa tgacttcgag gagacaaaag cacacattcg tttctctgag 660

tcagctgaac ccctaggcaa cggggagcag cacttcaaaa acggggagct gaagttggaa 720

gaggcttccc ggcagccctg cggtcagcag ctgagcggag gagcgtcgga cagcggccgt 780

ggcccccaga ggccggacgc tcggctcctc cgtagccaga gcaccttcca gctctccagc 840

gagccagaga gggagcccga gtggcgggac aggcccggct cgcccgaatc gcccctgctg 900

gatgccccct tcagccgcgc ctaccggaac agcatcaagg acgcacagtc ccgtgtcttg 960

ggggccacct cctttcgacg tcgagacctg gagctggggg cgcccgtggc gtcgaggtcc 1020

tggcggccac ggccttcctc ggcccacgtg gggctgcgga gccccgaggc gtcggcctcc 1080

gcctccccgc acacgccccg ggagcggcac agcgtgaccc ctgctgaggg cgacctggcc 1140

aggcccgtgc cccctgccgc ccggagaggt gctcgccggc gcctgactcc cgagcagaag 1200

aagcgctcct actcggagcc cgagaagatg aacgaggtgg ggatcgtgga ggaggccgaa 1260

ccggcacccc tgggcccgca gagaaatggg atgcgtttcc cggagagcag cgtggccgac

1320

cggcgccgtc tcttcgagcg cgatggcaag gcctgctcca cgctcagcct gtcggggccc
1380

gagctgaagc agttccagca gagcgccctg gcggactaca tccagcgcaa gaccggcaag
1440

cggcctacct ccgccgccgg ctgcagcctc caggagcccg ggccactgcg tgagcgcgcc
1500

cagagtgcct acctccagcc cggccccgcg gcgctcgaag gctccggcct cgcctcggcc
1560

tccagcttga gctcactgcg ggagcccagc ctgcagcccc gcagggaggc cacgctcctg
1620

ccggccacag ttgcagaaac ccagcaggct ccccgagatc gcagcagctc cttcgccggt
1680

ggccgccgcc tcggggaacg gcgacgcggg gacctgctta gcggagcaaa cggtggaaca
1740

aggggcaccc agagagggga tgagaccccc agggagccat cctcctgggg ggccagggcc
1800

gggaagtcca tgtcggccga ggacctgctg gaacgctcgg acgtccttgc gggccctgtc
1860

catgtgaggt ccaggtcatc tcccgccacc gcagacaagc gccaggatgt gcttttgggg
1920

caagacagtg gctttggtct tgtgaaggat ccatgttatt tggctggtcc tggatctagg
1980

tcactcagtt gttcagaaag aggccaagaa gagatgctgc tgctcttcca ccatctcacc
2040

cctcgttggg gtggttcagg ctgcaaagcc attggtgatt cctccgttcc tagtgaatgt
2100

cctggaaccc tggaccatca gaggcaagcc agtaggacac cctgccccag gccaccactg
2160

gcaggaacgc aagggctggt cacagacacc agggctgcac ccctgacccc aattggcacc
2220

cctctgcctt cagccattcc ctctggctac tgctcacagg acggtcagac agggcgacag
2280

cctctcccgc cctacacccc tgccatgatg cacagaagca atggtcacac cctgacccag
2340

cctcccggtc caagaggctg tgagggcgat ggcccagagc atggggtaga agagggaacg
2400

aggaagaggg tctcgctgcc tcagtggcca cctccttctc gagcaaagtg ggcccacgca
2460

gccagagagg acagccttcc tgaggaatcc tcagcccctg attttgcaaa cctgaagcac
2520

tatcaaaaac agcagagtct tccaagttta tgcagcactt ctgacccaga cacacctctt
2580

ggggccccga gcactccagg gaggatctcc ctccgaatat ctgagtctgt cctgcgggac

2640

tccccgccac ctcatgagga ttatgaagac gaagtgtttg tgagggatcc gcacccccaag
2700

gccacgtcca gccccacatt tgaacctctt cccccacccc cacctcctcc accgagtcag
2760

gaaaccccgg tgtatagcat ggatgacttc cctccacctc ctccccacac tgtatgtgag
2820

gcgcagctgg acagtgagga tcccgagggg ccacgcccca gcttcaacaa actttctaaa
2880

gtgacaattg caagggaaag gcacatgcct ggtgcagccc atgtggtagg tagtcagaca
2940

ctggcttcca gactccaaac ttctatcaag ggttcagagg ctgagtccac accaccctcc
3000

ttcatgagcg ttcacgccca acttgctggg tctcttggtg ggcagccagc acccatccag
3060

actcaaagcc tcagccatga tccagtcagt ggaactcagg gtttagaaaa gaaagtcagt
3120

cctgatcctc agaagagttc agaagacatc agaacagagg ctttggccaa ggaaattgtc
3180

caccaagaca aatctctagc agacattttg gatccagact ccaggctgaa gacaacaatg
3240

gacctgatgg aaggtttgtt tccccgagat gtgaacttgc tgaaggaaaa cagtgtaaag
3300

aggaaggcca tacagagaac tgtcagctct tcaggatgtg aaggcaagag gaatgaagac
3360

aaggaagcag tgagcatgtt ggttaactgc cctgcctact acagtgtgtc tgctcccaag
3420

gctgagctac tgaacaaaat caaagagatg ccagcagaag tgaatgagga agaggaacag
3480

gcagatgtca atgaaaagaa ggctgagctc attggaagtc tcacccacaa gctggagacc
3540

ctccaggagg cgaaggggag cctgctcacg gacatcaagc tcaacaacgc cctgggagaa
3600

gaggtggagg ctctgatcag cgagctctgc aagcccaatg agtttgacaa gtataggatg
3660

ttcatagggg atttggacaa ggtggtcaac ctgctgctct ccctctcggg gcgtctagcc
3720

cgtgttgaga atgtccttag cggccttggt gaagatgcca gtaatgaaga aaggagctct
3780

ctttacgaga aaaggaagat cctggctggt cagcatgagg atgcccggga gctgaaggag
3840

aacctggatc gcagggagcg agtagtgctg ggcatcttgg ccaattacct ttcagaggag
3900

cagctccagg actaccagca cttcgtgaaa atgaagtcca cgctcctcat tgagcaacgg

3960

aagctggatg acaagatcaa gctgggccag gagcaggtca agtgtctgct ggagagcctg
4020

ccctcagatt tcattcccaa ggctggggcc ctggctctgc cccaaacct cacgagtgag
4080

cccattcctg ctgggggctg tactttcagt ggtattttcc caacattaac ctctccactt
4140

taacctcttc taaaataccc aaccaaaaga tcactgtttc tctcaacact atttaatctg
4200

aaaaatgttt cagtacaaac cactgtttga actatctggg ttattggtgt ttgttcctga
4260

tgaaaggaaa aaaattctct ccaggaggaa gcctttttcc ttcttgccct tcctgattga
4320

tcttctgaga gctcgaatgc tgctggacac gtaccccttt ctattattac tttgtagtag
4380

aaagaaagtt aatgaaactg agaactgatt ggagggtgtt tgatcattta gtttttaaca
4440

ggctgaggca acatggatca gtgtgtgtcc ccctcaggaa tgtatccaca gtggccttcc
4500

ttgctggtgg gcagtgtatc ctgatggcag ggtacaagta ccattaatga agggtctgca
4560

acataaagcc ttaaaaagac acacactaag aaaactgtaa aaccttgaac attgttattt
4620

atattttta aaatggaaaa gatcactatg tttgttgtgc taaccactta tttgattctg
4680

ttttgtggtg gacatagatg attacgtttg agctttgtat tttgtgaaaa ccttaatgaa
4740

atgaattcca aagat
4755

<210> 7
<211> 2045
<212> DNA
<213> human

<400> 7
gaaacttgac cccggctcat cctgtctctg gctgtggccc ggcaaagcac tgaaaacccc
60

tctggtctca gagacagtag gggcagtgcc actttctaca acctgccaac ccacacactg
120

gagtaattct gaaaaaaatt attcctaaac tctctaagtg tggacggaga atgagcaagc
180

cccagaagta ttttacaacc agagtgggta atgaggaggg ggcttactgg aatcgtcata
240

tctctgaata ttgaaaacaa caactaaaaa agtggacctt ctcagaaaaa aagggcagca
300

aatgaccaag ggcgcccctt ctggccgtgc ttggcttgag taactgtctc tctttcccca
360

cccccatcac agggctttca gtttggcaaa ggaaaagcag ataaaaacag aacattccat
420

atgtttcttt ctccatcggc caaaaacatt ttgacacaat gtttgtgaaa cacctttgga
480

gaggtgcact tctgaatgct gcctctgccg taaatcctgg ggcaagggat cagcctcttc
540

ccaggaacca tcgccttcta taaaccgtga actcaagcag gcattttttt tttcttaccg
600

aaaggctgct attgtgcaag ggcacataat gggtctgttg ctcttattgg cttccaaatg
660

tgcatggcaa agagagagat gtgggcctag agcagatata ttcagcaagg tgacagcttc
720

ccataacaat tctaacactt cttatcttat gtgagaataa aatatttaag ggttgaacct
780

tattttgcca aatgtatctt ttctgctttt gaattgggca gaagatttta gcaactatat
840

tctacaaatg ttacttataa cacacacaca cacatctgaa atatatgccg aaaattgacg
900

tctttgacct cagggagagc acctgtccag gtctgcctaa aggaaatggc tccagtgggt
960

ctaaacaacc acatcctatc catggatagg tctagtcata acactttaga gagaatgtca
1020

gagcaggagg gaggcaagcc gcctcttctc ggccatcaac tgcagatgat gaaagagcgg
1080

gattcaactt tgttttcttt tcctgtggcc ccagtgaaac ctcctgccct ccctgcacgt
1140

ctgtgtcttc atttctaaaa tgggggtgat gctttcatat tgacctcacc ccatactacc
1200

tcacagatgt gttgtgagga ttaataaaat tatgtctatg gtattttcag tttctggaga
1260

aaaatactta tagacagttt aactattaca tagatatata agtgatctca gtttcttgtt
1320

tgctgtgata ctaatgtgtt gttttaactt attccataaa atgacagttg tgtcctagcc
1380

acatcagaca gctatctaag ctctggacta cccctttgtg cagctgaatc actgcagggt
1440

cgaccatgcc tggtgccaca gccatggttt ccatttctag atgaaaggat ggcctaggac
1500

ataggtctca aagactcttg gatcagaatc aggagattag ggaaaacagg atggatacct
1560

gagcactaac agcagtagac gtagacctct gtcctttacc atctgaggtc ttctggattc
1620

tttgtggggt taattttgat ttgatgtcat ctgtttgccc ttcatcttgc ttgcaagtgt
1680

gcatggttca atccctcaca tccaggaaat gaattttgca attgggccag atgctaattt
1740

gcacgttgat tcaccttctt tgcctttaag cctttttttt cttttttttt ttttttggca
1800

aatgaatgta ccatttcaac tttgatttta atagtgctag ttgatattgg taataatgct
1860

aaccaagaga tcaatgccag atttttctct tggggtaagt tagctgaagt catttaaaga
1920

tggaaaggtg ggaaaattct ttgatatttg atgtcattgt atccacattt gttgtaagac
1980

atattgcata ccaattataa ttatatcaat taaagttgat aaaagcttca aaaaaaaaaa
2040

aaaaa
2045

<210> 8
<211> 2096
<212> DNA
<213> human

<400> 8
atggagaacg agcctgtagc ccttgaggaa actcagaaga cagatcctgc tatggaacca
60

cggttcaaag tggtggattg ggacaaggac ctggtggact ggcgaaagcc tctcctgtgg
120

caggtgggcc acttgggaga gaagtacgat gagtgggttc accagccggt gaccaggccc
180

atccgcctct tccactcaga cctcattgag ggcctctcta agactgtctg gtacagtgtc
240

cccatcatct gggtgcccct ggtgctgtat ctcagctggt cctactaccg aacctttgcc
300

cagggcaacg tccgactctt cacgtcattt acaacagagt acacggtggc agtgcccaag
360

tccatgttcc ccgggctctt catgctgggg acattcctct ggagcctcat cgagtacctc
420

atccaccgct tcctgttcca catgaagccc cccagcgaca gctattacct catcatgctg
480

cacttcgtca tgcacggcca gcaccacaag gcacccttcg acggctcccg cctggtcttc
540

ccccctgtgc cagcctccct ggtgatcggc gtcttctact tgtgcatgca gctcatcctg
600

cccgaggcag taggggggcac tgtgtttgcg gggggcctcc tgggctacgt cctctatgac
660

atgacccatt actacctgca ctttggctcg ccgcacaagg gctcctacct gtacagcctg
720

aaggcccacc acgtcaagca ccactttgca catcagaagt caggatttgg tatcagcact 780

aaattgtggg attactgttt ccacaccctc actccagaga aaccccacct gaagacgcag 840

tgacaactcc caccccctcc gtcctgccct cagcccggcc ctggcccctt cccgaccccc 900

acccgccatt cagaccccat taagaaggtt ggcttggcca ggcaggatgg gctgtgtccg 960

gccctgcagc ctagtggaag gtgctgaggg ggccctgagg caggaccgcc ctcctgaccc 1020

ctggtaggag ggtcacatcc acttggtgca ggtggccctt ggtgacccac ttcttcctgg 1080

agcgtccctg cctagagctc agcccacagg actgcttcag gccgtggcca caggtagcag 1140

ccgcaagggg aaatgaagaa aactgagccc tcgtggccac ctgtgtcacc cttgtgcctt 1200

agcctcatgg gctgcctagg agctgcctgc acggcacagc tcgctttcac agtcagaagt 1260

gggtctgtgg gatctgtggt ccctgtcctc cctgctgtcc cttctgggga ggctttggtg 1320

gctctgaggt ggacaaagag ctctcgcaag aagagacagc gtgatgcctc ccacagtcca 1380

ccccagaccc tggggcagcc cctctggccc tgccagctgc ctgcgtcgtt gggcccaggg 1440

tggctggcag gagtcccagc tgcttgcttt aggacctggc agcttttctt gccgtccctc 1500

ccctgcctcc agaatcacag cccttctccc caagggaggc tgaggaggct tctccaccag 1560

tggcagcccc accccgtccc tggccattct tggcctccac cccgctcagg cccctactcg 1620

ggcgctccca gaaggagcca cctctcagtg cctcacctcc ccctgcctcc cagcctccgc 1680

agatgaggtt cctgcccctt cctcctcgta accaaaaccc tcactgctcc caggacggtc 1740

ttatttataa accagataca tgttcttagt ctggtcccag accaaggagc tggtcagacg 1800

gcccttttcta atcctacatg ttgagcttat gtaaaaaatg ttgtttcctc ctgtttttgg 1860

ttcctttctt acccacaaac cattactact tgaaacttaa aaaactcgcc aagtgtaaag 1920

gctaaagaga agcagtttga cggaccttgt gatttgtact gtttgctgcg gagctattta 1980

aagattttgg aataaatata caaaactacg gttgtgaaat aaaaacttaa attgtatatt 2040

ttgaaaaata aaacactgaa aagaaaccaa caaaaaaaaa aaaaaaaaaa aaaaaa
2096

<210> 9
<211> 5640
<212> DNA
<213> human

<400> 9
ggaaacgcag aaaacagaga gaggcattct gagtcatctg actggatgaa gactgttcca
60

agttacaacc aaacaaatag ctccatggac tttagaaatt atatgatgag agatgagact
120

ctggaaccac tgcccaaaaa ctgggaaatg gcctacactg acacagggat gatctacttc
180

attgaccaca ataccaagac aaccacctgg ttggatcctc gtctttgtaa gaaagccaaa
240

gcccctgaag actgtgaaga tggagagctt ccttatggct gggagaaaat agaggaccct
300

cagtatggga catactatgt tgatcacctt aaccagaaaa cccagtttga aaatccagtg
360

gaggaagcca aaaggaaaaa gcagttagga caggttgaaa ttgggtcttc aaaaccagat
420

atggaaaaat cacacttcac aagagatcca tcccagctta aaggtgtcct tgttcgagca
480

tcactgaaaa aaagcacaat gggatttggt tttactatta ttggtggaga tagacctgat
540

gagttcctac aagtgaaaaa tgtgctgaaa gatggtcccg cagctcagga tgggaaaatt
600

gcaccaggcg atgttattgt agacatcaat ggcaactgtg tcctcggtca cactcatgca
660

gatgttgtcc agatgtttca attggtacct gtcaatcagt atgtaaacct cactttatgt
720

cgtggttatc cacttcctga tgacagtgaa gatcctgttg tggacattgt tgctgctacc
780

cctgtcatca atggacagtc attaaccaag ggagagactt gcatgaatcc tcaggatttt
840

aagccaggag caatggttct ggagcagaat ggaaaatcgg gacacacttt gactggtgat
900

ggtctcaatg gaccatcaga tgcaagtgag cagagagtat ccatggcatc gtcaggcagc
960

tcccagcctg aactagtgac tatccctttg attaagggcc ctaaagggtt tgggtttgca
1020

attgctgaca gccctactgg acagaaggtg aaaatgatac tggatagtca gtggtgtcaa
1080

ggccttcaga aaggagatat aattaaggaa atataccatc aaaatgtgca gaatttaaca

1140

catctccaag tggtagaggt gctaaagcag tttccagtag gtgctgatgt accattgctt
1200

atcttaagag gaggtcctcc ttcaccaacc aaaactgcca aaatgaaaac agataaaaag
1260

gaaaatgcag gaagtttgga ggccataaat gagcctattc ctcagcctat gccttttcca
1320

ccgagcatta tcaggtcagg atccccaaaa ttggatcctt ctgaggtcta cctgaaatct
1380

aagactttat atgaagataa accaccaaac accaaagatt tggatgtttt tcttcgaaaa
1440

caagagtcag ggtttggctt cagggtgcta ggaggagatg gacctgacca gtctatatat
1500

attggggcta ttattcccct gggagcagct gagaaagatg gtcggctccg cgcagctgat
1560

gaactaatgt gcattgatgg aattcctgtt aaagggaaat cacacaaaca agtcttggac
1620

ctcatgacaa ctgctgctcg aaatggccat gtgttactaa ctgtcagacg gaagatcttc
1680

tatggagaaa aacaacccga ggacgacagc tctcaggcct tcatttcaac acagaatgga
1740

tctccccgcc tgaaccgggc agaggtccca gccaggcctg caccccagga gccctatgat
1800

gttgtcttgc aacgaaaaga aaatgaagga tttggctttg tcatcctcac ctccaaaaac
1860

aaaccacctc caggagttat tcctcataaa attggccgag tcatagaagg aagtccggct
1920

gaccgctgtg gaaaactgaa agttggagat catatctctg cagtgaatgg gcagtccatt
1980

gttgaactgt ctcatgataa cattgttcag ctgatcaaag atgctggtgt caccgtcaca
2040

ctaacggtca ttgctgaaga agagcatcat ggtccaccat caggaacaaa ctcagccagg
2100

caaagcccag ccctgcagca caggcccatg ggacagtcac aggccaacca catacctggg
2160

gacagaagtg ccctagaagg tgaaattgga aaagatgtct ccacttctta cagacattct
2220

tggtcagacc acaagcacct tgcacagcct gacaccgcag taatttcagt tgtaggcagt
2280

cggcacaatc agaaccttgg ttgttatcca gtagagctgg agagaggccc ccggggcttt
2340

ggattcagcc tccgaggggg gaaggagtac aacatggggc tgttcatcct tcgtcttgct
2400

gaagatggtc ctgccatcaa agatggcaga attcatgttg gtgaccagat tgttgaaatc

2460

aatggggaac ctacacaagg aatcacacat actcgagcaa ttgagctcat tcaggctggt
2520

ggaaataaag ttcttcttct tttgaggcca ggaactggct tgatacctga ccatggtttg
2580

gctccttccg gtctgtgctc ctacgtgaaa cccgagcaac attaaggctt tcagggcttt
2640

tcttggtctt tccttaaaaa gacttggtga ttgggatatt aataatcctt cgtcttcaaa
2700

tgtgatttat gatgaacagt caccattacc cccatcttca cattttgctt ccatatttga
2760

agagtctcac gtgccagtaa ttgaagaatc tttgagagtt cagatatgtg aaaaggcaga
2820

agaattaaag gacattgtgc ctgaaaagaa aagcacttta aatgaaaatc agcctgagat
2880

aaagcatcag tctcttctcc agaaaaatgt gagtaagagg gatccaccca gcagtcatgg
2940

gcacagtaac aagaaaaatc tattaaaagt agaaaatggt gttacacgaa gaggtagatc
3000

ggttagtccc aaaaagccag ccagtcaaca ttcagaggaa catttggata agattcctag
3060

tcctctaaaa aataacccca aaagaagacc cagagatcaa tccctcagcc ccagcaaagg
3120

ggaaaataaa agttgtcagg tcagcaccag ggcaggctct ggacaagatc agtgcagaaa
3180

aagcagaggt cggtcggcca gcccaaaaaa gcagcaaaaa attgaaggaa gcaaagctcc
3240

atcaaatgct gaggccaaat tattagaggg taagagtcga agaatagcag gctatacggg
3300

cagtaatgct gagcagatcc cagatgggaa ggaaaaatca gacgtcatca ggaaagatgc
3360

aaagcagaat cagttggaaa aaagcagaac aaggtctcca gagaaaaaaa tcaaaagaat
3420

ggttgagaaa tctcttccat ccaaaatgac taataagact acaagtaaag aagtatctga
3480

aaatgaaaaa ggaaagaaag taaccacagg agaaacaagt tctagtaacg ataaaatagg
3540

agaaaatgtc cagctatcag aaaagaggct gaagcaagaa cctgaagaga aggtagtttc
3600

aaacaaaaca gaagatcaca aagggaaaga actagaggca gctgacaaaa acaaagagac
3660

tggaaggttc aaaccggaaa gcagttctcc agttaagaaa acactgataa ctccagggcc
3720

ctggaaggtt ccaagtggaa ataaagtcac aggcactatt ggtatggctg agaaacggca

3780
gtaaccttta gtataaaaca aagaaaaaca agttgtaatc ttttcttaca gcagcatttt
3840
tccagaaaaa gccttttttt ttttttcaga tattctgaaa cagataagta catgttaatg
3900
tgagcctcaa gttacctagg ctgcatgaag ggcctttagg attgctaaga accaactgtc
3960
cccctggccg gctgccctcc ctcgctctca ggaaggagct gcatccacat gctcatctga
4020
cccgccctgc tcaggctgcc cagctcgtct tcatgagtgt ctgaacaaat gacatatgtt
4080
gatattaaca atgtggtcac aactcacttt gtatttgtgc caagttatct actgtatcat
4140
gtctgttttt atcctttttg ttcagctgtt tccacagtaa tgaaaaagtt aggtttggct
4200
tggaagttga tgatctcaat agcatgttgc atgtttacag agagaaatat gtgagtcctt
4260
gcagaagaag agactgttaa ctcatcgtta aagatggccg ttgtctcttc taacagctac
4320
tgatgatgtc ccactttaaa aataaaaccc ccaaacatca ctactttaag gaaaaaaaaa
4380
atgtagtcca atattgatgc tttcttatgg ctttttattt taatttggct ggataagttg
4440
tttcaaataa ctgttaaaga tattacttac aattgaatgt ttgaaataag aaagtacttt
4500
aagcaataga gttcatctcc tgctgtgtta tccaacctcg atgtatactt acagcatctc
4560
aggtcacccct ttttatttca gttatttaat tatgaaacca taaagaagca tgtggaaata
4620
gtgtttattg ctctttgaag aaaaaccacc aactatttct ggatattttg gctgtaccta
4680
ctactaaagt cattagtctt taatacataa tacatatttg aaaagtaaac atattatata
4740
gattatgtga gggacttaat catgaaacca gtttcacagt ccaagtacca actcttctgg
4800
tagcaggtgc acaagcttgg gtgtttaaaa acaacctgtg tagggtatgc ccagcaaatg
4860
aggacaaatg tgtagacagt acttactgga tcttatttaa cttttagcta cattaactaa
4920
ctttcttatt taaaaacaag aaagggagac taaacatctg cttaacttgt acacattttc
4980
agaattcttt ttaaaagtct agttaaagat gtttcttaga agttggagac tgttaacaac
5040
ttccataaaa tagatccagg tttttcagtt ccctgaagca gcattcagta gcatctatat

5100

aaataaaggc accttctgag aataaaacta ttttatggag tgtgtgaaca cacttgttct
5160

gtcacctggg ttcatcttgt tgtgaagcac attaggtcca ggtccttccc tctgggagtc
5220

tgactgtgaa actctttaac ccaacaactc aattagcccc tgtagataag acatgcttcc
5280

cagagtgaga tttttgaaat cccctttca tccagaacta tatttaccca cctattgtaa
5340

ctattcaaat agagcaaaat taggaggctt gataaatact aagaatttag taccacagaa
5400

attatttatt attttccctg tagtccacaa ttagtgataa cgaatcctat ttttgttaac
5460

tgtgacataa ctttgatgtc atatgttgtc ctatgtggtt cttcctaagt aaactctgta
5520

ctgattatat actgacttag caatgtggcc ttggaatgct gagcaaaatg tggatgtact
5580

ggttgtaaat gtttatatat tgtacagtac ctttatatat acacttgagg ttctgattag
5640

<210> 10
<211> 457
<212> DNA
<213> human

<220>
<221> misc_feature
<222> (242)..(242)
<223> any kind of base

<220>
<221> misc_feature
<222> (369)..(369)
<223> any kind of base

<220>
<221> misc_feature
<222> (394)..(394)
<223> any kind of base

<220>
<221> misc_feature
<222> (406)..(406)
<223> any kind of base

<220>
<221> misc_feature
<222> (457)..(457)
<223> any kind of base

<400> 10

tcagtcactc tttcaccctg ccaaagcttc actgtcctac tgattgaatt gtatgtgaga
60

aataaaatgt catcatatta agccactggg atttgtatgt ttatctgtta tagcagcaag
120

tcttaattta cctaatacac acattgtgac agatgttctt aatgtcccac cccatattgt
180

tacatgtcca gctttgagga tccctggcat gtgggggtag gagtttctgg gcatgctgga
240

tncaattccc acttttaagg catctgtggc ctctgtggcc tctgtggcct tcactgttat
300

ggaagggatt tatctggggc accataggaa actttaccat ggcacagtgg acaacctagg
360

agggggtgng gaggaggggc cttcaggccc aacngggggg accagngttc gtggggttag
420

ggtggtttgg ggggttttcc ctcttacccg tgggggn
457

<210> 11
<211> 1493
<212> DNA
<213> human

<400> 11
aatagggttg gcggctgcag cgggcggcaa acagcccgcc cggcaccacc atgctcgccc
60

tggaggctgc acagctcgac gggccacact tcagctgtct gtacccagat ggcgtcttct
120

atgacctgga cagctgcaag cattccagct accctgattc agaggggct cctgactccc
180

tgtgggactg gactgtggcc ccacctgtcc cagccacccc ctatgaagcc ttcgacccgg
240

cagcagccgc ttttagccac ccccaggctg cccagctctg ctacgaaccc cccacctaca
300

gccctgcagg gaacctcgaa ctggccccca gcctggaggc cccggggcct ggcctccccg
360

cataccccac ggagaacttc gctagccaga ccctggttcc cccggcatat gccccgtacc
420

ccagccctgt gctatcagag gaggaagact taccgttgga cagccctgcc ctggaggtct
480

cggacagcga gtcggatgag gccctcgtgg ctggccccga ggggaaggga tccgaggcag
540

ggactcgcaa gaagctgcgc ctgtaccagt tcctgctggg gctactgacg cgcggggaca
600

tgcgtgagtg cgtgtggtgg gtggagccag gcgccggcgt cttccagttc tcctccaagc
660

acaaggaact cctggcgcgc cgctggggcc agcagaaggg gaaccgcaag cgcatgacct
720

accagaagct ggcgcgcgcc ctccgaaact acgccaagac cggcgagatc cgcaaggtca
780

agcgcaagct cacctaccag ttcgacagcg cgctgctgcc tgcagtccgc cgggcctgag
840

cacacccgag gctcccacct gcggagccgc tgggggacct cacgtcccag ccaggatccc
900

cctggaagaa aaagggcgtc cccacactct aggtgatagg acttacgcat ccccaccttt
960

tggggtaagg ggagtgctgc cctgccataa tccccaagcc cagcccgggc ctgtctggga
1020

ttccccactt gtgcctgggg tccctctggg atttctttgt catgtacaga ctccctggga
1080

tcctcatgtt ttgggtgaca ggacctatgg accactatac tcggggaggc agggtagcag
1140

tgcttccaga gtcccaagag cttctctggg attttcttgt gatatctgat tccccagtga
1200

ggcctgggac ctttttaaga tcgctgtgtg tctgtaaacc ctgaatctca tctggggtgg
1260

gggccctgct ggcaaccctg agccctgtcc aaggttccct cttgtcagat ctgagatttc
1320

ctagttatgt ctggggccct ctgggagctg ttatcatctc agatctcttc gcccatctat
1380

ggctgtgttg tcacatctgt cccctcattt ttgagatccc ccaattctct ggaactattc
1440

tgctgcccct ttttatgtgt ctggagttcc ccaatcacat ctagggctcc tcc
1493

<210> 12
<211> 2292
<212> DNA
<213> human

<400> 12
ccatgggttc cccttcagcc tgtccataca gagtgtgcat tccctggcag ggctcctgc
60

tcacagcctc gcttttaacc ttctggaacc tgccaaacag tgcccagacc aatattgatg
120

tcgtgccgtt caatgtcgca gaagggaagg aggtccttct agtagtccat aatgagtccc
180

agaatcttta tggctacaac tggtacaaag gggaaagggt gcatgccaac tatcgaatta
240

taggatatgt aaaaaatata agtcaagaaa atgccccagg gcccgcacac aacggtcgag
300

agacaatata ccccaatgga accctgctga tccagaacgt tacccacaat gacgcaggat
360

tctataccct acacgttata aaagaaaatc ttgtgaatga agaagtaacc agacaattct

420

acgtattctc ggagccaccc aagccctcca tcaccagcaa caacttcaat ccggtggaga
480

acaaagatat tgtggtttta acctgtcaac ctgagactca gaacacaacc tacctgtggt
540

gggtaaacaa tcagagcctc ctggtcagtc ccaggctgct gctctccact gacaacagga
600

ccctcgttct actcagcgcc acaaagaatg acataggacc ctatgaatgt gaaatacaga
660

acccagtggg tgccagccgc agtgacccag tcaccctgaa tgtccgctat gagtcagtac
720

aagcaagttc acctgacctc tcagctggga ccgctgtcag catcatgatt ggagtactgg
780

ctgggatggc tctgatatag cagccttggt gtagtttctg catttcggga agagtgtttt
840

tattatccac ctgcagactg gactggattc ttctagctcc ttcaatccca ttttctcctg
900

tggcatcact aagtataaga cctgctctct tcctgaagac ctataagctg gaggtggaca
960

actcaatgta aatttcaagg aaaaaccctc atgcctgaga tgtgggccac tcagagctaa
1020

ccaaaatgtt caacaccata actagagaca ctcaaattgc caaccaggac aagaagttga
1080

tgacttcatg ctgtggacag tttttcccaa gatgtcccaa gcctcatcgt gacgaggctc
1140

ttatcccact ccatttttcc ctgctcatgc ctgcctcttt aatttggtaa gataatgctg
1200

taactagaat ttcacaatca gcgccttgtg caggcaattt gacagagtgt tggatgtgtc
1260

atgtcatcat gtcaaaccca aatatttgac ctaagggatc ctttattctg cccagtggct
1320

aactttaaca acatccctaa tacaactgtt tattcaaatg cacggtggtc cctgttagag
1380

ttagacctct agactcacct gttctcacgc cctgttttaa tttaacccag ctatgggatg
1440

ccagataaca gaattgctgc ctacgagctg aacagggagg agtttgtgca gttgctgaca
1500

cttcttgttg cacataaata aatacagtgg gtactataga gactcagttg caaaaattaa
1560

caaatatgct gcttgattaa aatgggtagg cttctcatgt ggctcattct ttaatctatt
1620

ctcttttatt tggtttggtt catgggtct ctgcctatgg atcatacttc aaactcttgg
1680

tgtgatcctc ctgattgtca caatattagt taccctggtg tgctgtattc tctaaaacct

1740

ttaaatgttt gcatgcagcc attcgtcaaa tgtcaaatat tctctctttg gctggaatga
1800

caaaaactca aataaatgta tgattaggag gacatcataa cctatgaatg atggaagtcc
1860

aaaatgatgg taactgacag tagtgttaat gccttatgtt tagtcaaact ctcatttagg
1920

tgacagcctg gtgactccag aatggagcca gtcatgctaa atgccatata ctcacactga
1980

aacatgagga agcaggtaga tcccagaaca gacaaaactt tcctaaaaac atgagagtcc
2040

aggctgtctg agtcagcaca gtaagaaagt cctttctgct ttaactctta gaaaaaagta
2100

atatgaagta ttctgaaatt aaccaatcag tttatttaaa tcaatttatt tatattcttc
2160

tgttcctgga ttcccatttt acaaaaccca ctgttctact gttgtattgc ccagtaggag
2220

ctatcactat attttgcaga atggaaactg ccctgactct tgaatcacaa ataaaagcca
2280

attgtatctg tt
2292

<210> 13
<211> 519
<212> DNA
<213> human

<220>
<221> misc_feature
<222> (212)..(212)
<223> any kind of base

<220>
<221> misc_feature
<222> (451)..(451)
<223> any kind of base

<400> 13
gaaacaacaa cagtgtaatc tttaacaggg atgttaaagg taagaagtca ggaagataaa
60

ccaaaatgat tgagtatgat aaagaatttt gcatggcgat taaaatagaa aacctataaa
120

tgtagaaaaa gcaggtctgg acttagcaaa gaaacaatat agtttggaga aggcatgaaa
180

taagttcttt tcatgttcac tgctggtcac ancataacag agagtgatgt ggagagcttt
240

gggaaggttt cacgttgagt tacatcagtg gtcaacaatg gagcaacaag actccgtaga
300

ggatgccacc ctgggagaat tgcaagggaa aggaggctga agcacaactg gtaatagcct
360

tcagatattt aatggatatg caaataaagc tctgattaat tgtattttca cttattatat
420

atcatctttg gacctttcta aaagtgggac nctagaaaag atatactgaa actccaaaag
480

aatacttcag ctcgagttga atggattcaa gatgttgtt
519

<210> 14
<211> 5294
<212> DNA
<213> human

<400> 14
ggctcgcatc cccatagtgc tgggttacag tgaaggtacg ccccgcgctc tgctctggag
60

aggcagggtg ggatagggaa cgtctcgagt ggcgcccgca gtcatggtgg tgttcgttgg
120

ccgccgcctc ccggcgctcc tagggctgtt taagaagaag ggctctgcca aggctgagaa
180

tgacaaacat ctaagtgtag ggcctggcca ggggccaggg tctgcagtgg atgagcacca
240

ggacaacgtc ttctttccca gtgggcgacc cccccacctg gaagagctgc acactcaggc
300

ccaggagggg ctccgctccc tacaacacca agagaaacag aaactgaaca agggtggctg
360

ggaccatgga gacacccaga gtatccagtc ctcccggacg gggccggatg aagacaacat
420

ctccttctgc agtcagacca catcctacgt ggctgagagc tccacagcag aggacgcgct
480

ctccatccgc tcggagatga tccagcgcaa aggctccacc ttccgacccc atgactcatt
540

tcccaaatct ggaaagtcag ggcggcgtcg gcgggagcgg cggagcactg tgctgggact
600

cccgcagcat gtgcagaagg agcttggcct gaggaatgag cgtgaggcac caggcacgcc
660

ccgggctcct ggtgcacggg atgccgtacg catccccaca gtggacggcc gcccccgagg
720

cacctcaggg atgggggccc gggtgtccct gcaggcgctg gaggcggagg cagaggctgg
780

cgctgagaca gaggccatgc tgcagcgcca cattgaccgt gtctaccggg atgacacctt
840

tgttggccgg tccacgggta cccgggcccc accattgacc cggcccatgt ccctagcagt
900

gcctggattg acaggagggg cagggcctgc agagcccctg agcccggcca tgtccatctc
960

cccccaggcc acctacctgt cgaagttgat tccacatgct gtgctgccgc ctacagtgga
1020

cgtggtggcc ctaggccgct gcagcctgcg cacactaagc cgctgcagcc tgcactcggc
1080

cagcccagcc tcagtccgct cgctggggcg cttctcctcc gtctccagcc cacagccccg
1140

cagccgccac ccatcctcct ccagtgacac ctggagccac tctcaatcct ccgacaccat
1200

tgtgtctgac ggttccaccc tctcctctaa gggtggctct gagggccagc cggagagctc
1260

tacggctagc aatagcgtgg tacccccctcc ccagggaggc agtgggaggg gctctcccag
1320

tgggggcagc actgctgagg cctcagacac actcagcatt cggagcagtg ggcagttgtc
1380

tggccggagt gtgtccctgc gtaagctgaa gcggcctcca ccccctcccc gccggaccca
1440

ctccctccat cagcggggct tagcagtgcc tgatgggcca ttagggttgc cccctaagcc
1500

tgagcgtaag cagcagcccc agctgcctcg gccacccacc actggtggct cagaaggggc
1560

gggggcagca ccctgtccac ccaacccagc caacagctgg gtacctggct tgtctccggg
1620

tggttcccgg cgccccccac ggtccccaga acggacactt tcgccctcca gtggatactc
1680

gagccaaagt ggtactccca ccctccctcc caagggcctg gcaggtcccc ctgcttcccc
1740

aggcaaggcc cagcccccta aaccagagcg tgtcacgtct cttcgctccc ctggggcctc
1800

cgtctcctct tccctcacgt ctttatgttc ctcctcctct gacccagccc cctcagaccg
1860

ctctgggcca cagatattga cccccctggg tgacaggttt gtcatacctc ctcaccccaa
1920

ggtgcctgcc cccttctccc cacctccctc caagcccagg agccctaacc cagctgcccc
1980

tgctctagcc gcccctgctg tggttcctgg gcctgtttct accactgacg ccagtcctca
2040

gtcccctccc actccccaga caaccttgac tccactgcag gagtctcctg tcatctccaa
2100

agaccagtca cccccacctt ccccaccccc atcttatcat ccacccccac cacccactaa
2160

gaagccagag gtggttgtgg aggcaccatc tgcctcagag actgctgagg agcccctcca
2220

agatcccaac tggcccccctc ccccaccccc tgcccctgag gagcaggacc tgtccatggc
2280

tgacttcccc ccaccagagg aggctttttt ctctgtggcc agccctgagc ctgcaggccc
2340

ttcaggctcc ccagagcttg tcagctcccc ggctgcttcg tcctcctcag ctactgcttt
2400

gcagattcag cccccgggta gcccagaccc tcctccagct ccgccagccc cagctcctgc
2460

tagttccgcc ccagggcatg tggccaagct ccctcagaag gaaccggtgg gctgtagcaa
2520

gggtggtggg cctcccaggg aggacgtagg tgcgcccctg gtcacgccct cgctcctgca
2580

gatggtgcgg ctgcgctccg tgggtgctcc aggaggggct cccaccccag cactggggcc
2640

atcggccccc cagaaaccac tgcgaagggc cctgtcaggg cgggccagcc cagtgcctgc
2700

cccctcctca gggctccatg ctgcggtccg actcaaggcc tgcagcctgg ccgccagtga
2760

aggcctctca agtgctcagc ccaacggacc gcctgaggca gagccacggc ctccccagtc
2820

ccctgcctca acggccagtt tcatcttctc caagggctct aggaagctgc agctggagcg
2880

gcccgtgtcc cctgagaccc aggctgacct ccagcggaat ctggtggcag aactccggag
2940

catctcagag cagcggccac cccaggcccc aaagaagtca cctaaggctc ccccacctgt
3000

ggcccgcaag ccgtctgtgg gagtcccccc acccgcctcc cccagttacc ctcgagctga
3060

gccccttact gctcctccca ccaatgggct ccctcacacc caggacagga ctaagaggga
3120

gctggcggag aatggaggtg tcctgcagct ggtgggccca gaggagaaga tgggcctccc
3180

gggctcagac tcacagaaag agctggcctg accaccaggc acctcactgg cactgctgac
3240

ccatcccaga aacacaatct cagggacccg agcagctcca aggacgagag gatacagcag
3300

acacaaccta atagagaggg cgcctgcagc cttaacctcc acggccttcg atacttatgc
3360

aagcctggtg ttgctcctgt cctcagagtc atcctgcgct catgcctttt cccgaatggg
3420

ttcacctctg gcagttgccg cttcagtctt ggccttagcc tcatcttgaa gtgggtagct
3480

ggcgggagag ggtggctgcg ccccctgctg gccctgaggc tgcagagttg ggagcaggac
3540

acctcacctg agtttcattt tttttcatgt ccaaaccatg cacatactat agtccagaat
3600

caaagcactt ttgaaaagtg gctgcatggc catcctccag ggcccaggaa gttgcattcc 3660

aagggcctgt ttacatggca gcagaatcca tccccggcag tcagcccata gcttgggacc 3720

agtctgtgcc ctcctgccca gtccagttta ctcctcttgg ttcctgaagg tggccaagtc 3780

attgtgttcc cacaggcttc tctaggctgg gggcaggtgt ggggctgtgg aattccaaag 3840

cacaaaaggt gcagagggga ttggccttcc tgtgcctcaa ctcaccaacc accctcctgc 3900

cttccagttc tgccaggtgc tccatgctgg ggacaagtag gagactgcca gggcccaaag 3960

aaatgggtga gcagtagagt catctcgggg cacttggcag tgtcaagcac ctgccccttg 4020

cctccttgac cacactgggg tgggtgggcc cccagcactt cagaggcagg agcctttggg 4080

ctgagcaagc actgaggagg tggatggaag ggagcatctg gagggggga gcttccttga 4140

gcagtgggcc caggcctggc cctccacact tcattctctg acctttctct ctcctcattt 4200

cggtgcatgt cctttctgca gctgcctttc agcacaggtg gttccactgg gggcagctaa 4260

cgctgagtga caaggatggg aagccacagg tgcattttac tcaagtcttc tctagtcaat 4320

gaggggcacc cagtgcttct agggcaggct gggtggtggt cccctaggta tcagcctctc 4380

ttactgtact ctccgggaat gttaaccttt ctattttcag cctgtgccac ctgtctaggc 4440

aagctggctt ccccattggc ccctgtgggt ccacagcagc gtggctgccc cccagggcca 4500

ccgcttcttt cttgatcctc tttccttaac agtgacttgg gcttgagtct ggcaaggaac 4560

cttgctttta gcttcaccac caaggagaga ggttgacatg acctccccgc cccctcacca 4620

aggctgggaa cagaggggat gtggtgagag ccaggttcct ctggccctct ccagggtgtt 4680

ttccactagt cactactgtc ttctccttgt agctaatcaa tcaatattct tcccttgcct 4740

gtgggcagtg gagagtgctg ctgggtgtac gctgcacctg cccactgagt tggggaaaga 4800

ggataatcag tgagcactgt tctgctcaga gctcctgatc taccccaccc cctaggatcc 4860

aggactgggt caaagctgca tgaaaccagg ccctggcagc aacctgggaa tggctggagg 4920

tgggagagaa cctgacttct ctttccctct ccctcctcca acattactgg aactctatcc
4980

tgttaggatc ttctgagctt gtttccctgc tgggtgggac agaggacaaa ggagaaggga
5040

gggtctagaa gaggcagccc ttctttgtcc tctggggtaa atgagcttga cctagagtaa
5100

atggagagac caaaagcctc tgatttttaa tttccataaa atgttagaag tatatatata
5160

catatatata tttctttaaa tttttgagtc tttgatatgt ctaaaaatcc attccctctg
5220

ccctgaagcc tgagtgagac acatgaagaa aactgtgttt catttaaaga tgttaattaa
5280

atgattgaaa cttg
5294

<210> 15
<211> 988
<212> DNA
<213> human

<400> 15
gtcgtgaggc gggccttcgg gctggctcgc cgtcggctgc cggggggttg gcctgggtgt
60

cattggctct gggaagcggc agcagaggca gggaccactc ggggtctggt gtcggcacag
120

ccatggcggg cgcgttggtg cggaaagcgg cggactatgt ccgaagcaag gatttccggg
180

actacctcat gagtacgcac ttctggggcc cagtagccaa ctggggtctt cccattgctg
240

ccatcaatga tatgaaaaag tctccagaga ttatcagtgg gcggatgaca tttgccctct
300

gttgctattc tttgacattc atgagatttg cctacaaggt acagcctcgg aactggcttc
360

tgtttgcatg ccacgcaaca aatgaagtag cccagctcat ccagggaggg cggcttatca
420

aacacgagat gactaaaacg gcatctgcat aacaatggga aaaggaagaa caaggtcttg
480

aagggacagc attgccagct gctgctgagt cacagatttc attataaata gcctccctaa
540

ggaaaataca ctgaatgcta tttttactaa ccattctatt tttatagaaa tagctgagag
600

tttctaaacc aactctctgc tgccttacaa gtattaaata ttttacttct ttccataaag
660

agtagctcaa aatatgcaat taatttaata atttctgatg atgttttatc tgcagtaata
720

tgtatatcat ctattagaat ttacttaatg aaaaactgaa gagaacaaaa tttgtaacca

780

ctagcactta agtactcctg attcttaaca ttgtctttaa tgaccacaag acaaccaaca
840

gctggccacg tacttaaaat tttgtcccca ctgtttaaaa atgttacctg tgtatttcca
900

tgcagtgtat atattgagat gctgtaactt aatggcaata aatgatttaa atatttgtta
960

aaaaaaaaaa aaaaaaaaaa aaaaaaaa
988

<210> 16
<211> 4908
<212> DNA
<213> human

<400> 16
ggataacctc gcagggtggg ccggagggcg ggcgccgccg ctgcctgtgc tgcggcgatg
60

gcccagtgtg tacaatcagt gcaggagcta atcccggact ccttcgtccc ctgtgtcgct
120

gcgctgtgca gcgacgaagc cgagcggctc actcgtctca atcacctcag cttcgcggag
180

ctgcttaagc ccttctcccg cctcacttcc gaggttcaca tgagagatcc taataatcaa
240

cttcacgtaa ttaaaaattt gaagatagca gtaagcaaca ttgtcaccca gccacctcag
300

cctggagcca tccggaagct tttgaatgat gttgtttctg gcagtcagcc tgcagaagga
360

ttagtagcta atgtgattac agcaggagat tatgacctta acatcagtgc cactactcca
420

tggtttgagt cttacagaga aacctttctt cagtcgatgc cagcatcgga tcatgaattt
480

ctgaaccact atttagcatg tatgttggta gcgtcatcta gtgaagctga acctgtggaa
540

cagttttcaa agttgtcaca agaacagcat cgaattcagc acaacagtga ttattcctac
600

cccaagtggt ttataccaaa tacacttaaa tactatgtac ttttacatga tgtaagtgca
660

ggagatgaac agagagctga atcaatttat gaagaaatga aacagaaata tggaactcag
720

ggttgctatt tacttaaaat taattctcga acatctaatc gagcatcaga tgaacagata
780

ccagatcctt ggagtcagta tctccagaaa aatagtattc aaaaccagga atcatatgaa
840

gatggccctt gtactataac ttcaaataag aattctgata ataacttgct ttcattggat
900

ggattagata acgaagtcaa agatggctta ccaaataact ttagagctca cccacttcag 960

ttggagcaat ccagtgaccc ttctaacagt attgatggcc cagatcatct aagatctgct 1020

tcatcgttac atgaaacaaa gaaaggaaat actggaataa ttcatggtgc atgtttaaca 1080

cttactgatc atgatagaat tcgacagttt atacaagagt tcacatttcg ggcctttttg 1140

ccacatatag agaaaacaat taggcaatta aacgatcagc taatatcaag aaaaggtttg 1200

agtcgatctc tattttctgc aactaaaaaa tggtttagtg gcagtaaagt tccagaaaag 1260

agcattaatg acctgaaaaa tacatctggc ttgctgtatc ctccggaagc accagaactt 1320

caaatcagga aaatggctga cttatgtttt ttggtgcagc attatgattt ggcttacagt 1380

tgctatcata ctgcaaagaa agattttctt aatgatcaag caatgcttta tgcagctggt 1440

gccttggaaa tggcagcagt gtctgctttt cttcaaccag gagcacctag gccatatcct 1500

gctcattaca tggatacagc aattcagaca tacagagata tctgcaagaa tatggtgttg 1560

gctgaaagat gtgtgttgct tagtgctgaa cttttaaaaa gccaaagcaa atattcagag 1620

gctgcagctc tcctaatacg gttgaccagt gaggattctg atcttcgaag tgcacttctt 1680

ttggaacagg cagcacattg ctttataaac atgaaaagtc ccatggttag aaaatatgca 1740

tttcatatga tattggcagg ccatcgattt agtaaagcag ggcagaaaaa gcatgcttta 1800

cgctgttatt gtcaagccat gcaagtttac aaaggaaaag gctggtctct tgcagaggat 1860

cacattaatt tcactattgg gcgccagtcc tatactctta gacagctgga taatgctgtg 1920

tctgctttta ggcatattct aattaatgaa agtaaacaat ctgctgctca acagggggct 1980

ttcctcagag aatatcttta tgtttacaag aatgtaagtc agctgtcacc agatggtcct 2040

ttgccacagc ttcctttacc gtatattaac agttcagcaa cacgggtttt ttttggccat 2100

gacagacgac cagcggatgg tgaaaaacaa gcagctactc atgtaagtct tgatcaagaa 2160

tatgattctg aatcctctca gcagtggcga gaacttgagg aacaagttgt ttctgtggtt 2220

aacaaaggag taattccatc caattttcat cccacacaat actgtttgaa cagttactca 2280

gataattcaa gatttccact tgcagttgta gaagaaccaa ttacagtgga agtggctttt 2340

agaaaccctt tgaaagttct acttttgttg actgatttgt cattgctttg gaagtttcat 2400

cctaaagatt tcagtggaaa ggataatgaa gaagttaaac aactagttac aagtgaacct 2460

gaaatgattg gagctgaagt tatttcagag ttcttaatta atggcgaaga atcaaaagtg 2520

gcaagactaa agctctttcc ccatcacata ggggagctgc atattctggg agttgtttat 2580

aatcttggca ctattcaggg ctctatgaca gtagatggca ttggtgctct tcccggatgt 2640

cacacaggaa aatattcctt gagtatgtca gtccgaggga agcaggattt agaaattcaa 2700

ggtcctcgac ttaacaacac aaaagaagag aaaacatctg ttaaatatgg ccctgatcga 2760

cgtttagatc ccataatcac agaagaaatg ccactgttgg aggtgttctt tatacatttt 2820

cctacagggc ttctctgtgg agaaatccga aaagcatatg tagaatttgt caatgtcagc 2880

aaatgtccac ttactggatt gaaggttgtt tctaaacgtc cagagttctt tactttcggt 2940

ggtaatactg ctgttctaac accactaagt ccctcagctt ctgagaattg tagtgcttac 3000

aagactgttg tgacagatgc tacctctgtg tgtacagcac tcatatcatc agcttcttct 3060

gtagactttg gcattggcac aggaagtcaa ccagaggtga ttcctgttcc ccttcctgac 3120

actgttcttc tacccggagc ctcagtgcag ctgccaatgt ggttacgtgg gcctgatgaa 3180

gaaggtgtcc atgaaattaa ctttttgttt tactatgaaa gtgtcaaaaa gcagccaaaa 3240

atacggcaca gaatattaag acacactgca attatttgta ccagtcggtc tttaaatgta 3300

cgggccactg tctgcagaag taattctctt gaaaatgaag aaggcagagg aggcaatatg 3360

ctagtctttg tggatgtgga aaataccaat actagtgaag caggcgttaa ggaattccac 3420

atagtgcaag tatcaagtag tagcaaacac tggaagttac agaaatctgt aaatctttct 3480

gaaaacaaag atgccaaact tgccagtagg gagaagggaa agttttgctt taaggcaata 3540

agatgtgaga aagaagaagc ggccacacag tcctctgaaa aatatacctt tgcagatatc 3600

atctttggaa atgaacagat aataagttca gcaagcccat gtgcagactt cttttatcga 3660

agtttatctt ctgaattgaa aaaaccacaa gctcacttgc ctgtgcatac agaaaaacag 3720

tcaacagagg atgctgtgag attgattcaa aaatgcagtg aggtagattt gaatattgtc 3780

atattatgga aggcatacgt tgtggaagac agtaaacagc ttattttgga aggtcaacat 3840

catgttattc ttcgcactat aggaaaagaa gccttttcat atcctcagaa acaggagcca 3900

ccagaaatgg aactattgaa atttttcagg ccagaaaaca ttacagtttc ctcaaggcca 3960

tcagtagagc agctttctag tctcattaaa acgagtcttc actacccaga atcatttaat 4020

catccatttc atcaaaaaag cctttgttta gtaccagtca ctcttttact ttccaattgt 4080

tctaaggctg atgtagatgt catagttgat cttcggcata aaacaacaag tccagaagca 4140

ctggaaatcc atggatcatt cacatggctt ggacaaacac agtataaact tcaacttaaa 4200

agccaggaga ttcacagtct gcagctgaaa gcatgctttg ttcatacagg tgtttataac 4260

cttggaactc ctagggtatt tgccaagtta tcggaccaag ttacagtgtt tgaaacaagt 4320

cagcagaatt ccatgcctgc cctgatcatc atcagtaatg tgtgacaact tggaaatttg 4380

tactgaaatc cacaataatc agtttttgct ggatgggttt tacagcagta tttgatatac 4440

ctaacttgtt atggaggttg attgatatct gatccctgca aaatactttg acttgtcatt 4500

ttgttgatga tgcaaagcac gttggactga gaatacttaa cattcttttt ctgtatttct 4560

ttaaaccctg agaataattt acatgctcat aatacaggat atcagcatat ttgtgcacct 4620

tattaagccc catcttaaga aaacacaaag tctaagtctg ctgttacaac ttgtcaatgg 4680

tatacgaata ttaggagatg attctgagaa aggaaaggcc ttgttggcag tactcctgtt 4740

aagccattag tctctaaatt ccagctttac tgtgaagttc tatagagtgt taaatacaaa 4800

ttttcctgtc ttgcttcaca cagttcctta aaatcagttt tgaactttgg tcatagagtc 4860

ttcatatttc agtatttggt ggtccctatg acttatacat aactttgt
4908

<210> 17
<211> 435
<212> DNA
<213> human

<220>
<221> misc_feature
<222> (30)..(30)
<223> any kind of base

<220>
<221> misc_feature
<222> (49)..(49)
<223> any kind of base

<220>
<221> misc_feature
<222> (75)..(75)
<223> any kind of base

<220>
<221> misc_feature
<222> (76)..(76)
<223> any kind of base

<220>
<221> misc_feature
<222> (78)..(78)
<223> any kind of base

<220>
<221> misc_feature
<222> (79)..(79)
<223> any kind of base

<220>
<221> misc_feature
<222> (109)..(109)
<223> any kind of base

<220>
<221> misc_feature
<222> (136)..(136)
<223> any kind of base

<220>
<221> misc_feature
<222> (137)..(137)
<223> any kind of base

<220>
<221> misc_feature
<222> (149)..(149)

<223> any kind of base

<220>
<221> misc_feature
<222> (227)..(227)
<223> any kind of base

<220>
<221> misc_feature
<222> (236)..(236)
<223> any kind of base

<220>
<221> misc_feature
<222> (246)..(246)
<223> any kind of base

<220>
<221> misc_feature
<222> (342)..(342)
<223> any kind of base

<220>
<221> misc_feature
<222> (363)..(363)
<223> any kind of base

<220>
<221> misc_feature
<222> (389)..(389)
<223> any kind of base

<220>
<221> misc_feature
<222> (426)..(426)
<223> any kind of base

<400> 17
ggtagaaatg attgtgatgt acaaattttn tattttgatc atacttaana agacagagca
60

gactcacatt cattnncnna atagtatcac tgtacacata gcgaatttnt ggcgctttta
120

gattgctctg aaaatnnctg aagagttgnc catagcagcc tggtaagcct tttcctttcc
180

cccaaagctc tcctgcccctt tgcagaaaga ctgttggtga caactgntgc taactnaata
240

gcatgnggtt gaacttcgcc aaaatccttc cacctcctcc catagggcaa caggggtgac
300

ttgggcttaa agggcattga gtaagcaagt aggttatcag anaacagagg gaagattcca
360

ttntagataa tttccaaata ttacaattng tggaactcag agttcaactg ctcagttcct
420

tcttcngctg accct
435

<210> 18
<211> 2224
<212> DNA
<213> human

<400> 18
ctttagatct gtgcagcctt tgcgtgccaa acttgtgaaa ttccttttac ctttttttgga
60

gtacttgcta taaagccacc tgtcaacaaa cccccattat gtacagaata ggacctatcc
120

agtagccagg ccagtaggca gttggggaag gtgggaagga tccagcgagg cccctgagcc
180

tgcacctgga caggtgtacg tctgcaccca tcaccctcag caccaggcca ccctgcagtc
240

cacttactgt actgtgttgt ggaaggatat gctaagtgat gaaagttgcg agcagtctca
300

ctggtcgtgt aaactttttt tttttttgg aaattgaagc tgtagagtgc tgcccgaaat
360

ctctaggaag ttggtggcaa gggacagcac tcacactctt ctggtcatga tctctgatct
420

ccacctcaaa tgacaataaa aaactggtcc aacgaagaca ctgctcagca cttcagccat
480

caggactaat ccatcgatga ctggaaaaga ggctagcttt gaggaaaaca gcctgggctc
540

ttgggagcag agtccagtgg gtgtgaggct gacttgccga cggtcggcag gtaatggctc
600

tcagccggcg aggcggtccc acagctctcc tcccagggca gcctgaggag gaggaggccg
660

ggtgcctgtt tggtggcagc ttcagcctag ggatacctga agctgttgag caacaccttt
720

atgaaatgtt gccagagcag caacacttcc ctgtgggcac agccccggga aatccggtac
780

caagtgagca aggtggcagg acccacccaa gcctgatacg catctgggcc cgccgggctc
840

agcaggggag gctgctacgg ctgcccactt cccagcaccg tctgtcaggc ttgaacccct
900

ctgtgctgtt cccttcctgg ctaataggga gacccttcgc aggcacccac tgtttcaact
960

tgaccctccc accccctgct actctcctcc acacacccct ccgttccgct agcctaccct
1020

gtcagccttt caataaaagt tatgcacaaa tgtgaacacc tgagatggag ctgaacattt
1080

cttcactttg ttctttttct gaagtcaaac tcttatcaaa tgccctaaaa ttattaccac

1140

ccaagagaaa caggaaaaag gttacatgtt tttgtttact gagagtaaga tcacctgcat
1200

ctggaagacg ggctggtaaa ttggtttggc tacagaacag aaagaaaaca aaaacaaacc
1260

tcgtaaggga agtatcgcac tcagacacca ccacttccta gagccaaatg agcaatccca
1320

aactgcaagt gccgtaagtg ggcctgtgac gtcacaccgc ccggcccgag gtatcgcatg
1380

tgcgggggag gcccacacta cagctgtcct ctcgtctaga aggcaccacc tcgctttcat
1440

gtcccgtgtg ttttggaaaa gcagtatggt gtgtcatgtc tagcggcgaa cacttccctc
1500

cctctgtcct tgaggttgta atataaaaac tgtgtttctg tacgtgtggg tgggaattct
1560

ctgacggtgc tcgttcatag cacaagctta cgctgagttc tgaactgtcg ttcacagctg
1620

cgtgtctgca tggtgtcgca tctgttgtac ctttggggaa aatttgtatg taaatgtaca
1680

gaaataaaaa cgttgcccca ttaacagatt tcctctggaa tgtcttccct acctcacctg
1740

atggtatcca ccgaagggca tttcactacc attaatggtg agtaataaaa tcctccgtgt
1800

tcattcagac ctcactgcgt cactactttg aacgcctctg taagctgtgt cttcacccgc
1860

cccgaggtgg gtggagggag gcctctcact ctgcttcgag tcctggtctt aaaggtagtc
1920

agaggcagag gctggattaa acacacactg tttaccaagt gccactctca gaccacctga
1980

gagacggggg gccatcagta aaattaagag gaattttttt cccttgttcg tgtatgttct
2040

gctgatccgt ggcctgaagg ttcctagaga cgtcaagaaa tgaatatctt acactgtgat
2100

tctgtgagga aagactggta acccaaaact ctcttctcta atgtattttt taacgaaaat
2160

gacaatattt ctttaataaa gtatttatac caaaaaaaaa aaaaaaaaaa aaaaaaaaaa
2220

aaaa
2224

<210> 19
<211> 2244
<212> DNA
<213> human

<400> 19

gttgtttaaa agcaaggcat gcttgtggat gactctgtaa cagactaatt ggaattgttg 60

aagctgctcc ctggttccac tctggagagt aatctgggac atcttagtgt tttgttttgt 120

ttttttccct cctctttttt tggggggggag tgtgtgtggg gtttgttttt tagtcttgtt 180

tttttaattc attaaccagt ggttagcctt aaggggagga ggacggattg attccacatt 240

ccacttccta gatctagttt agaaaacatg ttccccatct ggtgctctta ggaaggagta 300

tagtaaatgc ctcatttaat aacatactcc tttttgaaag ttgccttttc tctccaccct 360

tgagtagatc cagtatttga tgaaactcat gaaagtgggt ggagcccatc ttccccctcc 420

tcttttctag gacgcactat atgtgactgt gactttaagg acatttgttt gccatttgct 480

gatttttttg ggaagttaat ttctaacttc tttcactgat aaatgaagaa aagtattgca 540

cctttgaaat gcaccaaatg aattgagttt gtaattaaaa aaattttttt tccctttcag 600

tcattgtctt atatgcttag catagatttg cagctcagta gtatatgtgt tcctagaatg 660

cagctgaaga cctgttatgt agaggaaata cgaggggtgg tgctagaaga cagacatctg 720

tggaatgatt cacatcctct caagttagga ggatggaggc ctgcttcatt aagaagctgg 780

gggtagggtg ggggtgggga gaacacttaa caacatgggg accagtcagg ggaatcccct 840

tatttctgtt ttgcatatga ggaaccctag agcagccagg tgaggctctc tagtttaata 900

aaaatcatgg aaagactctt aatgcagact cttcttaagt gttaataggg atttttttcag 960

cttattttgg ttgcagtttc caatttttaa aaatgttgag gtaatctttc ccaccttccc 1020

aaacctaatt cttgtagatg cattagtgtt gaaccaatgc ttctcatgtc tcaatcttgt 1080

atatcatctt ttcagatgta ttaacaaaca aaaccttaaa aagagtagat gaattgccaa 1140

acacaattcc taccaataat aaatcgatca actctatcta ttcaggaaag caggaagcat 1200

ttggaccaca gtgcatgaaa acttcaacat tctgttatta gataatgaat caaccaaatg 1260

aacaatccag agaaaagaaa attgcaataa taaaaggtaa attaacagaa agataatata 1320

agcaagatag taatagttga ccattctgaa aagcttataa catcactcat catccagcat
1380

cctttctgaa aacaaaggat ttttaaatca ctttatgcac atatacaaca taggaggttg
1440

gcaaaataat gcactatttc ttaacagcca tgtctcttgt agaacttcaa gttaatctac
1500

aaatgaccat tgtgtcttaa tttagattat gaataccaca ttagtcaggt atttgcacta
1560

acccttaata gtatatacag tttctatgga aaattcagtg gtccaaaaat ttccgtagaa
1620

tttgagagga cgttggtggg ctgaagatag ctccttgagg gtcactgatg taggctgcaa
1680

tgggggttca caaggccctg acaccgtatt tatagtctaa cctttttatg aaaatctgac
1740

tacagctatt taaggagtag tcttaatagc tgaaaatgaa gatagagaaa gacaccaaga
1800

atatgacaca gtttacattc tagtgaggga cacaacaaaa tcaaatttaa aaaagagtgt
1860

aatagatgct gataaatact gtagataaag cacataagaa aatagaaata aaggctgtca
1920

atggagaagt catgattttt attttattta tttatttatt tatttgagac agagtcaggc
1980

tctgtgcagg ctggagtgca atggtgtgat ctcgctcact acaacctctg ctcctggctc
2040

aagctatcct cccacctcag ctctcaagta gctgggatca caggtgcgtg ctaccatgcc
2100

cggctaattt tttgtagaga tgaggttttg ccatgttgcc caggctggtc tcgaactcct
2160

ggactcaact gaccccacct cggcctctca aagtgctgag attataggcg tgcagccggc
2220

agctggccat tgtttatgtt ctgc
2244

<210> 20
<211> 351
<212> DNA
<213> human

<220>
<221> misc_feature
<222> (62)..(62)
<223> any kind of base

<220>
<221> misc_feature
<222> (121)..(121)
<223> any kind of base

<220>
<221> misc_feature
<222> (207)..(207)
<223> any kind of base

<220>
<221> misc_feature
<222> (220)..(220)
<223> any kind of base

<220>
<221> misc_feature
<222> (276)..(276)
<223> any kind of base

<220>
<221> misc_feature
<222> (300)..(300)
<223> any kind of base

<220>
<221> misc_feature
<222> (315)..(315)
<223> any kind of base

<220>
<221> misc_feature
<222> (336)..(336)
<223> any kind of base

<400> 20
tctacttcca catcggcgag accgagaagc gctgtttcat cgaggaaatc cccgacgaga
60

cnatggtcat cggcaactat cgtacccaga tgtgggataa gcagaaggag gtcttcctgc
120

nctcgacccc tggcctgggc atgcacgtgg aagtgaagga ccccgacggc aaggtggtgc
180

tgtcctggca gtacggctcg gagggcnctt tcacgttcan ctcccacacg cccggtgacc
240

atcaaatctg tctgcactcc aattcttacc aggatngctc tctttcgctg gtgggcaaan
300

tgcgtgttgc atctngacat ccaggtttgg gggagnatgc caacaaatta c
351

<210> 21
<211> 2631
<212> DNA
<213> human

<400> 21
accttccaac ccagccctcg gctgagccgc gccgcaccat gcccgccgtg gacaagctcc
60

tgctagagga ggcgttgcag gacagccccc agactcgctc tttactgagc gtgtttgaag

120

aagatgctgg caccctcaca gactatacca accagctgct ccaggcaatg cagcgcgtct
180

atggagccca gaatgagatg tgcctggcca cacaacagct ttctaagcaa ctgctggcat
240

atgaaaaaca gaactttgct cttggcaaag gtgatgaaga agtaatttca acactccact
300

atttttccaa agtggtggat gagcttaatc ttctccatac agagctggct aaacagttgg
360

cagacacaat ggttctacct atcatacaat tccgagaaaa ggatctcaca gaagtaagca
420

ctttaaagga tctatttgga ctcgctagca atgagcatga cctctcaatg gcaaaataca
480

gcaggctgcc taagaaaaag gagaatgaga aggtgaagac cgaagtcgga aaagaggtgg
540

ccgcggcccg gcggaagcag cacctctcct cccttcagta ctactgtgcc ctcaacgcgc
600

tgcagtacag aaagcaaatg gccatgatgg agcccatgat aggctttgcc catggacaga
660

ttaacttttt taagaaggga gcagagatgt tttccaaacg tatggacagc tttttatcct
720

ccgttgcaga catggttcaa agcattcagg tagaactgga accgaggcgg aaaagatgcg
780

ggtgtcccag caagaattac tttctgttga tgaatctgtt tacactccag actctgatgt
840

ggccgcacca cagatcaaca ggaacctcat ccagaaggct ggttacctta atcttagaaa
900

caaaacaggg ctggtcaccg ccacctggga gaggctttat ttcttcaccc aaggcgggaa
960

tctcatgtgt cagcccaggg gagccgtggc tggaggtttg atccaggacc tggacaactg
1020

ctcagtgatg gccgtggatt gcgaagaccg gcgctactgc tttcagatca ccacgcccaa
1080

tggaaaatcg ggaataatcc tccaggctga gagcagaaag gaaaatgaag agtggatatg
1140

tgcaataaac aacatctcca gacagatcta cctgaccgac aaccctgagg cagtcgcgat
1200

caagttgaat cagaccgctc tgcaagcagt gactcctatt acaagttttg gaaaaaaaca
1260

agaaagctca tgccccagcc agaacctgaa aaattcagag atggaaaatg aaaatgacaa
1320

gattgttccc aaagcaacag ccagtctacc tgaagcagag gagctgatcg cgcctggagc
1380

gccgattcaa ttcgatattg tgcttcctgc tacagaattc cttgatcaga acagagggag

1440

caggcgtacc aacccttttg gtgaaactga ggatgaatca tttccagaag cagaagattc
1500

tcttttgcag cagatgttta tagttcggtt tttgggatca atggcagtta aaacagacag
1560

cactactgaa gtgatttatg aagcgatgag acaagtattg gctgctcggg ctattcataa
1620

catcttccgc atgacagaat cccatctgat ggtcaccagc caatctttga ggttgataga
1680

tccacagact caagtatcaa gggccaattt tgaacttacc agtgtcacac aatttgctgc
1740

tcatcaagaa aacaagagac tggttggttt tgtcatccgt gttcctgaat ccactggaga
1800

agaatctctg agtacataca tttttgaaag caactcagaa ggcgaaaaga tatgttatgc
1860

tattaatttg ggaaaagaaa ttattgaggt tcagaaggat ccagaagcac tggctcaatt
1920

aatgctgtcc ataccactaa ccaatgatgg aaaatatgta ctgttaaacg atcaaccaga
1980

tgacgatgat ggaaatccaa atgaacatag aggcgcagaa tccgaagcat aactcacttg
2040

cgcctgtggg ggaagagcga acaggaagga gagctacctc ctaagggttt taacgtctct
2100

gacatacagg cacactgacc tgatttccga aggctgacaa tcgtttgtgg aatgtaatct
2160

tgatgccttg atactgagac ttgggaggga aactaagaaa tggttgacag cgttcccacc
2220

catctacaat gttattttag gtgctttgtg gtaagtcttt tttcttagat tgcgctaaaa
2280

tttcttagat tgttcagcgc tcagaacaaa agtttgaaaa atgcattgtt catatgaatg
2340

tcatctcttt tcagtttcca gtatcctttt taaaaaatgg caaaagccta gatttacaat
2400

ttgatgaaca ctaaatattt cttattaata taatctattt ttgtatttta cttaatgagc
2460

tttaagtgcc tgtcgttctg aaaattgtgt atttataatt cagcttatct cataattgga
2520

cctaatagca tttctttgtg cagttaggtg atgagcactg ctttgaggcc caagcactag
2580

tagagatgcg cgatacaggt ctagtttcgg taactgttcc agacatcaag c
2631

<210> 22
<211> 2851
<212> DNA

<213> human

<400> 22
agcatctcag gccatcatcc tgaaacttgg cagccttcgt ggagtataag gacagcatta 60

ttagccatca ttgggtttac tgccaacaaa aggagaggga gccataggtt ctctagatta 120

cactcctgag gaaagaagag cacttgccaa aaaatcacaa gatttctgtt gtgaaggatg 180

tggctctgcc atgaaggatg tcctgttgcc tttaaaatct ggaagcgatt caagccaagc 240

tgaccaagaa gccaaagaac tggctaggca aataagcttt aaggcagaag tcaattcatc 300

tggaaagact atctctgagt cagacttaaa ccactctttt tcactaactg atttacaaga 360

tgatatacct acaacattcc agggtgctac ggccagtaca tcgtacggac tccagaattc 420

ctcagcagca tcctttcatc aacctaccca acctgtagct aagaatacct ccatgagccc 480

tcgacagcgc cgggcccagc agcagagtca gagaaggttg tctacttcac cagatgtaat 540

ccagggccac cagccaagag acaaccacac tgatcatggt gggtcagctg tactgattgt 600

catcctgact ttggcattgg cagctcttat attccgacga atatatctgg caaacgaata 660

catatttgac tttgagttat aatatggttt tgtgacttat gagctgtgac tcaactgctt 720

cattaaacat tctgcattgg gtataatcta agaattgttt acaaaaagat tattttgtat 780

ttacccttca ttcctttttt tgatccttgt aagtttagta taaatatatc tagacattca 840

gactgtgtct agcagttacg tcctgcttaa agggactaga agtcaaagtt ccttgtctca 900

ctatttgatc tgctttgcag ggaaataact tgttttttct catgtttcat cttcttttta 960

tgtaaatttg taatactttc ctatattgcc ctttgaaatt tttggataaa agatgatgtt 1020

ttaagttcca atgagtatta ctagttactc aataccactt attgagtact ctgtttctac 1080

gtatgtagaa tgtataggga tagaagagtt gaaaagggaa agcaaaactt cttaagtggc 1140

ttccttaaaa tgtcattcat aggagatgta ctggaattgc tcattctgtg actttatttg 1200

tgtcctaaac attcttcagt gaaaataatt ttatttcagt caaacattta tgaggaaatg 1260

agatcacatc tttgtcactg gatgctactt gaagagggag tactttgtaa ccactttgat
1320

atgctgttat caccaccccc tgccctctgc tgccataatc acacaaattt aaaaagaaag
1380

aaaacagtct tccatagatt tttaaggaag aaagggccca agtcaggaga tcgcttggtt
1440

ttcttccaga agttaaatgg ggggatctga agatttgaat gttcggtctg ctttgaaatg
1500

tatgtctttt gggaatgtat tatatgccta gctttataat caggtataaa attttaatta
1560

ttcccaggaa tatgcataat attgaatatt tcatgtccta ttttaataga aaacctcagg
1620

gcccaagtaa ccagtgatag aagttagaaa aaccccttta cttagaattg tccacctagt
1680

cagagcccaa gaaagaattt tcagtggaaa aatcaatata taacttagtg ctagctagcg
1740

ccacagactc tagtagataa tattatcatc ataatggctg gtgaaaccat ataatcacag
1800

aaaaacattg ccttcagcat gttcagttcg cagcactgag ggcactcttg agggtgttgt
1860

taatgaagat ttaattttta aatacaggtg gttccaagct ttcaaatagg ttatgctcca
1920

aaagtgttat ttgtaagtta atttttttac aagtcaaaca atgttggaag tggtatttag
1980

gttctagatc ggtccacgaa agttagccca tatgtatatc ttgaatagta taggggaggg
2040

tattcataaa gtccttatgt ggttttaact aagtgaaatt atggacaaga gaaataattg
2100

taaaatcgtc ttaaaggcaa atttaatttt tacccctgtt tatgggacat tcgttctatt
2160

aactgtcaga cacaatttct gttttcatct gagagccagg tttcctttat ttctacatct
2220

aaaataagaa catattgtac actattatat aatacagaat tgtcttacac tttaataaat
2280

tcgcatttta aaggtgttta caggattatt ttttatatct gtagctgaat ttgttaaagt
2340

ctaaaaagct caaggacttt atgaagatct cattatatga ggaaaatcat aggttaccat
2400

tttataactc tattgccata agaaaataca ctctaaaatc ttgatttgaa acatattaga
2460

aaccttgatt cagtgctcag tggtctccta gtaagaagtc accgacggta gcgtcatatg
2520

agaagaaaga aatccccacc acctcaacct ctgctgagat tgtgtgctag gaacagcctt
2580

ccctccgttt cccctcagtc aaacttgagc cagcctctgg atcgatgtga tcttattgca 2640

tgtttccatg gggtgtacct atactttaag ccaatcctgc tgcattcact gctaagttaa 2700

ataaaaagcc aagaagaaaa aaaaaatttt gcactgtgca gatcctttgc tatctgactt 2760

gcatctcttc ccccacctgt cagctagcca cctgcttgtt tgtgttggga tattttttag 2820

cacctgaagc accatctgaa aggggcacca t 2851

<210> 23
<211> 3473
<212> DNA
<213> human

<400> 23
aagagcagcg gcgaggcggc ggtggtggct gagtccgtgg tggcagaggc gaaggcgaca 60

gctctagggg ttggcaccgg ccccgagagg aggatgcggg tccggatagg gctgacgctg 120

ctgctgtgtg cggtgctgct gagcttggcc tcggcgtcct cggatgaaga aggcagccag 180

gatgaatcct tagattccaa gactactttg acatcagatg agtcagtaaa ggaccacact 240

actgcaggca gagtagttgc tggtcaaata tttcttgatt cagaagaatc tgaattagaa 300

tcctctattc aagaagagga agacagcctc aagagccaag agggggagag tgtcacagaa 360

gatatcagct ttctagagtc tccaaatcca gaaaacaagg actatgaaga gccaaagaaa 420

gtacggaaac cagctttgac cgccattgaa ggcacagcac atggggagcc ctgccacttc 480

ccttttcttt tcctagataa ggagtatgat gaatgtacat cagatgggag ggaagatggc 540

agactgtggt gtgctacaac ctatgactac aaagcagatg aaaagtgggg cttttgtgaa 600

actgaagaag aggctgctaa gagacggcag atgcaggaag cagaaatggt gtatcaaact 660

ggaatgaaaa tccttaatgg aagcaataag aaaagccaaa aaagagaagc atatcggtat 720

ctccaaaagg cagcaagcat gaaccatacc aaagccctgg agagagtgtc atatgctctt 780

ttatttggtg attacttgcc acagaatatc caggcagcga gagagatgtt tgagaagctg 840

actgaggaag gctctcccaa gggacagact gctcttggct ttctgtatgc ctctggactt 900

ggtgttaatt caagtcaggc aaaggctctt gtatattata catttggagc tcttgggggc
960

aatctaatag cccacatggt tttgggttac agatactggg ctggcatcgg cgtcctccag
1020

agttgtgaat ctgccctgac tcactatcgt cttgttgcca atcatgttgc tagtgatatc
1080

tcgctaacag gaggctcagt agtacagaga atacggctgc ctgatgaagt ggaaaatcca
1140

ggaatgaaca gtggaatgct agaagaagat ttgattcaat attaccagtt cctagctgaa
1200

aaaggtgatg tacaagcaca ggttggtctt ggacaactgc acctgcacgg agggcgtgga
1260

gtagaacaga atcatcagag agcatttgac tacttcaatt tagcagcaaa tgctggcaat
1320

tcacatgcca tggccttttt gggaaagatg tattcggaag gaagtgacat tgtacctcag
1380

agtaatgaga cagctctcca ctactttaag aaagctgctg acatgggcaa cccagttgga
1440

cagagtgggc ttggaatggc ctacctctat gggagaggag ttcaagttaa ttatgatcta
1500

gcccttaagt atttccagaa agctgctgaa caaggctggg tggatgggca gctacagctt
1560

ggttccatgt actataatgg cattggagtc aagagagatt ataaacaggc cttgaagtat
1620

tttaatttag cttctcaggg aggccatatc ttggctttct ataacctagc tcagatgcat
1680

gccagtggca ccggcgtgat gcgatcatgt cacactgcag tggagttgtt taagaatgta
1740

tgtgaacgag gccgttggtc tgaaaggctt atgactgcct ataacagcta taaagatggc
1800

gattacaatg ctgcagtgat ccagtacctc ctcctggctg aacagggcta tgaagtggca
1860

caaagcaatg cagcctttat tcttgatcag agagaagcaa gcattgtagg tgagaatgaa
1920

acttatccca gagctttgct acattggaac agggccgcct ctcaaggcta tactgtggct
1980

agaattaagc tcggagacta ccatttctat gggtttggca ccgatgtaga ttatgaaact
2040

gcatttattc attaccgtct ggcttctgag cagcaacaca gtgcacaagc tatgtttaat
2100

ctgggatata tgcatgagaa aggactgggc attaaacagg atattcacct tgcgaaacgt
2160

ttttatgaca tggcagctga agccagccca gatgcacaag ttccagtctt cctagccctc
2220

tgcaaattgg gcgtcgtcta tttcttgcag tacatacggg aaacaaacat tcgagatatg 2280

ttcacccaac ttgatatgga ccagcttttg ggacctgagt gggaccttta cctcatgacc 2340

atcattgcgc tgctgttggg aacagtcata gcttacaggc aaaggcagca ccaagacatg 2400

cctgcaccca ggcctccagg gccacggcca gctccacccc agcaggaggg gccaccagag 2460

cagcagccac cacagtaata ggcactgggt ccagccttga tcagtgacag cgaaggaagt 2520

tatctgctgg gaacacttgc atttgattta ggaccttgga tcagtggtca cctcccagaa 2580

gaggcacggc acaaggaagc attgaattcc taaagctgct tagaatctga tgcctttatt 2640

ttcagggata agtaactctt acctaaactg agctgaatgt ttgtttcagt gccatatgga 2700

ataacaactt tcagtggctt tttttttct tttctggaaa catatgtgag acactcagag 2760

taatgtctac tgtatccagc tatctttctt ggatcctttt ggtcattatt tcagtgtgca 2820

taagttctta atgtcaacca tctttaaggt attgtgcatc gacactaaaa actgatcagt 2880

gtaaaaagga aaacccagtt gcaagtttaa acgtgttcga aagtctgaaa atagaacttg 2940

ccttttaagt taaaaaaaaa aaaagctatc ttgaaaatgt tttggaactg cgataactga 3000

gaaactctta ccagtccaca tgcaattaga catattcagc atatttgtta ttttaaaagg 3060

gagggttggg aggtttctta ttggtgattg tcacacggta taccatactc ctctccttca 3120

aagaatgaaa ggccttgtta aggagttttt tgtgagcttt acttctttgg aatggaatat 3180

acttatgcaa aaccttgtga actgactcct tgcactaacg cgagtttgcc ccacctactc 3240

tgtaatttgc ttgtttgttt tgaatataca gagccttgat ccagaagcca gaggatggac 3300

taagtgggag aaattagaaa acaaaacgaa ctctggttgg ggtactacga tcacagacac 3360

agacatactt ttcctaaagt tgaagcattt gttcccagga tttattttac tttgcatttc 3420

cttttgcaca aagaacacat caccatttcc ttttgcacaa agaacacatc acc 3473

<210> 24

<211> 401
<212> DNA
<213> human

<220>
<221> misc_feature
<222> (252)..(252)
<223> any kind of base

<220>
<221> misc_feature
<222> (303)..(303)
<223> any kind of base

<220>
<221> misc_feature
<222> (390)..(390)
<223> any kind of base

<400> 24
ttagattatt ttcaatttat tattcagaat aaatatatct tttttcttta acttctcaaa
60

tagttattga attgtattgg tttaaattaa atgcgtcatg tgtatatatc agtattaatt
120

caagagatac aaaaggaaat tgagtgaaaa ataagtctgc ctccttccca tcactctcat
180

gtctctacct agaggcaatt attgtcaaca gtttttgatg tgtctttcaa aaaatagtcc
240

attaagcctg gngtactaga tctcttttaa aagtttacaa cctgttacag aatatatata
300

aangttcaat tactagtaac accttattac agatacagat tacaacttag gaaatatatt
360

ttcatggacc attgatgtca tttggattcn cccctacaat c
401

<210> 25
<211> 1820
<212> DNA
<213> human

<400> 25
aatgtcttag aaaaaggctt tctaaaagaa aaagagcaag aggccatttc ttttcaagat
60

agatacaaag aacttcagga aaaacataaa caagaattgg aagacatgag gaaagctggt
120

cacgaagccc tcagcattat tgtggatgaa tataaggcac tactgcagtc ttcagttaag
180

caacaagtag aagctattga aaaacagtac atttctgcaa ttgagaaaca ggcacacaag
240

tgtgaggagt tgctaaatgc tcagcatcag aggctccttg aaatgctaga tacagagaag
300

gaactgttaa aagaaaaaat aaaggaagct ttgattcagc aatctcaaga acagaaggaa 360

atattggaaa agtgtttgga ggaagaaagg caaagaaata aagaggcatt agtatccgct 420

gcaaagcttg aaaaagaagc agtgaaggat gcagttttaa aagtcgtaga agaagaaaga 480

aaaaatttag aaaaagcgca tgctgaagaa agggaattat ggaagacaga acatgcaaaa 540

gatcaagaaa aagtatctca ggaaattcaa aaagctatac aagaacaaag aaaaataagt 600

caggaaactg ttaaggcagc aataatagaa gagcagaaac gaagtgaaaa ggctgtggaa 660

gaggcagtga aaagaacaag agatgaattg atagagtata taaaagaaca gaaaaggctc 720

gatcaagtca tccgccaaag aagcctgtcc agtttggaac tgttcctctc ctgtgcacag 780

aaacagttaa gtgctttaat agctacggaa ccagttgaca ttgaataaaa agaacatgac 840

aaacccacac tggcattgga taaatcatat tacaccttca aaatacacac tctgaattat 900

aaagatgtgt ttgttttctt tccaaatcat gtagaattga tttccagttc aaggataaac 960

caaaacaata tttagaacta tcaagtgatc taatttattt tcttttggtt tcttctttac 1020

atttactgtt attttattat tattagtagt agcagcaaca gagtatgata tgacccaaaa 1080

gccattgtaa agtgccacat taccaaaatt aattaagtaa actttatagc ctgtgggagt 1140

ctattatata ttattttgca aaagtagtaa atatattatt gtttcatgat gactcttgat 1200

gagatgctag aatgtaacca tacatttatc ttattttgag gatagaaata gcatggattt 1260

caacatcact tatttatctg tataattgga aataaaacac cgatatgata gagaatcatt 1320

ccggcattac ctaacctctt ctgcagttgg atctatgtat tttcattggt ctactgaaaa 1380

cgaacaatac aattaaaagc actaaagatt attatattaa ttcaactttg atctgatata 1440

tcacttaaac taaaggggtg tgtgtggtgt atgcttgttt cctatttctg ctctttaaag 1500

atactttgaa tcaataaaac cattagtcta caaatcaaat tgtgaactta atctctagaa 1560

agagaatata actcagccat ttataggaat ttaggttcaa gtacaggata tatgaaatct 1620

tttcccagta tttcagaatg tacttaattc acaggcagga tgcttcaatg caaaatcatg
1680

aatattttta attcaaaact aaaatgtcat taatatgtat gtatgcaaat gttttatctt
1740

attttctgaa atgcatctac tttcatgggc tttgtacgtt tctgagattt ctcagtgtaa
1800

taaaaagagc tcccaaactt
1820

<210> 26
<211> 280
<212> DNA
<213> human

<220>
<221> misc_feature
<222> (261)..(261)
<223> any kind of base

<220>
<221> misc_feature
<222> (237)..(237)
<223> any kind of base

<400> 26
tcaagtcata agataaagtt taatcatttg atcatgttaa aagacacaaa acacagccaa
60

tctaaccaaa tttcaggcat gcatttacat aaatatatta aattaagaaa agaaattgta
120

cacttaaacg tccttttcac ctagaaatca ttaaatccac agatcaacaa taaaaccaat
180

tctctgcatt taccacttca agatacaatt gttctatttt aaagataaca caaactncac
240

tagtctggtt aggaatttat ntgcattata catatattat
280

<210> 27
<211> 392
<212> DNA
<213> human

<400> 27
ttggtttgaa atggcacccc aggactttgg gcctgcctta cttgatagcc tcgttcagtg
60

agcaaagact tagtgagcag ctcttgtatg ccaagtattt tgctaagctc tggaaaaaag
120

ataaacaaga catggttctt gctttcaagg agtgtgtaat tctttagcca gatatggaaa
180

cctggaccct gagtgggaga aaggagacag atgaaaggag tccgtgattt tgtaaccaag
240

agctgcctgc atggttatga gtatcactga ttttagggac gcccacagag ctaaagcatt

300

tttttaatcc gagaagactt ttgtaactca tattagttaa tcttctagct ctgagatagc
360

aacacagctc ttagaattct gtaagtaagc tt
392

<210> 28
<211> 2299
<212> DNA
<213> human

<400> 28
cgaaccccca cagctggagg gcgaggccag ctgtacccgg ccccagtgcc ctttcgcggc
60

cacaagcggc cgtcctcctg gtccggtgct ccggcgcctg atctaggttc atggagccgg
120

ggctgtggct ccttttcggg ctcacagtga cctccgccgc aggattcgtg ccttgctccc
180

agtctgggga tgctggcagg cgcggcgtgt cccaggcccc cactgcagcc agatctgagg
240

gggactgtga agagactgtg gctggccctg gcgaggagac tgtggctggc cctggcgagg
300

ggactgtggc cccgacagca ctgcagggtc caagccctgg aagccctggg caggagcagg
360

cggccgaggg ggcccctgag caccaccgat ccaggcgctg cacgtgcttc acctacaagg
420

acaaggagtg tgtctactat tgccacctgg acatcatttg gatcaacact cccgaacaga
480

cggtgcccta tggactgtcc aactacagag gaagcttccg ggcaagagg tctgcggggc
540

cacttccagg gaatctgcag ctctcacatc ggccacactt gcgctgcgct tgtgtgggga
600

gatatgacaa ggcctgcctg cacttttgca cccaaactct ggacgtcagc agacaggttg
660

aagtcaagga ccaacaaagc aagcaggctt tagacctcca ccatccaaag ctcatgcccg
720

gcagtggact cgccctcgct ccatctacct gcccccgctg cctctttcag gaaggagccc
780

cttaggagga caggcctgca gcatcctggt ctcgggaggc ttctgtcatt gctcacacac
840

agttcagatt tccacctctt tatagacaag aagtgaattt gcctggggca gaacacccac
900

ccaaagagtc cccacttaac aataccccc ccccacggca agaatgccca aatccgaatg
960

accccagttt tcctaatgag taaaatgatc ccagatgtgc cccagagcat gacgcctgca
1020

gctccggttt catgcaggaa attggttttg gagagttttg gcaagttgga aagccactta
1080

ctggcttttg acatgacttc tcttggagaa taagtggact ccaagctaac tctttgcaaa
1140

tgtaaacaca tgtccatctt gtaataaatg caaaatgccc gtgcagcaga agcatgcgac
1200

tttcatatcc ttgcctagaa taggctgcat ggtgtatgtc agtgagggcc acgaggcgtc
1260

ggctttagac acagatcata gctctacagg agtttatgaa tttgaagctt atgggatttt
1320

ggcagagaaa ttttcagctg tgcttgatac ccaccaaaag aatgtatctc gaaagaatga
1380

aggaagaaga aaaaaggatc cttgatgttt gtgacaagaa aatgagaaag ttagtatctg
1440

caatacagag cttgttcctg ttcagtgact gaccctctgt attctgtata gacaccaggc
1500

cgatacacag tggagttccc aggccttgtt tgcaggaagc cgactgtaaa gacagcccca
1560

gctcaaggct attaggttga atatttgctt tcatgagtaa atgtggatct ttggggaatg
1620

gcttcaaaat aagtcacgaa cacaaattct ttgtaaatta tgtaaattcc tgtttatata
1680

aattggcaac aacttatacc gtctgacagt tcaaaatctc tttcagctgc gctcttccca
1740

ccgagccgag cttactgtga gtgtggagat gttatcccac catgtaaagt cgcctgcgca
1800

ggggagggct gcccatctcc ccaacccagt cacagagaga taggaaacgg catttgagtg
1860

ggtgtccagg gccccgtaga gagacattta agatggtgta tgacagagca ttggccttga
1920

ccaaatgtta aatcctctgt gtgtatttca taagttatta caggtataaa agtgatgacc
1980

tatcatgagg aaatgaaagt ggctgatttg ctggtaggat tttgtacagt ttagagaagc
2040

gattatttat tgtgaaactg ttctccactc caactccttt atgtggatct gttcaaagta
2100

gtcactgtat atacgtatag agaggtagat aggtaggtag attttaaatt gcattctgaa
2160

tacaaactca tactccttag agcttgaatt acatttttaa aatgcatatg tgctgtttgg
2220

caccgtggca agatggtatc agagagaaac ccatcaattg ctcaaatact cagaaagtac
2280

tgtcaaaagc ctaataaaa
2299

<210> 29
<211> 1339
<212> DNA
<213> human

<400> 29
ctaaacaaaa tcattcactt ccctgatttt gataagaaaa ttcctgtaaa gctgtttcct 60

ctgcctctcc tctacgttgg aaaccacata agtggattat caagcacaag taaattaagc 120

ctaccgatgt tcaccgtgct caggaaattc accattccac ttaccttact tctggaaacc 180

atcatacttg ggaagcagta ttcactcaac atcatcctca gtgtctttgc cattattctc 240

ggggctttca tagcagctgg gtctgacctt gcttttaact tagaaggcta tatttttgta 300

ttcctgaatg atatcttcac atcagcaaat ggagtttata ccaaacagaa aatggaccca 360

aaggagctag ggaaatacgg agtacttttc tacaatgcct gcttcatgat tatcccaact 420

cttattatta gtgtctccac tggagacctc caacaggcta ctgaattcaa ccaatggaag 480

aatgttgtgt ttatcctaca gtttcttctt tcctgttttt tggggtttct gctgatgtac 540

tccacggttc tgtgcagcta ttacaattca gccctgacga cagcagtggt tggagccatc 600

aagaatgtat ccgttgccta cattgggata ttaatcggtg gagactacat tttctctttg 660

ttaaactttg tagggttaaa tatttgcatg gcagggggct tgagatattc ctttttaaca 720

ctgagcagcc agttaaaacc taaacctgtg ggtgaagaaa acatctgttt ggatttgaag 780

agctaaagag tctgcagcag gattggagac tgacttgtga ctgcgggctg ggggggcatt 840

cccagtagga atgtgaagcc agaggtttcg gattcgtgac atccaccccc tgggcaagtg 900

agagcatctg caaaatgcaa agagaactac ctcatatgca ggatgagcca atggcagtct 960

caagaaatgt actcgggcga caccttacct gtggaaagca aatcttttca aaataagcca 1020

ctgggactcg gtaggtggag ccccagctgc tcttctaggg acctatgggg ccttcgtggc 1080

atctctgtgc tgtgtgctgg ggaggaggtt gatgtaatgg tgactctttt ctgatcagca 1140

ccttggccgt gattcccaag gtcccagcca aagcaaaggg ccagttgttt cagtttaaac 1200

agacatgtct ttagtctaat aaaattagtt aactgccagt aaagttattt gttagctttg
1260

atgaaagcta tgttggtatc tttccctaat catcaaagta aataaaaaat catttctatg
1320

taaaaaaaaa aaaaaaaaa
1339

<210> 30
<211> 4250
<212> DNA
<213> human

<400> 30
gaacacatcg cgtttgcatc ccagaaagta gtcgccgcga ctatttcccc caaagagaca
60

agcacacatg taggaatgac aaaggcttgc gaaggagaga gcgcagcccg cggcccggag
120

agatcccctc gataatggat tactaaatgg gatacacgct gtaccagttc gctccgagcc
180

ccggccgcct gtccgtcgat gcaccgaaaa gggtgaagta gagaaataaa gtctccccgc
240

tgaactacta tgaggtcaga agccttgctg ctatatttca cactgctaca ctttgctggg
300

gctggtttcc cagaagattc tgagccaatc agtatttcgc atggcaacta tacaaaacag
360

tatccggtgt ttgtgggcca caagccagga cggaacacca cacagaggca caggctggac
420

atccagatga ttatgatcat gaacggaacc ctctacattg ctgctaggga ccatatttat
480

actgttgata tagacacatc acacacggaa gaaatttatt gtagcaaaaa actgacatgg
540

aaatctagac aggccgatgt agacacatgc agaatgaagg gaaaacataa ggatgagtgc
600

cacaacttta ttaaagttct tctaaagaaa aacgatgatg cattgtttgt ctgtggaact
660

aatgccttca acccttcctg cagaaactat aagatggata cattggaacc attcggggat
720

gaattcagcg gaatggccag atgcccatat gatgccaaac atgccaacgt tgcactgttt
780

gcagatggaa aactatactc agccacagtg actgacttcc ttgccattga cgcagtcatt
840

taccggagtc ttggagaaag ccctaccctg cggaccgtca agcacgattc aaaatggttg
900

aaagaaccat actttgttca agccgtggat tacggagatt atatctactt cttcttcagg
960

gaaatagcag tggagtataa caccatggga aaggtagttt tcccaagagt ggctcaggtt

1020

tgtaagaatg atatgggagg atctcaaaga gtcctggaga aacagtggac gtcgttcctg 1080

aaggcgcgct tgaactgctc agttcctgga gactctcatt tttatttcaa cattctccag 1140

gcagttacag atgtgattcg tatcaacggg cgtgatgttg tcctggcaac gttttctaca 1200

ccttataaca gcatccctgg gtctgcagtc tgtgcctatg acatgcttga cattgccagt 1260

gtttttactg ggagattcaa ggaacagaag tctcctgatt ccacctggac accagttcct 1320

gatgaacgag ttcctaagcc caggccaggt tgctgtgctg gctcatcctc cttagaaaga 1380

tatgcaacct ccaatgagtt ccctgatgat accctgaact tcatcaagac gcacccgctc 1440

atggatgagg cagtgccctc catcttcaac aggccatggt tcctgagaac aatggtcaga 1500

taccgcctta ccaaaattgc agtggacaca gctgctgggc catatcagaa tcacactgtg 1560

gtttttctgg gatcagagaa gggaatcatc ttgaagtttt tggccagaat aggaaatagt 1620

ggttttctaa atgacagcct tttcctggag gagatgagtg tttacaactc tgaaaaatgc 1680

agctatgatg gagtcgaaga caaaaggatc atgggcatgc agctggacag agcaagcagc 1740

tctctgtatg ttgcgttctc tacctgtgtg ataaaggttc cccttggccg gtgtgaacga 1800

catgggaagt gtaaaaaaac ctgtattgcc tccagagacc catattgtgg atggataaag 1860

gaaggtggtg cctgcagcca tttatcaccc aacagcagac tgacttttga gcaggacata 1920

gagcgtggca atacagatgg tctgggggac tgtcacaatt cctttgtggc actgaatgac 1980

atttcaactc ctctaccaga taatgaaatg tcttacaaca cagtgtatgg gcattccagt 2040

tccctcttgc ccagcacaac cacatcagat tcgacggctc aagaggggta tgagtctagg 2100

ggaggaatgc tggactggaa gcatctgctt gactcacctg acagcacaga ccctttgggg 2160

gcagtgtctt cccataatca ccaagacaag aagggagtga ttcgggaaag ttacctcaaa 2220

ggccacgacc agctggttcc cgtcaccctc ttggccattg cagtcatcct ggctttcgtc 2280

atgggggccg tcttctcggg catcaccgtc tactgcgtct gtgatcatcg gcgcaaagac

2340

gtggctgtgg tgcagcgcaa ggagaaggag ctcacccact cgcgccgggg ctccatgagc
2400

agcgtcacca agctcagcgg cctctttggg gacactcaat ccaaagaccc aaagccggag
2460

gccatcctca cgccactcat gcacaacggc aagctcgcca ctcccggcaa cacggccaag
2520

atgctcatta aagcagacca gcaccacctg gacctgacgg ccctccccac cccagagtca
2580

accccaacgc tgcagcagaa gcggaagccc agccgcggca gccgcgagtg ggagaggaac
2640

cagaacctca tcaatgcctg cacaaaggac atgcccccca tgggctcccc tgtgattccc
2700

acggacctgc ccctgcgggc ctcccccagc cacatcccca gcgtggtggt cctgcccatc
2760

acgcagcagg gctaccagca tgagtacgtg gaccagccca aaatgagcga ggtggcccag
2820

atggcgctgg aggaccaggc cgccacactg gagtataaga ccatcaagga acatctcagc
2880

agcaagagtc ccaaccatgg ggtgaacctt gtggagaacc tggacagcct gccccccaaa
2940

gttccacagc gggaggcctc cctgggtccc ccgggagcct ccctgtctca gaccggtcta
3000

agcaagcggc tggaaatgca ccactcctct tcctacgggg ttgactataa gaggagctac
3060

cccacgaact cgctcacgag aagccaccag gccaccactc tcaaaagaaa caacactaac
3120

tcctccaatt cctctcacct ctccagaaac cagagctttg gcaggggaga caacccgccg
3180

cccgccccgc agagggtgga ctccatccag gtgcacagct cccagccatc tggccaggcc
3240

gtgactgtct cgaggcagcc cagcctcaac gcctacaact cactgacaag gtcggggctg
3300

aagcgtacgc cctcgctaaa gccggacgta ccccccaaac catcctttgc tcccctttcc
3360

acatccatga agcccaatga tgcgtgtaca taatcccagg gggagggggt caggtgtcga
3420

accagcaggc aaggcgaggt gcccgctcag ctcagcaagg ttctcaactg cctcgagtac
3480

ccaccagacc aagaaggcct gcggcagagc cgaggacgct gggtcctcct ctctgggaca
3540

caggggtact cacgaaaact gggccgcgtg gtttggtgaa ggtttgcaac ggcggggact
3600

caccttcatt ctcttccttc actttccccc acaccctaca acaggtcgga cccacaaaag

3660

acttcagtta tcatcacaaa catgagccaa aagcacatac ctaccccatc ccccaccccc
3720

acacacacac acacatgcac acaacacata cacacacacg cacagaggtg aacagaaact
3780

gaaacatttt gtccacaact tcacgggacg tggccagact gggtttgcgt tccaacctgc
3840

aaaacacaaa tacatttttt aaaatcaaga aaatttaaaa agacaaaaaa aaaagaattc
3900

attgataatt ctaactcaga ctttaacaat ggcagaagtt tactatgcgc aaatactgtg
3960

aaatgcccgc cagtgttaca gctttctgtt gcagcagata aatgccatgt tgggcaacta
4020

tgtcatagat ttctgctcct cctctctttt aatgaaataa cgtgaccgtt aacgcaagta
4080

actctttatt tattgttcac cctttttttc cttaaggaaa ggactcttcc aaatatcatc
4140

ctatgaacag ctcttcagaa agcccattga aagttaaact atttaacgtg aaatccatta
4200

actggaataa ttgagtttct ttatttttac aataaattca ctgagtaaat
4250

<210> 31
<211> 2785
<212> DNA
<213> human

<400> 31
ctttagccca acagtcaaaa ataattgatg ctaccctaca aatgtccaaa actctagtat
60

atcatatttc taagttacag caaatattag tcctgctaaa ccagggagct ttggcaaaaa
120

tgtttttttga cagtaaattt gtccttgatt atatattaac tagtcaaaga ggtgtttgta
180

acattattag agcttcttgt tgtaggtggg ttaacaccac caatcaagag gtcattctaa
240

cagaaagcct ggatcagaaa accatcaccc taaaaaaaca tgccttacat atttaacaca
300

ctctgaaatc cagtcaaaat atgactaaag gcccttgcca tgactgatgt attctcctgg
360

ccaacgccaa acaaatggga gcctggttac gagtcagcct tcagggactt gtcacatttc
420

tacttggttt cttccttgtt attgtcataa taaaatgttt tctatgctgt ttagtgcaac
480

ttaggcccta ttctgtagaa gtctcctcta ctattcaggc cactcaaaca ccccaaataa
540

ttgagttcaa aatcgacatc aagatataaa ggaatcagtg actaaatata tttcatatat
600

ggtattttta ttgattattg tgctgtcttg acctagtatg gaggccttgg ctagaggctg
660

gtcagtttcc tctcttgagc agctgattaa atccacaccc caaccacttc ccttatcagg
720

ttctcacact ctggggccac tatgtaccca ctctaatcac cacagggcca gacatcagac
780

aattaaggac agcgcccatg ccccaaagcc cgccaaaatt atgcaaatta ttcaaaatta
840

ttcaacctag ctaaccccac ccttttgct gtacataagc tgcccattcc ccctccagcc
900

tgtggtaccc agtcctcagg tgcaaccccc tgcgtggtcc tctgtggcag ccttctctca
960

ttcagagctg ttttccacag aggtagtgaa aagaactgga ttttcaagtt cactttgcaa
1020

gagaaaaaga aaactcagta gaagataatg gcaagtccag actggggata tgatgacaaa
1080

aatggtcctg aacaatggag caagctgtat cccattgcca atggaaataa ccaatcccct
1140

gttgatatta aaaccagtga aaccaaacat gacacctctc tgaaacctat tagtgtctcc
1200

tacaacccag ccacagccaa agaaattatc aatgtggggc attctttcca tgtaaatttt
1260

gaggacaacg ataaccgatc agtgctgaaa ggtggtcctt tctctgacag ctacaggctc
1320

tttcagtttc attttcactg ggcagtaca aatgagcatg gttcagaaca tacagtggat
1380

ggagtcaaat attctgccga gcttcacgta gctcactgga attctgcaaa gtactccagc
1440

cttgctgaag ctgcctcaaa ggctgatggt ttggcagtta ttggtgtttt gatgaaggtt
1500

ggtgaggcca acccaaagct gcagaaagta cttgatgccc tccaagcaat taaaaccaag
1560

ggcaaacgag ccccattcac aaattttgac ccctctactc tccttccttc atccctggat
1620

ttctggacct accctggctc tctgactcat cctcctcttt atgagagtgt aacttggatc
1680

atctgtaagg agagcatcag tgtcagctca gagcagctgg cacaattccg cagccttcta
1740

tcaaatgttg aaggtgataa cgctgtcccc atgcagcaca acaaccgccc aacccaacct
1800

ctgaagggca gaacagtgag agcttcattt tgatgattct gagaagaaac ttgtccttcc
1860

tcaagaacac agccctgctt ctgacataat ccagttaaaa taataatttt taagaaataa
1920

atttatttca atattagcaa gacagcatgc cttcaaatca atctgtaaaa ctaagaaact
1980

taaattttag ttcttactgc ttaattcaaa taataattag taagctagca aatagtaatc
2040

tgtaagcata agcttatctt aaattcaagt ttagtttgag gaattcttta aaattacaac
2100

taagtgattt gtatgtctat ttttttcagt ttatttgaac caataaaata attttatctc
2160

tttctttctg ttgtgcattc agtttctaaa accattaagt ttctactcca tttacattca
2220

aaaatcttaa atactttact tgcaagagta ttttgcttca aatacaacaa cctaagagca
2280

gctggagatg aaatattggg aaattcattt gcttactcct gaagacaaaa atatagctga
2340

gatgaccact ggatttaata tcgttatgct ggcccaacat tgctaccatt tgtgttgtct
2400

gtgatcaaaa tgattatctt ttatatagga agatgacgct tctggatatt gctttcactt
2460

cttctcccca cgttagcaag gacaatgctt ctctgccatt attacaacta gttagtttgc
2520

atggagaatc tttactttaa aattggaaga aaagtcacaa gtgaatggtt tataaaaatg
2580

ctaaagaagt cattcttgct tagaatcata tagaaacatc atgcaatctt ttagtcagat
2640

gtgcgcttca ccttatgcta tttttatctt taattgacac acaataattg tacatgttta
2700

tggagtatag tgtggtgttt tctgtttgtt tgtttgtttt ttgagacaag gtctcactct
2760

gccagtcagg gtggagtgcg atggt
2785

<210> 32
<211> 9588
<212> DNA
<213> human

<400> 32
ccgaccaaca ccaacaccca gctccgacgc agctcctctg cgcccttgcc gccctccgag
60

ccacagcttt cctcccgctc ctgcccccgg cccgtcgccg tctccgcgct cgcagcggcc
120

tcgggagggc ccaggtagcg agcagcgacc tcgcgagcct tccgcactcc cgcccggttc
180

cccggccgtc cgcctatcct tggccccctc cgctttctcc gcgccggccc gcctcgctta
240

tgcctcggcg ctgagccgct ctcccgattg cccgccgaca tgagctgcaa cggaggctcc 300

cacccgcgga tcaacactct gggccgcatg atccgcgccg agtctggccc ggacctgcgc 360

tacgaggtga ccagcggcgg cgggggcacc agcaggatgt actattctcg gcgcggcgtg 420

atcaccgacc agaactcgga cggctactgt caaaccggca cgatgtccag gcaccagaac 480

cagaacacca tccaggagct gctgcagaac tgctccgact gcttgatgcg agcagagctc 540

atcgtgcagc ctgaattgaa gtatggagat ggaatacaac tgactcggag tcgagaattg 600

gatgagtgtt ttgcccaggc caatgaccaa atggaaatcc tcgacagctt gatcagagag 660

atgcggcaga tgggccagcc ctgtgatgct taccagaaaa ggcttcttca gctccaagag 720

caaatgcgag ccctttataa agccatcagt gtccctcgag tccgcagggc cagctccaag 780

ggtggtggag gctacacttg tcagagtggc tctggctggg atgagttcac caaacatgtc 840

accagtgaat gtttggggtg gatgaggcag caaagggcgg agatggacat ggtggcctgg 900

ggtgtggacc tggcctcagt ggagcagcac attaacagcc accggggcat ccacaactcc 960

atcggcgact atcgctggca gctggacaaa atcaaagccg acctgcgcga gaaatctgcg 1020

atctaccagt tggaggagga gtatgaaaac ctgctgaaag cgtcctttga gaggatggat 1080

cacctgcgac agctgcagaa catcattcag gccacgtcca gggagatcat gtggatcaat 1140

gactgcgagg aggaggagct gctgtacgac tggagcgaca agaacaccaa catcgctcag 1200

aaacaggagg ccttctccat acgcatgagt caactggaag ttaaagaaaa agagctcaat 1260

aagctgaaac aagaaagtga ccaacttgtc ctcaatcagc atccagcttc agacaaaatt 1320

gaggcctata tggacactct gcagacgcag tggagttgga ttcttcagat caccaagtgc 1380

attgatgttc atctgaaaga aaatgctgcc tactttcagt tttttgaaga ggcgcagtct 1440

actgaagcat acctgaaggg gctccaggac tccatcagga agaagtaccc ctgcgacaag 1500

aacatgcccc tgcagcacct gctggaacag atcaaggagc tggagaaaga acgagagaaa 1560

atccttgaat acaagcgtca ggtgcagaac ttggtaaaca agtctaagaa gattgtacag
1620

ctgaagcctc gtaacccaga ctacagaagc aataaaccca ttattctcag agctctctgt
1680

gactacaaac aagatcagaa aatcgtgcat aagggggatg agtgtatcct gaaggacaac
1740

aacgagcgca gcaagtggta cgtgacgggc ccgggaggcg ttgacatgct tgttccctct
1800

gtggggctga tcatccctcc tccgaaccca ctggccgtgg acctctcttg caagattgag
1860

cagtactacg aagccatctt ggctctgtgg aaccagctct acatcaacat gaagagcctg
1920

gtgtcctggc actactgcat gattgacata gagaagatca gggccatgac aatcgccaag
1980

ctgaaaacaa tgcggcagga agattacatg aagacgatag ccgaccttga gttacattac
2040

caagagttca tcagaaatag ccaaggctca gagatgtttg gagatgatga caagcggaaa
2100

atacagtctc agttcaccga tgcccagaag cattaccaga ccctggtcat tcagctccct
2160

ggctatcccc agcaccagac agtgaccaca actgaaatca ctcatcatgg aacctgccaa
2220

gatgtcaacc ataataaagt aattgaaacc aacagagaaa atgacaagca agaaacatgg
2280

atgctgatgg agctgcagaa gattcgcagg cagatagagc actgcgaggg caggatgact
2340

ctcaaaaacc tccctctagc agaccagggg tcttctcacc acatcacagt gaaaattaac
2400

gagcttaaga gtgtgcagaa tgattcacaa gcaattgctg aggttctcaa ccagcttaaa
2460

gatatgcttg ccaacttcag aggttctgaa aagtactgct atttacagaa tgaagtattt
2520

ggactatttc agaaactgga aaatatcaat ggtgttacag atggctactt aaatagctta
2580

tgcacagtaa gggcactgct ccaggctatt ctccaaacag aagacatgtt aaaggtttat
2640

gaagccaggc tcactgagga ggaaactgtc tgcctggacc tggataaagt ggaagcttac
2700

cgctgtggac tgaagaaaat aaaaaatgac ttgaacttga agaagtcgtt gttggccact
2760

atgaagacag aactacagaa agcccagcag atccactctc agacttcaca gcagtatcca
2820

ctttatgatc tggacttggg caagttcggt gaaaaagtca cacagctgac agaccgctgg
2880

caaaggatag ataaacagat cgactttaga ttatgggacc tggagaaaca aatcaagcaa 2940

ttgaggaatt atcgtgataa ctatcaggct ttctgcaagt ggctctatga tcgtaaacgc 3000

cgccaggatt ccttagaatc catgaaattt ggagattcca acacagtcat gcggtttttg 3060

aatgagcaga agaacttgca cagtgaaata tctggcaaac gagacaaatc agaggaagta 3120

caaaaaattg ctgaactttg cgccaattca attaaggatt atgagctcca gctggcctca 3180

tacacctcag gactggaaac tctgctgaac atacctatca agaggaccat gattcagtcc 3240

ccttctgggg tgattctgca agaggctgca gatgttcatg ctcggtacat tgaactactt 3300

acaagatctg gagactatta caggttctta agtgagatgc tgaagagttt ggaagatctg 3360

aagctgaaaa ataccaagat cgaagttttg gaagaggagc tcagactggc ccgagatgcc 3420

aactcggaaa actgtaataa gaacaaattc ctggatcaga acctgcagaa ataccaggca 3480

gagtgttccc agttcaaagc gaagcttgcg agcctggagg agctgaagag acaggctgag 3540

ctggatggga agtcggctaa gcaaaatcta gacaagtgct acggccaaat aaaagaactc 3600

aatgagaaga tcacccgact gacttatgag attgaagatg aaaagagaag aagaaaatct 3660

gtggaagaca gatttgacca acagaagaat gactatgacc aactgcagaa agcaaggcaa 3720

tgtgaaaagg agaaccttgg ttggcagaaa ttagagtctg agaaagccat caaggagaag 3780

gagtacgaga ttgaaaggtt gagggttcta ctgcaggaag aaggcacccg gaagagagaa 3840

tatgaaaatg agctggcaaa ggtaagaaac cactataatg aggagatgag taatttaagg 3900

aacaagtatg aaacagagat taacattacg aagaccacca tcaaggagat atccatgcaa 3960

aaagaggatg attccaaaaa tcttagaaac cagcttgata gactttcaag ggaaaatcga 4020

gatctgaagg atgaaattgt caggctcaat gacagcatct tgcaggccac tgagcagcga 4080

aggcgagctg aagaaaacgc ccttcagcaa aaggcctgtg gctctgagat aatgcagaag 4140

aagcagcatc tggagataga actgaagcag gtcatgcagc agcgctctga ggacaatgcc 4200

cggcacaagc agtccctgga ggaggctgcc aagaccattc aggacaaaaa taaggagatc
4260

gagagactca aagctgagtt tcaggaggag gccaagcgcc gctgggaata tgaaaatgaa
4320

ctgagtaagg taagaaacaa ttatgatgag gagatcatta gcttaaaaaa tcagtttgag
4380

accgagatca acatcaccaa gaccaccatc caccagctca ccatgcagaa ggaagaggat
4440

accagtggct accgggctca gatagacaat ctcacccgag aaaacaggag cttatctgaa
4500

gaaataaaga ggctgaagaa cactctaacc cagaccacag agaatctcag gagggtggaa
4560

gaagacatcc aacagcaaaa ggccactggc tctgaggtgt ctcagaggaa acagcagctg
4620

gaggttgagc tgagacaagt cactcagatg cgaacagagg agagcgtaag atataagcaa
4680

tctcttgatg atgctgccaa aaccatccag gataaaaaca aggagataga aaggttaaaa
4740

caactgatcg acaaagaaac aaatgaccgg aaatgcctgg aagatgaaaa cgcgagatta
4800

caaagggtcc agtatgacct gcagaaagca aacagtagtg cgacggagac aataaacaaa
4860

ctgaaggttc aggagcaaga actgacacgc ctgaggatcg actatgaaag ggtttcccag
4920

gagaggactg tgaaggacca ggatatcacg cggttccaga actctctgaa agagctgcag
4980

ctgcagaagc agaaggtgga agaggagctg aatcggctga agaggaccgc gtcagaagac
5040

tcctgcaaga ggaagaagct ggaggaagag ctggaaggca tgaggaggtc gctgaaggag
5100

caagccatca aaatcaccaa cctgacccag cagctggagc aggcatccat tgttaagaag
5160

aggagtgagg atgacctccg gcagcagagg gacgtgctgg atggccacct gagggaaaag
5220

cagaggaccc aggaagagct gaggaggctc tcttctgagg tcgaggccct gaggcggcag
5280

ttactccagg aacaggaaag tgtcaaacaa gctcacttga ggaatgagca tttccagaag
5340

gcgatagaag ataaaagcag aagcttaaat gaaagcaaaa tagaaattga gaggctgcag
5400

tctctcacag agaacctgac caaggagcac ttgatgttag aagaagaact gcggaacctg
5460

aggctggagt acgatgacct gaggagagga cgaagcgaag cggacagtga taaaaatgca
5520

accatcttgg aactaaggag ccagctgcag atcagcaaca accggaccct ggaactgcag 5580

gggctgatta atgatttaca gagagagagg gaaaatttga gacaggaaat tgagaaattc 5640

caaaagcagg ctttagaggc atctaatagg attcaggaat caaagaatca gtgtactcag 5700

gtggtacagg aaagagagag ccttctggtg aaaatcaaag tcctggagca agacaaggca 5760

aggctgcaga ggctggagga tgagctgaat cgtgcaaaat caactctaga ggcagaaacc 5820

agggtgaaac agcgcctgga gtgtgagaaa cagcaaattc agaatgacct gaatcagtgg 5880

aagactcaat attcccgcaa ggaggaggct attaggaaga tagaatcgga aagagaaaag 5940

agtgagagag agaagaacag tcttaggagt gagatcgaaa gactccaagc agagatcaag 6000

agaattgaag agaggtgcag gcgtaagctg gaggattcta ccagggagac acagtcacag 6060

ttagaaacag aacgctcccg atatcagagg gagattgata aactcagaca gcgcccatat 6120

gggtcccatc gagagaccca gactgagtgt gagtggaccg ttgacacctc caagctggtg 6180

tttgatgggc tgaggaagaa ggtgacagca atgcagctct atgagtgtca gctgatcgac 6240

aaaacaacct tggacaaact attgaagggg aagaagtcag tggaagaagt tgcttctgaa 6300

atccagccat tccttcgggg tgcaggatct atcgctggag catctgcttc tcctaaggaa 6360

aaatactctt tggtagaggc caagagaaag aaattaatca gcccagaatc cacagtcatg 6420

cttctggagg cccaggcagc tacaggtggt ataattgatc cccatcggaa tgagaagctg 6480

actgtcgaca gtgccatagc tcgggacctc attgacttcg atgaccgtca gcagatatat 6540

gcagcagaaa aagctatcac tggttttgat gatccatttt caggcaagac agtatctgtt 6600

tcagaagcca tcaagaaaaa tttgattgat agagaaaccg gaatgcgcct gctggaagcc 6660

cagattgctt caggggggtgt agtagaccct gtgaacagtg tctttttgcc aaaagatgtc 6720

gccttggccc gggggctgat tgatagagat ttgtatcgat ccctgaatga tccccgagat 6780

agtcagaaaa actttgtgga tccagtcacc aaaaagaagg tcagttacgt gcagctgaag 6840

gaacggtgca gaatcgaacc acatactggt ctgctcttgc tttcagtaca gaagagaagc 6900

atgtccttcc aaggaatcag acaacctgtg accgtcactg agctagtaga ttctggtata 6960

ttgagaccgt ccactgtcaa tgaactggaa tctggtcaga tttcttatga cgaggttggt 7020

gagagaatta aggacttcct ccagggttca agctgcatag caggcatata caatgagacc 7080

acaaaacaga agcttggcat ttatgaggcc atgaaaattg gcttagtccg acctggtact 7140

gctctggagt tgctggaagc ccaagcagct actggcttta tagtggatcc tgttagcaac 7200

ttgaggttac cagtggagga agcctacaag agaggtctgg tgggcattga gttcaaagag 7260

aagctcctgt ctgcagaacg agctgtcact gggtataatg atcctgaaac aggaaacatc 7320

atctctttgt tccaagccat gaataaggaa ctcatcgaaa agggccacgg tattcgctta 7380

ttagaagcac agatcgcaac cgggggggatc attgacccaa aggagagcca tcgtttacca 7440

gttgacatag catataagag gggctatttc aatgaggaac tcagtgagat tctctcagat 7500

ccaagtgatg ataccaaagg atttttgac cccaacactg aagaaaatct tacctatctg 7560

caactaaaag aaagatgcat taaggatgag gaaacagggc tctgtcttct gcctctgaaa 7620

gaaaagaaga aacaggtgca gacatcacaa aagaataccc tcaggaagcg tagagtggtc 7680

atagttgacc cagaaaccaa taaagaaatg tctgttcagg aggcctacaa gaagggccta 7740

attgattatg aaaccttcaa agaactgtgt gagcaggaat gtgaatggga agaaataacc 7800

atcacgggat cagatggctc caccagggtg gtcctggtag atagaaagac aggcagtcag 7860

tatgatattc aagatgctat tgacaagggc cttgttgaca ggaagttctt tgatcagtac 7920

cgatccggca gcctcagcct cactcaattt gctgacatga tctccttgaa aaatggtgtc 7980

ggcaccagca gcagcatggg cagtggtgtc agcgatgatg tttttagcag ctcccgacat 8040

gaatcagtaa gtaagatttc caccatatcc agcgtcagga atttaaccat aaggagcagc 8100

tctttttcag acaccctgga agaatcgagc cccattgcag ccatctttga cacagaaaac 8160

ctggagaaaa tctccattac agaaggtata gagcggggca tcgttgacag catcacgggt 8220

cagaggcttc tggaggctca ggcctgcaca ggtggcatca tccacccaac cacgggccag 8280

aagctgtcac ttcaggacgc agtctcccag ggtgtgattg accaagacat ggccaccagc 8340

gtgaagcctg ctcagaaagc cttcataggc ttcgagggtg tgaagggaaa gaagaagatg 8400

tcagcagcag aggcagtgaa agaaaaatgg ctcccgtatg aggctggcca gcgcttcctg 8460

gagttccagt acctcacggg aggtcttgtt gacccggaag tgcatgggag gataagcacc 8520

gaagaagcca tccggaaggg gttcatagat ggccgcgccg cacagaggct gcaagacacc 8580

agcagctatg ccaaaatcct gacctgcccc aaaaccaaat taaaaatatc ctataaggat 8640

gccataaatc gctccatggt agaagatatc actgggctgc gccttctgga agccgcctcc 8700

gtgtcgtcca agggcttacc cagcccttac aacatgtctt cggctccggg gtcccgctcc 8760

ggctcccgct cgggatctcg ctccggatct cgctccgggt cccgcagtgg gtcccggaga 8820

ggaagctttg acgccacagg gaattcttcc tactcttatt cctactcatt tagcagtagt 8880

tctattgggc actagtagtc agttgggagt ggttgctata ccttgacttc atttatatga 8940

atttccactt tattaaataa tagaaaagaa aatcccggtg cttgcagtag agtgatagga 9000

cattctatgc ttacagaaaa tatagccatg attgaaatca aatagtaaag gctgttctgg 9060

ctttttatct tcttagctca tcttaaataa gcagtacact tggatgcagt gcgtctgaag 9120

tgctaatcag ttgtaacaat agcacaaatc gaacttagga tttgtttctt ctcttctgtg 9180

tttcgatttt tgatcaattc tttaattttg gaagcctata atacagtttt ctattcttgg 9240

agataaaaat taaatggatc actgatattt tagtcattct gcttctcatc taaatatttc 9300

catattctgt attaggagaa aattaccctc ccagcaccag ccccccctctc aaaccccccaa 9360

cccaaaacca agcattttgg aatgagtctc ctttagtttc agagtgtgga ttgtataacc 9420

catatactct tcgatgtact tgtttggttt ggtattaatt tgactgtgca tgacagcggc 9480

aatcttttct ttggtcaaag ttttctgttt attttgcttg tcatattcga tgtactttaa
9540

ggtgtcttta tgaagtttgc tattctggca ataaactttt agactttt
9588

<210> 33
<211> 366
<212> DNA
<213> human

<220>
<221> misc_feature
<222> (351)..(351)
<223> any kind of base

<400> 33
gaagtgccat ttatatttat acaaaaatat tacataattc agttagtatt ggtgacataa
60

tttagttagt atgggtgata taatggtcat aatttttagc atctaataaa gatcttttta
120

tgagtcccat ataaaatatg tgaacaaagc aatcttgtca taagatttgt gatgatttag
180

gagaaagtac tttgagataa tttttttctg tctctttgtg aactctctca acagtagttc
240

tctttagatt agagccagca ggtcggccat aacagttttc ttcaaatttg ggcaacagtt
300

atacaaatgc ttgaatttca agacaacata ttaaagggtc tatgaactgg naatctaacc
360

tgggtt
366

<210> 34
<211> 1466
<212> DNA
<213> human

<400> 34
agccccaagc ttaccacctg cacccggaga gctgtgtgtc accatgtggg tcccggttgt
60

cttcctcacc ctgtccgtga cgtggattgg tgctgcaccc ctcatcctgt ctcggattgt
120

gggaggctgg gagtgcgaga agcattccca accctggcag gtgcttgtgg cctctcgtgg
180

cagggcagtc tgcggcggtg ttctggtgca cccccagtgg gtcctcacag ctgcccactg
240

catcaggaac aaaagcgtga tcttgctggg tcggcacagc ctgtttcatc ctgaagacac
300

aggccaggta tttcaggtca gccacagctt cccacacccg ctctacgata tgagcctcct
360

gaagaatcga ttcctcaggc caggtgatga ctccagccac gacctcatgc tgctccgcct
420

gtcagagcct gccgagctca cggatgctgt gaaggtcatg gacctgccca cccaggagcc
480

agcactgggg accacctgct acgcctcagg ctggggcagc attgaaccag aggagttctt
540

gaccccaaag aaacttcagt gtgtggacct ccatgttatt tccaatgacg tgtgtgcgca
600

agttcaccct cagaaggtga ccaagttcat gctgtgtgct ggacgctgga caggggggcaa
660

aagcacctgc tcgggtgatt ctgggggccc acttgtctgt aatggtgtgc ttcaaggtat
720

cacgtcatgg ggcagtgaac catgtgccct gcccgaaagg ccttccctgt acaccaaggt
780

ggtgcattac cggaagtgga tcaaggacac catcgtggcc aacccctgag cacccctatc
840

aaccccctat tgtagtaaac ttggaacctt ggaaatgacc aggccaagac tcaagcctcc
900

ccagttctac tgacctttgt ccttaggtgt gaggtccagg gttgctagga aaagaaatca
960

gcagacacag gtgtagacca gagtgtttct taaatggtgt aattttgtcc tctctgtgtc
1020

ctggggaata ctggccatgc ctggagacat atcactcaat ttctctgagg acacagatag
1080

gatggggtgt ctgtgttatt tgtggggtac agagatgaaa gaggggtggg atccacactg
1140

agagagtgga gagtgacatg tgctggacac tgtccatgaa gcactgagca gaagctggag
1200

gcacaacgca ccagacactc acagcaagga tggagctgaa aacataaccc actctgtcct
1260

ggaggcactg ggaagcctag agaaggctgt gagccaagga gggagggtct tcctttggca
1320

tgggatgggg atgaagtaag gagagggact ggacccccctg gaagctgatt cactatgggg
1380

ggaggtgtat tgaagtcctc cagacaaccc tcagatttga tgatttccta gtagaactca
1440

cagaaataaa gagctgttat actgtg
1466

<210> 35
<211> 187
<212> DNA
<213> human

<400> 35
gatctggtgc attccggtcg acactctcgt ttatttggac tgtaagtctg acctctatga
60

ataattactt cagcccctga gtgctcccgg gccaagctcc ttggccaaac tttcacctta
120

gcttctgata agtcttgggc caagctaagc agcatctatc aatcatccct tcagctcctg
180

attgatc
187

<210> 36
<211> 2913
<212> DNA
<213> human

<400> 36
actgggtacc gaggactggg tgtgtttaag gcagacagcc aggtgaggat cccagctact
60

ggggcctgct gtcatctcct gggagtaccc gggggtcagg agcctagggg actcttgcac
120

ttcacatcca gccatgctaa ttacactttt tggcaaagga aacagctagg agcagtttct
180

ttcactccta cagccccgtt ttctcagtgt ttagacctcg aattattact gggctagagg
240

gaaggcagcc tctgaagtgt ggcaggagga ggggaagtct gcctgcatct tggtgtgtct
300

gtcagatgcc agcactaata acctggcttc tgtgaggcct gtcagtgctc tcaggaatga
360

aaggggaccc ctgagaggtg ctcagtacca gcaggctgtg aatgctctct acccaccacc
420

ctcacctcct cgttaaagat ggtgctacct gccacacagc agacatctgg tcgctgcaca
480

cccgaaagac cccaaggcag tctgcccctt gtccagccac acgccagcac ccaccctcct
540

ggcccctgcc tcggcctccc cagaccagct gcacccagcc cccaacacgc accccttctc
600

cagatgtgtg cagggcctca ttttgcagag caaagacaga tgtttcagcc acacgcttta
660

ttaacttcta aaacctgtgc tcaggacact cttcaacagt catgaaaagt ttgatcactt
720

gccacagtca ggacctttgt gtggggctct gatctgatgt tcggtctcat catctcccaa
780

accagcagtc gtttgtaccc caaccctctg ctcaggggct catacccccca aatgattttc
840

ctgatttatg tatttcccta caaagggctt tctataccta gcatctgcct ccagcatgag
900

aaggggggaat aggtgagacc catttgccag tagcagacgg ggaccctggg gagaaaatgg
960

cagagcctgt tggagactcc ctgtctccag ctgaccagcc aatgggattc ctcttccctc

1020

cactgtctcc cacaaagtag aagaatcctg gtacatttag cccatgagcc tggcacagat
1080

ccctatctag acatgaggcc ctttagacat gactttggca ttgaccagcc tgttggcaat
1140

gggtcgggga ggcagagggg atgctcacac cagtaattct catcccctga atgcttggga
1200

tcacctgggg agagttcaca aaatactggt gcaggggtcc cacctctgat gatgctgagt
1260

ggtgggtctg gggtgtggcc caggcatcat gatgtttcag gcccccaggt gacttcttag
1320

gcagcccagc taagccccta gagccttgca atttccccca aatgacctca gagggcccga
1380

tttgagggaa atgcctaact tcaggggccg taagaatccc ccagggagca tgtgaaatgc
1440

agataccagg cccaccccca gagatgagct gaggtgggtc aggggtgaag tgcagggatc
1500

agtgtttttc acaagctcca tacctccagg aaatggtgtt gtggttgggc ccgtagaaaa
1560

cattctgaga gtcctgttgc ctgtgccttg gtgcacgtgg ggtggaatcc cagtggccct
1620

gccttgagga ggatgtgcat taacgtggta ggggagacag agacagctcc acctgccccc
1680

tgtcccaccg gggacctcca aaaacttcat ggatgttaga gcaagcagcc atgctgcagc
1740

agaggatgag gctggcggat ttagtaagag ccctctgtgt ttgggctgag ttctttctct
1800

agttgccctg tcatctggcc tctggataac ccacctctcc tccctcatcc taaaattaca
1860

gatggcgaaa gacggccaca tttagtgaga cccctaaggt cctccaacta gggtgggtcc
1920

acagtggccc ctggtgcatg gaccacacac tctcttccct cctctggctc aggactacgg
1980

tctgaaatta gggagatatg aatgtctttc ttgaaaactt ctcttcccag tcttcccact
2040

ttgcttgggg gtccttggtc aaggccagct ttggactagg gcttgttgcg actaccagct
2100

gtctcatttt gctgtactgc aaactcaggc ttggttccaa gcttatgggg gccctgtcct
2160

tcccctagta gggtttgttt tggggtcaca tctggtcata cccttcagag agctcttccc
2220

cagcctctac atcagggaga gaggtaggta gggaggagca ttcaaggatt agaagaagga
2280

ctaaagtaca acagccttgg aggaactgcc aggaactaag ggcgagcact ggagaaggca

2340

acctgggacc ccctgcgctt ctgagcagga agaccaagac cttcaggggc cctaagcact
2400

gaaaacatca ttcctcatcc ccaagccctg gcatccccct gttcttctaa aataattctt
2460

ttctaggtat ttctgattgc aaaattctgg atgggttcat ccaagctgac ctttgctgtt
2520

ttttcccttc ccaacaaggc ctcacttttt ggagccacct tagttggtgc ctaggcagag
2580

gggcagtcag cagtggttat caggatcctg gctctatggg ttgccttcct cctggtctgt
2640

aaagcccctg caggcaggga cttcttagat agctgcttcc ttagggcatg gcatgtggtg
2700

ggtggttaat gaatggaaga gagggaatga gtgatcaagg gagggaggag ggagtggagt
2760

ggagatttct catcctttcc tgttaattta tgacatcctc ctgcctatga gtccttgact
2820

ctggagtttt acaaagcagt cacatttcaa ataaaagtct gggaaagcaa cacatcatcg
2880

ccaactttta attttgctaa ataaggatat tag
2913

<210> 37
<211> 1466
<212> DNA
<213> human

<400> 37
agccccaagc ttaccacctg cacccggaga gctgtgtgtc accatgtggg tcccggttgt
60

cttcctcacc ctgtccgtga cgtggattgg tgctgcaccc ctcatcctgt ctcggattgt
120

gggaggctgg gagtgcgaga agcattccca accctggcag gtgcttgtgg cctctcgtgg
180

cagggcagtc tgcggcggtg ttctggtgca cccccagtgg gtcctcacag ctgcccactg
240

catcaggaac aaaagcgtga tcttgctggg tcggcacagc ctgtttcatc ctgaagacac
300

aggccaggta tttcaggtca gccacagctt cccacacccg ctctacgata tgagcctcct
360

gaagaatcga ttcctcaggc caggtgatga ctccagccac gacctcatgc tgctccgcct
420

gtcagagcct gccgagctca cggatgctgt gaaggtcatg gacctgccca cccaggagcc
480

agcactgggg accacctgct acgcctcagg ctggggcagc attgaaccag aggagttctt
540

gaccccaaag aaacttcagt gtgtggacct ccatgttatt tccaatgacg tgtgtgcgca
600

agttcaccct cagaaggtga ccaagttcat gctgtgtgct ggacgctgga caggggggcaa
660

aagcacctgc tcgggtgatt ctgggggccc acttgtctgt aatggtgtgc ttcaaggtat
720

cacgtcatgg ggcagtgaac catgtgccct gcccgaaagg ccttccctgt acaccaaggt
780

ggtgcattac cggaagtgga tcaaggacac catcgtggcc aacccctgag cacccctatc
840

aaccccctat tgtagtaaac ttggaacctt ggaaatgacc aggccaagac tcaagcctcc
900

ccagttctac tgacctttgt ccttaggtgt gaggtccagg gttgctagga aaagaaatca
960

gcagacacag gtgtagacca gagtgtttct taaatggtgt aattttgtcc tctctgtgtc
1020

ctggggaata ctggccatgc ctggagacat atcactcaat ttctctgagg acacagatag
1080

gatggggtgt ctgtgttatt tgtggggtac agagatgaaa gaggggtggg atccacactg
1140

agagagtgga gagtgacatg tgctggacac tgtccatgaa gcactgagca gaagctggag
1200

gcacaacgca ccagacactc acagcaagga tggagctgaa aacataaccc actctgtcct
1260

ggaggcactg ggaagcctag agaaggctgt gagccaagga gggagggtct tcctttggca
1320

tgggatgggg atgaagtaag gagagggact ggacccccctg gaagctgatt cactatgggg
1380

ggaggtgtat tgaagtcctc cagacaaccc tcagatttga tgatttccta gtagaactca
1440

cagaaataaa gagctgttat actgtg
1466

<210> 38
<211> 462
<212> DNA
<213> human

<220>
<221> misc_feature
<222> (197)..(197)
<223> any kind of base

<220>
<221> misc_feature
<222> (116)..(116)
<223> any kind of base

<220>
<221> misc_feature
<222> (334)..(334)
<223> any kind of base

<220>
<221> misc_feature
<222> (402)..(402)
<223> any kind of base

<220>
<221> misc_feature
<222> (429)..(429)
<223> any kind of base

<220>
<221> misc_feature
<222> (438)..(438)
<223> any kind of base

<220>
<221> misc_feature
<222> (443)..(443)
<223> any kind of base

<220>
<221> misc_feature
<222> (459)..(459)
<223> any kind of base

<400> 38
taaggtttta taattatttt tatttttctt ttcttttttt tttatggctt ggatgacact
60

ttattttcag atccaatact agaagttgtt tccatgttca cattttcctt cctggnttaa
120

aaaaaagagt tgtatttttt ttttttgctt tttttaaatt atactttaag ttttagggta
180

catgtgcaca acgcagnggt tagctacata tgtatacatg tgccatgttg gcgtgctgca
240

tccagtaact cgtcatttaa cattaggtat atctccaaat gctatccttc cccccattgt
300

atttttcata gcttaaaaat cattgacata ggantaattc caactaaagt acggtattaa
360

atccctgggg gaataaattt tgtcttaaca agggtaaggt tngtgaaaag gatggttttg
420

tcacagggna aaaggganat ccncccattt taaaacccnc ct
462

<210> 39
<211> 1490
<212> DNA
<213> human

<400> 39
ctcgtgcccc ccacggaggg gactgctctc ccccgctgca tcctttctgt gaggtacctt 60
acccacctca gcacctgaga gggtgaaata gaattctaac ctcgacattc gggaagtgtt 120
tttgagaagt ctcggtcggt aagggaagtc ttccaagtcc gtgcagcact aacgtattgg 180
cacctgcctc ctcttcggcc accccccaga tgaggcagct gtgactgtgt caagggaagc 240
cacgactctg accatagtct tctctcagct tccactgccg tctccacagg aaacccagaa 300
gttctgtgaa caagtccatg ctgccatcaa ggcatttatt gcagtgtact atttgcttcc 360
aaaggatcag gggatcaccc tgagaaagct ggtacggggc gccaccctgg acatcgtgga 420
tggcatggct cagctcatgg aagtactttc cgtcactcca actcagagcc ctgagaacaa 480
tgaccttatt tcctacaaca gtgtctgggt tgcgtgccag cagatgcctc agataccaag 540
agataacaaa gctgcagctc ttttgatgct gaccaagaat gtggattttg tgaaggatgc 600
acatgaagaa atggagcagg ctgtggaaga atgtgaccct tactctggcc tcttgaatga 660
tactgaggag aacaactctg acaaccacaa tcatgaggat gatgtgttgg ggtttcccag 720
caatcaggac ttgtattggt cagaggacga tcaagagctc ataatcccat gccttgcgct 780
ggtgagagca tccaaagcct gcctgaagaa aattcggatg ttagtggcag agaatgggaa 840
gaaggatcag gtggcacagc tggatgacat tgtggatatt tctgatgaaa tcagccctag 900
tgtggatgat ttggctctga gcatatatcc acctatgtgt cacctgaccg tgcgaatcaa 960
ttctgcgaaa cttgtatctg ttttaaagaa ggcacttgaa attacaaaag caagtcatgt 1020
gacccctcag ccagaagata gttggatccc tttacttatt aatgccattg atcattgcat 1080
gaatagaatc aaggagctca ctcagagtga acttgaatta tgacttttca ggctcatttg 1140
tactctcttc ccctctcatc gtcatggtca ggctctgata cctgctttta aaatggagct 1200
agaatgcttg ctggattgaa agggagtgcc tatctatatt tagcaagaga cactattacc 1260
aaagattgtt ggttaggcca gattgacacc tatttataaa ccatatgcgt atatttttct

1320

gtgctatata tgaaaaataa ttgcatgatt tctcattcct gagtcatttc tcagagattc
1380

ctaggaaagc tgccttattc tctttttgca gtaaagtatg ttgttttcat tgtaaagatg
1440

ttgatggtct caataaaatg ctaacttgcc agtgattaaa aaaaaaaaaa
1490

<210> 40
<211> 1677
<212> DNA
<213> human

<400> 40
cttgacccta tttatagtgg ctctaaaggt ggtgttatta tgttttctag agcacttcga
60

ttatacaaac gtcaaggaat ccgagttaat gtgctttgcc ctgagtttgt tgaaacagac
120

atgggcacaa tgatcggtcc caaattcctt agtatgatgg ggggctttgt acctatggaa
180

atggtggtga aaggtgcttt tgagctcatc actgatgaga ataaagccgg cgattgccta
240

tggattacta atcggcgagg tcttgagtac tggcccaccc catcagaaga agcaaagtac
300

ttgctgcgtt ctacacgttc caggagaaga actgaataca aagctccacc aattaaacta
360

cctgagagtt ttgagaaaat agttgttcag accttgactc acaactttcg gaatgctacc
420

agtgtagtaa gagcaccact gagattacct atcaaaccaa actatgttct tgtgaagata
480

atctatgctg gtgtaaatgc tagtgatgta aattttagct caggtcgcta ttttggtggc
540

aataacagtg acactgcatc ccgtcttccg tttgatgcag gatttgaggc tgtgggagta
600

attgcagcag ttggggattc tgttactgac ttgaaagttg gcatgccttg tgcgttcatg
660

acttttggag gctatgctga atttacaatg attccttcga aatacgccct tccaatgcct
720

agaccagaac cggaaggtgt tgccatgctt acatcaggat taacagcttc aattgctcta
780

gaaaaggcag gacagatgga atctggaaaa gtggtccttg ttactgctgc ggcaggagga
840

actggtcagt ttgctgttca gcttgcaaaa ttagctggta ataccgtggt tgccacttgt
900

ggaggtgggg caaaggccaa gcttctgaaa gaattgggag tcgacagagt catagactat
960

cacagtgaag atataaaaac ggttctaagg aaagagttcc cgaaaggtat tgatatcatc
1020

tacgaatctg ttggtgggga catgttaaag ttgtgcttgg atgctttggc agtccatgga
1080

cgactcattg tcattggcat gatttctcag tatcaaggag aaaatggttg gacgccatca
1140

aaatatcctg gactatgtga gaagctcttg tcaaagagtc aaactgtggc tggctttttc
1200

ctggtgcaat atagtcacat gtaccaagaa caccttaaca agttatttga cctttactct
1260

tccggaaaac taaaggttgc tgtggatcca aagagattta taggccttca ttctgttgct
1320

gatgctgttg agtatctcca ttcaggcaaa agcgttggga aggtggttgt ctgcgtggac
1380

ccgaccttcg gtcatcaagt agccaaatta tgaatgaaca cggtgtcaaa tacagaaaga
1440

agtgaagttt tcaattctta gtctagagat tgttctcgaa tgttactgaa aatagctgct
1500

agaccagtgc tggaatattt attctcaatg ctttttcaat tttggattac ttgaaagaat
1560

aatccattta tgtataccat gtttatgttt acactataca acaactatga gcagaagaaa
1620

gcgagatatc tacaaaataa attataatcc tttcatttta aaaaaaaaaa aaaaaaa
1677

<210> 41
<211> 1330
<212> DNA
<213> human

<400> 41
atggcgcagt gggacagctt cactgatcaa caggaggaca ctgatagctg ttcagaatct
60

gtgaagtttg atgctcgctc caatacagct ttgcttcccc caaatcctaa aaatggccct
120

ccacttcaag aaaagctgaa atccttcaaa gctgcactga ttgcccttta tctccttgtg
180

tttgctgttc tcattcctat catcgcaata atggcagctc aactcctgaa gtgggaaatg
240

aagaattgca cagttggttc aattaatgca aacagtgtat cctccagtct cctgggaaga
300

ggaaatgaca gtgaagatga agtgagattt cgagaagttg ttatggaaca cattagcaag
360

atggagaaaa gaatccaata tatttcagat actgaagaaa atctcgtaga ttcagagcat
420

tttcaaaatt tcagtgtgac aactgatcaa cgatttgctg atgttcttct ccaactaagt
480

acctttggttc ccacagtcca gggacatggg aatgccgtag atgaaatcac caggtcctta
540

ataagtctga ataccacgct gcttgatttg cacctctatg tagaaacact gaatgtcaaa
600

ttccaggaga atacacttaa agggcaagag gaaatcagca aattaaagga gcgtgtgcac
660

aatgcatcag cagaaattat gtctatgaaa gaagaacaag tgcatttgga acaggaaata
720

aaaagagaag tgaaagtcct gaataacatc actaatgatc tcaggctgaa agattgggaa
780

cattctcaga cgttgagaaa tatcacttta attcaaggtc ctcctggacc cccaggagaa
840

aaaggagata gaggtccaac cggagaaagt ggtccaccag gcgttccagg tccagtaggt
900

cctccaggtc ttaagggtga tcgaggatct attggctttc cgggaagtcg aggatatcca
960

ggacaatcag ggaagactgg gaggacagga tatcctggac caaaaggcca aaagggagaa
1020

aaaggcagtg gaagcatcct gactccttct gcgactgtcc gactggttgg tggccgtggc
1080

cctcatgagg gtagagtgga gatattgcac aatggacagt ggggcacagt ttgtgatgat
1140

cactgggaac tgcgtgccgg gcaggttgtc tgcaggagct tgggataccg aggtgttaag
1200

agtgtgcaca agaaagctta ttttggacaa ggtactggtc ccatttggct gaatgaagta
1260

ccctgtttgg ggatggagtc atccattgaa gagtgcaaaa tcagacagtg gggcgtgaga
1320

gtctgttcac
1330

<210> 42
<211> 431
<212> DNA
<213> human

<220>
<221> misc_feature
<222> (97)..(97)
<223> any kind of base

<220>
<221> misc_feature
<222> (347)..(347)
<223> any kind of base

<220>
<221> misc_feature

<222> (349)..(349)
<223> any kind of base

<220>
<221> misc_feature
<222> (361)..(361)
<223> any kind of base

<220>
<221> misc_feature
<222> (362)..(362)
<223> any kind of base

<220>
<221> misc_feature
<222> (363)..(363)
<223> any kind of base

<220>
<221> misc_feature
<222> (401)..(401)
<223> any kind of base

<220>
<221> misc_feature
<222> (428)..(428)
<223> any kind of base

<400> 42
cttttatat ttattttcat cgctacacaa acatttttta ggagtttgat tctacctcca
60

ttttggttag atatacaaac tctaccccat gagggantgt atggtgtatt tctagattta
120

gcaacaattt tcttgaaaaa tgtacaatac tatagaaaaa tgaagatagt aaataccagg
180

tataagttaa taacagtgtt tcttttgttc agtaataatg aactgtgtac tagcactgaa
240

ctttaggccc tcctatttgc gtattttctg tttgtatatt tttaaataga ggaattgtga
300

ttataatatt attattttgg aatatcctaa atcataaatt caaaacntna tttagttttt
360

nnnttttttt tttaagatgg agtcccgctt tgtcccaggc nggagtgcag tggcatgatc
420

tcagctcnct g
431

<210> 43
<211> 669
<212> DNA
<213> human

<220>

<221> misc_feature
<222> (641)..(641)
<223> any kind of base

<400> 43
ttcttttgga aaaccaaaca tgctttattt catttttttc acaatttatt taaacatctc
60

acatatacaa aataggtaca atttaatttt tctgcttgcc caagaaacaa agcttctgtg
120

gaaccatgga agaagatgaa aatgagactg gcaaagaaca aatgctgaat ctgaagaaga
180

ggacaacttt gggcaaataa tctgcatact tttaattggg aataagatgg aaaatatgaa
240

tgctaaatca aattttttaa aaaatacacc acacgataca actcaataca ggagtatttc
300

ttctcaaatt cttctagcac catcaacatt cttcaagtat ctgaaatact attaattagc
360

acctttgtat tatgaacaaa acaaaacaag gacctcagtt catctctgtc taggtcagca
420

cctaacaatg tggatcacac tcatgggaaa gtgttttgag gtagtttaaa cctttggaag
480

tttgggtttt aaacttccct ctgtggaaga tattcaaaag ccacaagtgg tgcaaatgtt
540

tatggttttt atttttcaat ttttattttg gttttcttac aaaggttgac atttttcata
600

acaggtgtaa gagtgttgaa aaaaaaattt caatttttgg ngggaacggg ggaaggagtt
660

aatgaaact
669

<210> 44
<211> 287
<212> DNA
<213> human

<400> 44
gccggagagt ctacaatgtt acccagcatg ctgttggcat tgttgtaaac aaacaagtta
60

agggcaagat tcttgccaag agaattaatg tgcgtattga gcacattaag cactctaaga
120

gccgagatag cttcctgaaa cgtgtgaagg aaaatgatca gaaaaagaaa gaagccaaag
180

agaaaggtac ctgggttcaa ctaaagcgcc acgctgctcc acccagagaa gcacactttg
240

tgagaaccaa tgggaaggag cctgagctgc tggaacctat tccctat
287

<210> 45

<211> 383
<212> DNA
<213> human

<220>
<221> misc_feature
<222> (147)..(147)
<223> any kind of base

<220>
<221> misc_feature
<222> (309)..(309)
<223> any kind of base

<220>
<221> misc_feature
<222> (349)..(349)
<223> any kind of base

<220>
<221> misc_feature
<222> (365)..(365)
<223> any kind of base

<220>
<221> misc_feature
<222> (372)..(372)
<223> any kind of base

<220>
<221> misc_feature
<222> (380)..(380)
<223> any kind of base

<400> 45
ggaacggaaa aggagaattc aagtgtgacc ctcatgaggc aacgtgttat gatgatggga
60

agacatacca cgtaggagaa cagtggcaga aggaatatct cggtgccatt tgctcctgca
120

catgctttgg aggccagcgg ggctcgnctt gtgacaactg ccgcagacct gggggtgaa
180

cccagtcccg aaggcactac tggccagtcc tacaaccagt attcttcaga gataccattc
240

agagaacaaa cactaatgtt taatttgccc aatttgagtg cttcatgcct tttaggatgt
300

tacaggctng acagagaagg ttttcccgag gagttaaatc atctttttnc catttcccga
360

ggggncaagg cntgtttttn ttt
383

<210> 46
<211> 523
<212> DNA

<213> human

<400> 46
cagaggggca gggcggacgg ctaggagttc aagaaacatc ctggtctgag ggaaaggctg
60

cagctgcacc gccatgaata agcttttcag cttctggaag aggaagaatg agacccgcag
120

ccagggctac aaccttcgag aaaaggattt aaagaaactt cacagagctg cttcagtcgg
180

ggatttgaag aagctgaagg aataccttca gatcaagaaa tatgatgtaa atatgcagga
240

ctatgaatac agaacacctt tgcacctagc ctgtgctaat ggacatacag atgttgtact
300

tttcctaatt gagcaacaat gcaagataaa tgtccgggat agtgaaaaca aatccccatt
360

gattaaggca gtacagtgtc aaaatgagga ttgtgctact attctgctaa actttggtgc
420

agacccagat ctgagggata ttcgttataa tactgttctt cactatgctg tttgtggtca
480

aagtttgtca ttagttgaaa aactgcttga atacgaagct gat
523

<210> 47
<211> 390
<212> DNA
<213> human

<400> 47
tccaaggtca tggcaaaaca tctgaagttc atcgccagga ctgtgatggt acaggaaggg
60

aacgtggaaa gcgcatacag gaccctaaac agaatcctca ctatggatgg gctcattgag
120

gacattaagc atcggcggta ttatgagaag ccatgccgcc gcgacagagg gaaagctatg
180

aaaggtgccg gcggatctac aacatggaaa tggctcgcaa gatcaacttc ttgatgcgaa
240

agaatcgggc agatccgtgg cagggctgct gaggcctgtg ggtgggacac cagtgcgaaa
300

ccctcatcca gttttctctc catctctttt ctttgtacaa tcccatttcc tattaccatt
360

ctctgcaata aactcaaatc acatgtctgc
390

<210> 48
<211> 669
<212> DNA
<213> human

<220>
<221> misc_feature

<222> (641)..(641)
<223> any kind of base

<400> 48
ttcttttgga aaaccaaaca tgctttattt catttttttc acaatttatt taaacatctc 60

acatatacaa aataggtaca atttaatttt tctgcttgcc caagaaacaa agcttctgtg 120

gaaccatgga agaagatgaa aatgagactg gcaaagaaca aatgctgaat ctgaagaaga 180

ggacaacttt gggcaaataa tctgcatact tttaattggg aataagatgg aaaatatgaa 240

tgctaaatca aattttttaa aaaatacacc acacgataca actcaataca ggagtatttc 300

ttctcaaatt cttctagcac catcaacatt cttcaagtat ctgaaatact attaattagc 360

acctttgtat tatgaacaaa acaaaacaag gacctcagtt catctctgtc taggtcagca 420

cctaacaatg tggatcacac tcatgggaaa gtgttttgag gtagtttaaa cctttggaag 480

tttgggtttt aaacttccct ctgtggaaga tattcaaaag ccacaagtgg tgcaaatgtt 540

tatggttttt atttttcaat ttttattttg gttttcttac aaaggttgac atttttcata 600

acaggtgtaa gagtgttgaa aaaaaaattt caatttttgg ngggaacggg ggaaggagtt 660

aatgaaact 669

<210> 49
<211> 431
<212> DNA
<213> human

<220>
<221> misc_feature
<222> (97)..(97)
<223> any kind of base

<220>
<221> misc_feature
<222> (347)..(347)
<223> any kind of base

<220>
<221> misc_feature
<222> (349)..(349)
<223> any kind of base

<220>

<221> misc_feature
<222> (361)..(361)
<223> any kind of base

<220>
<221> misc_feature
<222> (362)..(362)
<223> any kind of base

<220>
<221> misc_feature
<222> (363)..(363)
<223> any kind of base

<220>
<221> misc_feature
<222> (401)..(401)
<223> any kind of base

<220>
<221> misc_feature
<222> (428)..(428)
<223> any kind of base

<400> 49
ctttttatat ttattttcat cgctacacaa acatttttta ggagtttgat tctacctcca
60

ttttggttag atatacaaac tctaccccat gagggantgt atggtgtatt tctagattta
120

gcaacaattt tcttgaaaaa tgtacaatac tatagaaaaa tgaagatagt aaataccagg
180

tataagttaa taacagtgtt tcttttgttc agtaataatg aactgtgtac tagcactgaa
240

ctttaggccc tcctatttgc gtattttctg tttgtatatt tttaaataga ggaattgtga
300

ttataatatt attattttgg aatatcctaa atcataaatt caaaacntna tttagttttt
360

nnnttttttt tttaagatgg agtcccgctt tgtcccaggc nggagtgcag tggcatgatc
420

tcagctcnct g
431